# EUROPEAN PATENT APPLICATION

(11) **EP 2 389 947 A1**
(43) Date of publication of application: **30.11.2011**
(21) Application number: 11167328.1
(22) Date of filing: 22.03.2007
(51) Int. Cl.: A61K 39/00, A61K 47/48, C07K 16/30, A61K 51/10, C12Q 1/68, G01N 33/574

(54) **Anti-tumor cell antigen antibody therapeutics**

(30) Priority: 23.03.2006 US 785704 P
(62) Divisional of application: 07773740.1
(71) Applicant: Novartis AG, 4056 Basel (CH)
(72) Inventor: Janatpour, Mary J., Emeryville, CA 94662-8097 (US); Yu, Guoying K., Emeryville, CA 94662-8097 (US)
(74) Representative: UEXKÜLL & STOLBERG

(57) **Abstract**

Methods of diagnosing, detecting or treating cancer where the cancer expresses a tumor cell antigen that is elevated relative to the normal adjacent tissue, by administering an antibody or antibody fragment which binds to that tumor cell antigen. The methods involve the use of antibodies that bind to the tumor cell antigens KIAA1815, LOC157378, FU20421, DSCD75, GPR160, GPCR41, and SLC1A5.

## Description

### FIELD OF THE INVENTION

The present invention relates generally to anti-tumor cell antigen agonists or antagonists, including humanized anti-cancer antibodies and methods of diagnosis and/or treatment utilizing these antibodies.

### DESCRIPTION OF RELATED ART

Despite significant advances in the development of anti-cancer therapeutics, cancer remains the second leading cause of death in the overall population (22.8% in 2002, most recently available statistics). In fact, it is estimated that an American male has a 1 in 2 chance of developing some form of cancer in his lifetime, while an American female has a 1 in 3 chance (from www.cancer.org). Most conventional cancer therapies act by killing actively proliferating cells, and as a result are often non-specific and can damage healthy tissues or lead to systemic toxicities. An alternate approach seeks to identify and exploit markers expressed on tumor cells in an effort to differentiate the targeted cancer cells from the normal healthy cells in the body. Agonist/antagonist molecules or antibodies can be developed which act as binding partners of those tumor markers and then act either directly on the tumor cells themselves, or induce an immunological response against those cancerous cells, or selectively deliver some manner of therapeutic payload (review, see Houshmand and Zlotnik (2003) Current Opinion in Cell Biology 15(5):640-44).

There are several anti-cancer antibody therapeutics that are either approved for therapeutic or diagnostic usage or are under preclinical and clinical development. Approved therapeutic anti-cancer antibodies currently available include Alemtuzumab (Campath) for chronic lymphocytic leukemia (CLL) (ILEX Oncology and Schering AG), Rituximab (Rituxan) for non-Hodgkin lymphoma (NHL) (IDEC and Hoffmann-LaRoche), and Trastuzumab (Herceptin) for breast cancer (Genentech and Hoffmann-LaRoche). A few examples of molecules that are currently undergoing clinical testing include the antibody huN901-DM1 for small cell lung cancer (Immunogen), Bivatuzumab mertansine (MLN2704) for prostate cancer (Millennium), and Pertuzumab (Omnitarg) for prostate, ovarian and breast cancer (Genentech). (See review: Krauss (2003) Mol. Biotechnol 25(1):1-17). Generally these antibodies target specific proteins found on the surface of tumor cells, and work by either eliciting an immunogenic response of some kind against the tumor cell (for example, complement-dependent cytotoxicity (CDC) or antibody dependent cellular cytotoxicity (ADCC)), or the antibodies deliver conjugated cytotoxic entities to the targeted cell following the interaction of the antibody with the tumor cell marker.

Despite the increase in anti-cancer antibody therapeutics that have been approved, there remains a significant unmet need in this area. Some of the approved therapeutics are known to have significant associated toxicities. Many of the adverse reactions are due to non-specific and undesired interaction of the therapeutic antibodies with healthy non-tumor tissues. In addition, not all patients respond to the desired extent using the currently approved antibody therapeutics. Thus the development of therapeutics that target tumor cell antigens with greater selectively for cancer cells would represent a significant advance in the area of cancer therapeutics.

When selecting tumor cell markers to use in the development of anti-cancer antibodies, the most desirable marker would be one that is abundantly and homogeneously expressed only upon cancer cells in the majority of patients for each tumor type, and is not expressed in any other tissue. Unfortunately, markers displaying this level of specificity are few, and thus, markers that show an acceptable amount of an expression differential between healthy tissues and specific cancerous tissues must be exploited. For example, the proteins that the currently approved antibody therapeutics recognize include CD52 (Campath), CD20 (Rituxan, US5736137), Zevalin, Bexxar), HER2 (Herceptin, US5821337), EGFR (Erbitux) and VEGF (Avastatin). Many of these cellular markers are expressed in healthy tissues as well as cancer cells, but often are more highly expressed in cancer when compared to normal tissue. (see Flynn and Byrd (2000) Current Opinion in Oncology 12(6): 574). These tissue specificity considerations are especially important issue for immunoconjugate antibody constructs that may carry potent cytotoxic drugs.

Specific targeting of cancer cells represents a significant advance in the development of anti-cancer therapeutics. Despite the identification of several markers that are associated with some types of cancers, there remains a marked need for additional markers that are more specific to individual cancer types for the development of potent anti-cancer therapeutics that will not damage healthy tissues. Exploitation of these markers in the development of targeting agents via antibody derived cancer therapeutics without harming normal cells in the body would represent an important therapeutic advance in an area with clear and significant unmet medical need.

### SUMMARY OF THE INVENTION

The instant invention provides a method of diagnosis and/or treatment for cancer in a mammal. The method comprises administration of an effective amount of anti-tumor antigen agonist or antagonist, wherein the tumor antigen is defined as being elevated at least three fold in expression relative to normal tissue, and wherein the tumor antigen is selected from the group of KIAA1815, LOC157378, FLJ20421, DSCD75, GPR160, GPCR41, and SLC1A5. In a particular embodiment, the agonist or antagonist is an anti-tumor cell antigen antibody or a small molecule.

In a further embodiment, the invention concerns a method of identifying agonists or antagonists to a tumor cell antigen that comprises contacting the tumor cell antigen with a candidate molecule and monitoring a biological effect mediated by said tumor cell antigen, or mediated by the interaction of said candidate molecule and said tumor cell antigen.

In a still further embodiment, the invention concerns a composition of matter comprising an agonist or antagonist of a tumor cell antigen as herein described, or an anti-tumor cell antigen antibody, in combination with a carrier. Optionally, the carrier is a pharmaceutically acceptable carrier.

Various forms of antibodies are contemplated herein. For example, the anti- tumor antigen antibody may be a full length antibody (e.g. having a human immunoglobulin constant region) or an antibody fragment (e.g. a F(ab'2), Fv or single chain antibody or other functional fragment that specifically binds to the target). Furthermore, the antibody may be labeled with a detectable label, immobilized on a solid phase, and/or conjugated with a heterologous compound (such as a cytotoxic agent). The antibody may be selected from the group of subhuman primate anti-tumor cell antigen antibody, murine monoclonal anti-tumor cell antigen antibody, chimeric anti-tumor cell antigen antibody, human anti-tumor cell antigen antibody, and humanized anti-tumor cell antigen antibody.

The invention provides a method of diagnosing or treating a cancer in a human, wherein the preferred forms of cancer include colon, rectal or colorectal, breast, and prostate cancers comprising administering to the human an effective amount of an antibody that binds to a tumor antigen selected from the group of KIAA1815, LOC157378, FLJ20421, DSCD75, GPR160, GPCR41, and SLC1A5.

Diagnostic uses of the antibodies are contemplated. In one diagnostic application, the invention provides a method for determining the presence of a tumor cell antigen selected from the group of KIAA1815, LOC157378, FLJ20421, DSCD75, GPR160, GPCR41, and SLC1A5 comprising exposing a sample suspected of containing the tumor cell antigen to the antibody of the invention and determining binding of the antibody to the sample. For this use, the invention provides a kit comprising antibody and instructions for using the antibody to detect the tumor cell antigens.

In further embodiments, the invention provides articles of manufacture for use (among other things) in the above methods. For example, the invention provides an article of manufacture comprising a container and a composition contained therein, wherein the composition can be used to treat cancer that expresses one of the tumor cell antigens bound by the agonist/antagonist or antibodies of the invention and further comprising a package insert indicating that the composition can be used to treat cancer which expresses one of the tumor antigens selected from the group of KIAA1815, LOC157378, FLJ20421, DSCD75, GPR160, GPCR41, and SLC1A5.

The invention further provides: isolated nucleic acid encoding the antibody; a vector comprising that nucleic acid, optionally operably linked to control sequences recognized by a host cell transformed with the vector; a host cell comprising that vector; a process for producing the antibody comprising culturing the host cell so that the nucleic acid is expressed and, optionally, further comprising recovering the antibody from the host cell culture (e.g. from the host cell culture medium).

The invention further pertains to an immunoconjugate comprising an antibody that binds to a tumor cell antigen selected from the group of KIAA1815, LOC157378, FLJ20421, DSCD75, GPR160, GPCR41, and SLC1A5 conjugated to one or more heterologous molecules and the use of such conjugates for the treatment of cancer wherein the cancerous cell express one of the tumor antigens selected from KIM1815, LOC157378, FLJ20421, DSCD75, GPR160, GPCR41, and SLC1A5 in a human. The antibody in the immunoconjugate may be an intact antibody (e.g., an intact IgG1 antibody) or an antibody fragment (e.g. a Fab, F(ab)2, diabody etc).

The invention further provides for multivalent, multispecific antibodies or fragments thereof, wherein the multivalent antibody has a binding site with affinity for a tumor cell antigen selected from the group of KIAA1815, LOC157378, FLJ20421, DSCD75, GPR160, GPCR41, and SLC1A5 and one of more additional binding sites.

The invention also pertains to a method of delivering a diagnostic/detection or therapeutic agent to a tumor cell expressing a tumor cell antigen selected from the group of KIAA1815, LOC157378, FU20421, DSCD75, GPR160, GPCR41, and SLC1A5. This method comprised providing the antibody or immunoconjugate composition of the invention and administering it to a patient in need thereof.

Also contemplated by the instant invention is the use of two antibodies or fragments thereof for detection/diagnosis or treatment of cancer, wherein at least one of the antibodies or fragments binds to a tumor cell antigen selected from the group of KIAA1815, LOC157378, FLJ20421, DSCD75, GPR160, GPCR41, and SLC1A5.

One embodiment of the invention is a method of diagnosing, detecting or treating cancer in a mammal, wherein the cancer expresses a tumor cell antigen that is elevated at least 3 fold relative to a normal adjacent tissue sample or pooled normal tissue sample, comprising administering to the mammal therapeutically effective amounts of an antibody or fragment thereof which binds to that tumor cell antigen, and wherein the tumor cell antigen is selected from the group of KIAA181S, LOC157378, FLJ20421, DSCD75, GPR160, GPCR41, and SLC1A5.

In a preferred embodiment, the antibody or fragment thereof is human, humanized, chimeric or fragment thereof.

In yet another embodiment, the antibody or fragment thereof is selected from the group consisting of Fv, F(ab')2, Fab' and Fab.

In an especially preferred embodiment, the antibody or fragment thereof used in the method of diagnosing, detecting or treating cancer in a mammal specifically binds to an extracellular domain of a tumor cell antigen, wherein the tumor cell antigen is selected from the group of KIAA1815, LOC157378, FLJ20421, DSCD75, GPR160, GPCR41, and SLC1A5.

In another embodiment, the antibody or fragment thereof used in the method of diagnosing, detecting or treating cancer in a mammal binds to an extracellular domain of KIAA1815, and the extracellular domain is chosen from the group of amino acids from about positions 1-408, from about positions 474- 489, from about positions 545- 581, from about positions 603- 621, from about positions 642- 653, and from about 674- 904.

In another embodiment, the antibody or fragment there of used in the method of diagnosing, detecting or treating cancer in a mammal binds to an extracellular domain of BC017881, and the extracellular domain is chosen from the group of amino acids from about positions 1-117, from about 140- 148, from about 165- 211, and from about 230- 240.

In another embodiment, the antibody or fragment there of used in the method of diagnosing, detecting or treating cancer in a mammal binds to an extracellular domain of FLJ20421, and the extracellular domain is from about amino acid positions 30- 359.

In another embodiment, the antibody or fragment there of used in the method of diagnosing, detecting or treating cancer in a mammal binds to an extracellular domain of DSCD75, and the extracellular domain is from about amino acid positions 20- 208.

In another embodiment, the antibody or fragment there of used in the method of diagnosing, detecting or treating cancer in a mammal binds to an extracellular domain of GPR160, and the extracellular domain is chosen from the group of amino acids from about positions 1- 15, from about positions 80- 93, from about positions 157- 182, from about positions 263- 276.

In another embodiment, the antibody or fragment there of used in the method of diagnosing, detecting or treating cancer in a mammal binds to an extracellular domain of GPCR41, and the extracellular domain is chosen from the group of amino acids from about positions. 31-49, from about positions 104- 112, from about positions 134- 145, from about positions 170-195, from about positions 212- 270, from about positions 299- 307, from about positions 360- 368, from about positions 390- 403 and from about positions 427- 445.

In yet a further embodiment, the method of diagnosing, detecting or treating cancer in a mammal the antibody or fragment thereof used is an immunoconjugate.

In a preferred embodiment, the diagnostic, detection or therapeutic immunoconjugate comprising an antibody that comprises an anti-tumor cell antigen antibody or fragment thereof or an anti-tumor cell antigen antibody fusion protein or fragment thereof is bound to at least one diagnostic/detection agent or at least one therapeutic agent.

In another embodiment, the diagnostic/detection immunoconjugate comprises a diagnostic/detection agent that comprises at least one photoactive diagnostic/detection agent wherein the photoactive diagnostic agent comprises a chromagen or dye.

In a preferred embodiment, the diagnostic/detection immunoconjugate is used in intraoperative, endoscopic, or intravascular tumor detection/diagnosis.

In a further embodiment, the therapeutic immunoconjugate comprises a therapeutic agent that is selected from the group consisting of a radionuclide, boron, gadolinium or uranium atoms, an immunomodulator, a cytokine, a hormone, a hormone antagonist, an enzyme, an enzyme inhibitor, a photoactive therapeutic agent, a cytotoxic drug, a toxin, an angiogenesis inhibitor, a different antibody and a combination thereof.

In an additional embodiment, the therapeutic immunoconjugate comprises a cytotoxic drug that is a drug, a prodrug, an enzyme or a toxin.

In yet another embodiment, the therapeutic immunoconjugate comprises a cytotoxic drug that is a calicheamicin or calicheamicin analog, a maytansine or maytansine derivative or an auristatin E or auristatin E derivative.

In another embodiment, the therapeutic immunoconjugate comprises a cytotoxic drug that is a toxin or fragment thereof, wherein said toxin is selected from the group consisting of plant, microbial, and animal toxins, and a synthetic variation thereof.

In a further embodiment, the therapeutic immunoconjugate comprises an immunomodulator that is selected from the group consisting of a cytokine, a stem cell growth factor, a lymphotoxin, a hematopoietic factor, a colony stimulating factor (CSF), an interferon (IFN), a stem cell growth factor, erythropoietin, thrombopoietin, an antibody and a combination thereof.

In a preferred embodiment, the therapeutic immunoconjugate comprises an enzyme that is a prodrug-activating enzyme.

In an additional embodiment, the therapeutic immunoconjugate comprises a prodrug-activating enzyme that is selected from the group of alkaline phosphatase , arylsulfatase, cytosine deaminase, proteases, D-alanylcarboxypeptidases, carbohydrate-cleaving enzymes such as .beta.-galactosidase and neuraminidase, beta.-lactamase, penicillin amidases, and antibodies with enzymatic activity.

In an additional embodiment, the multivalent, multispecific antibody or fragment thereof comprises one or more antigen binding sites having affinity toward a tumor cell antigen and one or more epitope binding sites having affinity towards epitopes, wherein said tumor cell antigen is selected from the group of KIAA1815, LOC157378, FLJ20421, DSCD75, GPR160, GPCR41 and SLC1A5.

In a preferred embodiment, the multivalent, multispecific antibody or fragment thereof comprises a multivalent, multispecific antibody or fragment thereof that is human, humanized or chimeric.

In yet another embodiment, the multivalent, multispecific antibody or fragment thereof of comprises a diagnostic/detection or therapeutic agent.

In another embodiment, the antibody fusion protein or fragment thereof comprises at least two anti-tumor cell antigen antibodies or fragments thereof, wherein the antibodies or fragments thereof are selected from said anti-tumor cell antigen antibodies or fragments thereof according to previously described embodiments.

In a preferred embodiment, the method for treating cancer in a mammal comprises treating with a therapeutically effect amount of the antibody or fragment thereof according to any one of the proceeding embodiments, wherein the antibody is formulated in a therapeutically acceptable formulation.

In another embodiment, the method of diagnosing/detecting cancer in a mammal comprises the step of administering to said mammal a diagnostically effective amount of an antibody or fragment thereof according to any of the proceeding embodiments, formulated in a pharmaceutically acceptable vehicle.

In a further embodiment, the method of treating or diagnosing/detecting cancer in a mammal comprises (i) administering to a mammal in need thereof the antibody or fragments thereof according to any one of the proceeding embodiments; (ii) waiting a sufficient amount of time for an amount of the non- binding protein to clear the mammal's bloodstream; and (iii) administering to said mammal a carrier molecule comprising a diagnostic agent, a therapeutic agent, or a combination thereof, that binds to a binding site of said antibody.

In a further embodiment, the method of diagnosing, detecting or treating cancer in a mammal includes a DNA sequence comprising a nucleic acid encoding an anti-tumor cell antigen antibody or fragment thereof or immunoconjugate according to any one of the proceeding embodiments.

In an additional embodiment, the method of diagnosing, detecting or treating cancer in a mammal includes an expression vector comprising the DNA sequence of an anti-tumor cell antigen antibody or fragment thereof or immunoconjugate according to any one of the proceeding embodiments.

In an additional embodiment, the method of diagnosing, detecting or treating cancer in a mammal includes a host cell comprising the expression vector.

In a further embodiment, the method of diagnosing, detecting or treating cancer in a mammal includes a method for delivering a diagnostic/detection or therapeutic agent, or a combination thereof, to a target that comprises (i) providing a composition comprising an immunoconjugate that comprises the antibody or fragment thereof according to any one of the proceeding embodiments and (ii) administering to a mammal in need thereof said composition.

In an additional embodiment, the method of treating cancer in a mammal comprising administering to said mammal a therapeutically effective amount of an antibody or fragment thereof comprises at least two antibodies or fragments thereof, and comprises use of the antibody according to any one of the proceeding embodiments that is formulated in a pharmaceutically suitable excipient.

In an additional embodiment, the method of treating cancer in a mammal that comprises administering to said mammal a therapeutically effective amount of an antibody or fragment thereof further comprises a second antibody or fragment that does not binds to a tumor cell antigen, wherein the tumor cell antigen is selected from the group of KIAA1815, LOC157378, FLJ20421, DSCD75, GPR160, GPCR41, and SLC1A5.

In an additional embodiment, the method of treating cancer in a mammal comprising administering to said mammal a therapeutically effective amount of an antibody or fragment thereof and further comprises a second antibody or fragment that binds to a tumor cell antigen, wherein the tumor cell antigen is selected from the group of KIAA1815, LOC157378, FL720421, DSCD75, GPR160, GPCR41, and SLC1A5.

In another embodiment, the method of treating cancer in a mammal comprising administering to said mammal a therapeutically effective amount of an antibody or fragment thereof and further comprises a second antibody or fragment thereof, where the second antibody is conjugated to a therapeutic or diagnostic/detection agent.

In an additional embodiment, the method of treating cancer in a mammal comprises administering to said mammal a therapeutically effective amount of an antibody or fragment thereof where the anti-tumor cell antigen antibody or fragment thereof is administered before, in conjunction with, or after a second conjugated antibody reactive with a second tumor cell antigen expressed by said malignancy.

In a further embodiment, the method according to any one of the proceeding embodiments comprises use of the anti-tumor cell antigen antibody that is administered in a dosage of 10 ng/kg to up to 100 mg/kg of mammal body weight per dose.

In a still further embodiment, the method according to any one of the proceeding embodiments comprises use of the anti-tumor cell antigen antibody where the dosage is repeatedly administered.

In a preferred embodiment, the method of detecting or diagnosing a breast, prostate or colon cancer in a mammal comprises use of the antibodies or immunoconjugates according to any one the proceeding embodiments.

In a preferred embodiment, the method of treating breast, prostate or colon cancer in a mammal comprises use of the antibodies or immunoconjugates according to any one of the proceeding embodiments.

In a yet further embodiment, the method of treating breast, prostate or colon cancer in a mammal, comprises use of the antibodies or immunoconjugates according to any one of the proceeding embodiments, wherein the tumor cell antigen is selected from the group of KIAA1815, LOC157378, FU20421, DSCD75, GPR160, GPCR41, and SLC1A5.

In another embodiment, the method comprises a kit for the diagnosis or detection of cancer in a mammal, wherein said kit comprises:
a) an antibody or fragment thereof, or an immunoconjugate or fragment thereof, according to any one of the proceeding embodiments, wherein said antibody or fragment is capable of specifically binding a tumor cell antigen wherein said tumor cell antigen is selected from the group of KIAA1815, LOC157378, FU20421, DSCD75, GPR160, GPCR41, and SLC1A5;
b) a detection method enabling said diagnosis or detection of said cancer in said mammal; and
c) instructions for use of the kit.

### BRIEF DESCRIPTION OF THE FIGURES

Figure 1 depicts a graphical representation of the microarray expression analysis of the tumor cell antigen KIAA1815 in both cancerous and normal tissues.
Figure 2 depicts a graphical representation of the microarray expression analysis of the tumor cell antigen LOC157378 in both cancerous and normal tissues.
Figure 3 depicts a graphical representation of the microarray expression analysis of the tumor cell antigen FLJ20421 in both cancerous and normal tissues.
Figure 4 depicts a graphical representation of the microarray expression analysis of the tumor cell antigen GPR160 in both cancerous and normal tissues.
Figure 5 depicts a graphical representation of the microarray expression analysis of the tumor cell antigen GPCR41 in both cancerous and normal tissues.
Figure 6 depicts a graphical representation of the microarray expression analysis of the tumor cell antigen SLC1A5 in both cancerous and normal tissues.

### DETAILED DESCRIPTION OF THE PREFERRED EMBODIMENTS

### I. Definitions

Unless indicated otherwise, the term "KIAA1815" when used herein refers to a protein of unknown function in the human genome wherein the protein was originally known as FLJ23309. An alternate name for the gene locus is LLID 79956 or bA207C16.3. The KIAA181S gene contains a hypothetical aminopetidase domain. KIAA1815 may be derived from any mammal, but preferably from a human. The KIAA1815 may be isolated from a natural source of the molecule or may be produced by synthetic means (e.g., using recombinant DNA technology.) The nucleic acid sequence for human KIAA1815 can be found on the NIH NCBI sequence viewer website under accession number NM_024896, for example. The amino acid sequence can be viewed using the accession number NP_079172. KIAA1815 may also refer to any KIAA1815 variant expressed exclusively in cancer cells wherein the amino acid homology with KIAA1815 expressed in non-cancerous cells may be 80%, 85%, 90%, 95%, 96%, 97%, 98%, 99% or 100%. In an alternate embodiment, KIAA1815 may be the mouse homolog of human KIAA1815 known as D19Wsu12e. Alternate names are Fxna, MGC28280, mFLJ23309 and D19Ertd410e. The nucleic acid sequence for mouse D19Wsu12e can be found on the NIH NCBI sequence viewer website under accession number XM_358328. In yet an alternate embodiment, KIAA1815 may be the rat homolog of the human KIAA1815 gene, known as Fxna. The sequence for rat Fxna may be viewed on the NIH NCBI sequence viewer using the accession number NM_184050. In another embodiment, KIAA1815 may be a primate homolog of the human KIAA1815 gene. For example, the Pan troglodytes (chimpanzee) homolog can be found on the NCBI sequence viewer using the accession number XM_526478 for the nucleic acid sequence, or XP520478 for the amino acid sequence.

"KIAA1815 ECD" refers to the extracellular domain (ECD) of the IQAA1815 tumor cell antigen. KIAA1815 ECD may also refer to any KIAA1815 ECD variant that may be expressed in exclusively in cancer cells. KIAA1815 ECD refers to any protein or peptide wherein the amino acid homology may be 80%, 85%, 90%, 95%, 96%, 97%, 98%, 99% or 100% of the portion of the protein that resides outside of the cell. Topology prediction programs can be used to estimate the regions of the protein that comprise the ECD (see Example 2). In alternate embodiments, the KIAA1815 ECD may be derived from the mouse, rat or primate homolog of the human KIAA1815 ECD.

Unless indicated otherwise, the term "BC017881" when used herein refers to a transmembrane protein of unknown function in the human genome alternately known by the name LOC157378 or TMEM65. BC017881 may be derived from any mammal, but preferably from a human. The BC017881 may be isolated from a natural source of the molecule or may be produced by synthetic means (e.g., using recombinant DNA technology.) The nucleic acid sequence for human BC017881 can be found on the NIH NCBI sequence viewer website under accession number NM_194291, for example. The amino acid sequence may be similarly viewed using the accession number NP_919267. BC017881 may also refer to any BC017881 variant expressed exclusively in cancer cells wherein the homology with BC017881 expressed in non-cancerous cells may be 80%, 85%, 90%, 95%, 96%, 97%, 98%, 99% or 100%. In an alternate embodiment, BC017881 may be the mouse homolog of human BC017881 known as 4930438D12Rik. An alternate name for 4930438D12Rik is 2610029013Rik. The nucleic acid sequence for mouse 4930438D12Rik may be found on the NIH NCBI sequence viewer website under the accession number NM_175212. In yet a further embodiment, BC017881 may be the rat homolog known as LOC500874. The nucleic acid sequence for LOC500874 may be viewed on the NIH NCBI sequence view using the accession number XM_576273. In another embodiment, BC017881 may be a primate homolog of the human BC017881 gene. For example, the Pan troglodytes (chimpanzee) homolog can be found on the NCBI sequence viewer using the accession number XM_528228 for the nucleic acid sequence, or XP 528228 for the amino acid sequence.

"BC017881 ECD" refers to the extracellular domain (ECD) of the BC017881 tumor cell antigen. BC017881 ECD may also refer to any BC017881 ECD variant that may be expressed in exclusively in cancer cells. BC017881 ECD refers to any protein or peptide wherein the amino acid homology may be 80%, 85%, 90%, 95%, 96%, 97%, 98%, 99%, or 100% of the portion of the protein that resides outside of the cell. Topology prediction programs can be used to estimate the regions of the protein that comprise the ECD (see Example 2). In alternate embodiments, the BC017881 ECD may be derived from the mouse, rat or primate homolog of the human BC017881 ECD.

Unless indicated otherwise, the term "FU20421" when used herein refers to a myo-inositol monophosphatase protein of unknown function in the human genome alternately known by the names BAA91158, IMPA3 and Impad1. FLJ20421 has a putative IPPase domain located from about amino acid 62 through amino acid 349. FLJ20421 may be derived from any mammal, but preferably from a human. The FLJ20421 may be isolated from a natural source of the molecule or may be produced by synthetic means (e.g., using recombinant DNA technology.) The nucleic acid sequence for human FLJ20421 can be found on the NIH NCBI sequence viewer website under accession number BC067814. The amino acid sequence may be similarly viewed using the accession number AAH67814, for example. FLJ20421 may also refer to any FLJ20421 variant expressed exclusively in cancer cells wherein the homology with FU20421 expressed in non-cancerous cells may be 80%, 85%, 90%, 95%, 96%, 97%, 98%, 99% or 100%. In an alternate embodiment, FLJ20421 may be the mouse homolog of human FLJ20421 known as Impad1. Impad1 is also known as AA08880, AI451589, AL022796, B23027P20 or 1110001C29Rik. The nucleic acid sequence for mouse Impad1 may be found on the NIH NCBI sequence viewer website under the accession number NM_177730. In yet an alternate embodiment, FU20421 may be the rat homolog of the human gene FLJ20421, known as RGD1306455. The nucleic acid sequence for RGD1306455 may be viewed on the NIH NCBI sequence viewer under accession number XM_575759. In another embodiment, FU20421 may be a primate homolog of the human FU20421 gene. For example, the Pan troglodytes (chimpanzee) homolog can be found on the NCBI sequence viewer using the accession number XM_519770 for the nucleic acid sequence, or XP_519770 for the amino acid sequence.

"FLJ20421 ECD" refers to the extracellular domain (ECD) of the FLJ20421 tumor cell antigen. FLJ20421 ECD may also refer to any FLJ20421 ECD variant that may be expressed in exclusively in cancer cells. FLJ20421 ECD refers to any protein or peptide wherein the amino acid homology may be 80%, 85%, 90%, 95%, 96%, 97%, 98%, 99% or 100% of the portion of the protein that resides outside of the cell. Topology prediction programs can be used to estimate the regions of the protein that comprise the ECD (see Example 2). In alternate embodiments, the FLJ20421 ECD may be derived from the mouse, rat or primate homolog of the human FLJ20421 ECD.

Unless indicated otherwise, the term "DSCD75" when used herein refers to a protein of unknown function expressed in mesenchymal stem cells and contains a FcbC domain which is predicted to have thioesterase function located from about amino acid 54 through amino acid 179. Alternate names for the DSCD75 locus is LOC51337 or C8orf55. DSCD75 may be derived from any mammal, but preferably from a human. The DSCD75 may be isolated from a natural source of the molecule or may be produced by synthetic means (e.g., using recombinant DNA technology.) The nucleic acid sequence for human DSCD75 can be found on the NIH NCBI sequence viewer website under accession number AF242773, for example. The amino acid sequence may be similarly viewed using the accession number AAF65450. DSCD75 may also refer to any DSCD75 variant expressed exclusively in cancer cells wherein the homology with DSCD75 expressed in non-cancerous cells may be 80%, 85%, 90%, 95%, 96%, 97%, 98%, 99% or 100%. In an alternate embodiment, DSCD75 may be the mouse homolog of the human DSCD75 gene, known as 4930572305Rik or MGC54796. The nucleic acid sequence for mouse 4930572J05Rik may be found on the NIH NCBI sequence viewer under the accession number NM_198607. In yet an alternate embodiment, DSCD75 may be the rat homolog of the human DSCD75 gene, known as MGC94661. The nucleic acids sequence for rat DSCD75 may be found on the NIH NCBI sequence viewer under the accession number NM_001007658. In another embodiment, DSCD75 may be a primate homolog of the human DSCD75 gene. For example, the Pan troglodytes (chimpanzee) homolog can be found on the NCBI sequence viewer using the accession number XM_519991 for the nucleic acid sequence, or XP_519991 for the amino acid sequence.

"DSCD75 ECD" refers to the extracellular domain (ECD) of the DSCD75 tumor cell antigen. DSCD75 ECD may also refer to any DSCD75 ECD variant that may be expressed in exclusively in cancer cells. DSCD75 ECD refers to any protein or peptide wherein the amino acid homology may be 80%, 85%, 90%, 95%, 96%, 97%, 98%, 99% or 100% or the portion of the protein that resides outside of the cell. Topology prediction programs can be used to estimate the regions of the protein that comprise the ECD (see Example 2). In alternate embodiments, the DSCD75 ECD may be derived from the mouse, rat or primate homolog of the human DSCD75 ECD.

Unless indicated otherwise, the term "GPR160" when used herein refers to a putative G-protein coupled receptor of unknown function. It was described by Takeda et al along with a series of many other putative G-protein receptors (FEBS Lett. 520 (1-3), 97-101 (2002)). It has also been referred to as GPCR1, or GPCR150, although there is more than one unique human locus known as GPCR1. GPR160 may be derived from any mammal, but preferably from a human. The GPR160 may be isolated from a natural source of the molecule or may be produced by synthetic means (e.g., using recombinant DNA technology.) The nucleic acid sequence for human GPR160 can be found on the NIH NCBI sequence viewer website under accession number NM_014373, for example. The amino acid sequence may be similarly viewed using the accession number NP_055188. GPR160 may also refer to any GPR160 variant expressed exclusively in cancer cells wherein the homology with GPR160 expressed in non-cancerous cells may be 80%, 85%, 90%, 95%, 96%, 97%, 98%, 99% or 100%. In an alternate embodiment, GPCR160 may be the mouse homolog of the human GPCR160 gene, known as Gpr160. The nucleic acid sequence for mouse Gpr160 may be found on the NIH NCBI sequence viewer under the accession number XM_896590. In yet an alternate embodiment, GPR160 may be the rat homolog of the human GPR160 gene, known as Gpr160. The nucleic acids sequence for rat Gpr160 may be found on the NIH NCBI sequence viewer under the accession number NM_001025147. In another embodiment, GPR160 may be a primate homolog of the human GPR160 gene. For example, the Pan troglodytes (chimpanzee) homolog can be found on the NCBI sequence viewer using the accession number XM_530685 for the nucleic acid sequence, or XP_530685 for the amino acid sequence.

"GPR160 ECD" refers to the extracellular domain (ECD) of the GPR160 tumor cell antigen. GPR160 ECD may also refer to any GPR160 ECD variant that may be expressed in exclusively in cancer cells. GPR160 ECD refers to any protein or peptide wherein the amino acid homology may be 80%, 85%, 90%, 95%, 96%, 97%, 98%, 99% or 100% of the portion of the protein that resides outside of the cell. Topology prediction programs can be used to estimate the regions of the protein that comprise the ECD (see Example 2). In alternate embodiments, the GPR160 ECD may be derived from the mouse, rat or primate homolog of the human GPR160 ECD.

Unless indicated otherwise, the term "GPCR41" when used herein refers to a putative G-protein coupled receptor of unknown function. GPCR41 is also known as PAR1 (protease-activated receptor 1) and PERV-A receptor (porcine endogenous retrovirus receptor), D15Ertd747e and GPR172A. It was described by Ericsson et al. as a receptor for pig endogenous retrovirus (Proc. Natl. Acad. Sci. U.S.A. 100 (11), 6759-6764 (2003)). GPCR41 contains a DUF1011 domain from amino acids 265- 371. A DUF domain is a domain of unknown function that is shared by several proteins. GPCR41 may be derived from any mammal, but preferably from a human. The GPCR41 may be isolated from a natural source of the molecule or may be produced by synthetic means (e.g., using recombinant DNA technology.) The nucleic acid sequence for human GPCR41 can be found on the NIH NCBI sequence viewer website under accession number BC002917, for example. The amino acid sequence may be similarly viewed using the accession number AAH02917. GPCR41 may also refer to any GPCR41 variant expressed exclusively in cancer cells wherein the homology with GPCR41 expressed in non-cancerous cells may be 80%, 85%, 90%, 95%, 96%, 97%, 98%, 99% or 100%. In an alternate embodiment, GPRC41 may refer to the mouse homolog of the human GPCR41 gene, known as Gpr172b or by its alternate names GPCR, PAR2, GPCR42, D15Ertd747e or 2010003P03Rik. The sequence for the mouse GPCR41 homolog may be viewed on the NIH NCBI sequence viewer using the accession number NM_029643, for example. In yet another embodiment, GPCR41 may refer to the rat homolog of the human GCPR41 gene, known as LOC362942. The sequence for the rat GCPR41 may be viewed on the NIH NCBI sequence viewer using the accession number XM_343272 for example. In another embodiment, GPCR41 may be a primate homolog of the human GCPR41 gene. For example, the Papio hamadryas (baboon) homolog can be found on the NCBI sequence viewer using the accession number AY070778 for the nucleic acid sequence, or AAL59884 for the amino acid sequence.

"GPCR41 ECD" refers to the extracellular domain (ECD) of the GPCR41 tumor cell antigen. GPCR41 ECD may also refer to any GPCR41 ECD variant that may be expressed in exclusively in cancer cells. GPCR41 ECD refers to any protein or peptide wherein the amino acid homology may be 80%, 85%, 90%, 95%, 96%, 97%, 98%, 99%, or 100% of the portion of the protein that resides outside of the cell. Topology prediction programs can be used to estimate the regions of the protein that comprise the ECD (see Example 2). In alternate embodiments, the GPCR41 ECD may be derived from the mouse, rat or primate homolog of the human GPCR41 ECD.

Unless indicated otherwise, the term "SLC1A5" when used herein refers to a neutral amino acid transporter referred to as ASCT-2 or ATBO and functions as a sodium dependent amino acid transporter that transports neutral amino acids such as alanine, serine, and cysteine. In addition, SLC1A5 serves as a receptor for a number of viruses such as feline endogenous virus, baboon endogenous virus, human endogenous virus type W and type D primate or simian retroviruses associated with infectious immunodeficiencies. It was described by Kekuda et al. as the broad-scope, neutral amino acid transporter Bo and was isolated from a human placental cell line (J. Biol. Chem. 271 (31), 18657-18661 (1996)). SLC1A5 contains a conserved sodium carboxylase symporter (SDF) domain from about amino acids 54 and 485. Other names for SLC1A5 include R16, AAAT, M7V1, RDRC, and M7VS1. SLC1A5 may be derived from any mammal, but preferably from a human. The SLC1A5 may be isolated from a natural source of the molecule, or may be produced by synthetic means (e.g., using recombinant DNA technology.) The nucleic acid sequence for human SLC1A5 can be found on the NIH NCBI sequence viewer website under accession number BC000062, for example. The amino acid sequence may be similarly viewed using accession number AAH00062.1. SLC1A5 may also refer to any SLC1A5 variant expressed exclusively in cancer cells wherein the homology with SLC1A5 expressed in non-cancerous cells may be 80%, 85%, 90%, 95%, 96%, 97%, 98%, 99% or 100%. In an alternate embodiment, SLC1A5 may refer to the mouse homolog of the human SLC1A5 gene, also known as Slc1a5 or alternatively R16, AAAT, ATBO, M7V1, RDRC, ASCT2, M7VS1, Slc1a7 or MGC46952. The sequence for mouse Slc1a5 may be viewed in the NIH NCBI sequence viewer using the accession number NM_009201, for example. In yet an alternate embodiment, Slc1a5 may refer to the rat homolog of human SLC1A5, also known as Slc1a5, or alternatively, Asct2, Slc1a7 or H4-ASCT2. The rat Slc1a5 sequence may be viewed on the NIH NCBI sequence viewer using accession number NM_175758, for example. In another embodiment, SLC1A5 may be a primate homolog of the human SLC1A5 gene. For example, the Macaca fasicularis (long-tailed macaque) homolog can be found on the NCBI sequence viewer using the accession number AB168329 for the nucleic acid sequence, or BAE00453.1 for the amino acid sequence.

"SLC1A5 ECD" refers to the extracellular domain (ECD) of the SLC1A5 tumor cell antigen. SLC1A5 ECD may also refer to any SLC1A5 ECD variant that may be expressed in exclusively in cancer cells. SLC1A5 ECD refers to any protein or peptide wherein the amino acid homology may be 80%, 85%, 90%, 95%, 96%, 97%, 98%, 99% or 100% or the portion of the protein that resides outside of the cell. Topology prediction programs can be used to estimate the regions of the protein that comprise the ECD (see Example 2). In alternate embodiments, the SLC1A5 ECD may be derived from the mouse, rat or primate homolog of the human SLC1A5 ECD.

A "native sequence" polypeptide is one that has the same amino acid sequence as a polypeptide derived from nature. Such native sequence polypeptides can be isolated from nature or can be produced by recombinant or synthetic means. Thus, a native sequence polypeptide can have the amino acid sequence of naturally occurring human polypeptide, murine polypeptide, or polypeptide from any other mammalian species.

The term "amino acid sequence variant" refers to polypeptides having amino acid sequences that differ to some extent from a native sequence polypeptide. Ordinarily, amino acid sequence variants will possess at least about 70% homology with at least one receptor binding domain of a native ligand or with at least one ligand binding domain of a native receptor, and preferably, they will be at least about 80%, more preferably at least about 90% homologous with such receptor or ligand binding domains. The amino acid sequence variants possess substitutions, deletions, and/or insertions at certain positions within the amino acid sequence of the native amino acid sequence.

"Homology" is defined as the percentage of residues in the amino acid sequence variant that are identical after aligning the sequences and introducing gaps, if necessary, to achieve the maximum percent homology. Methods and computer programs for the alignment are well known in the art.

The term "antagonist" is used in the broadest sense, and includes any molecule that partially or fully blocks, inhibits, or neutralizes a biological activity of a tumor cell antigen disclosed herein. In a similar manner, the term "agonist" is used in the broadest sense and includes any molecule that mimics a biological activity of a tumor cell antigen disclosed herein. Suitable agonist or antagonist molecules specifically include agonist or antagonist antibodies or antibody fragments, fragments or amino acid sequence variants of tumor cell antigens, peptides, antisense oligonucleotides, small organic molecules, etc. Methods for identifying agonists or antagonists of a tumor cell antigen may comprise contacting a tumor cell expressing the antigen of interest with a candidate agonist or antagonist molecule and measuring a detectable change in one or more biological activities normally associated with the tumor cell antigen. The antagonist may also be a peptide generated by rational design or by phage display (see, e.g., W098/35036 published 13 August 1998). In one embodiment, the molecule of choice may be a "CDR mimic" or antibody analogue designed based on the CDRs of an antibody. While such peptides may be antagonistic by themselves, the peptide may optionally be fused to a cytotoxic agent so as to add or enhance antagonistic properties of the peptide.

A "small molecule" is defined herein to have a molecular weight below about 500 Daltons.

The term "antibody" herein is used in the broadest sense and specifically covers intact monoclonal antibodies, polyclonal antibodies, multispecific antibodies (e.g. bispecific antibodies) formed from at least two intact antibodies, single chain antibodies, antibodies with modified Fc regions and antibody fragments, so long as they exhibit the desired biological activity, wherein desired biological activity can be defined as having binding specificity for the desired target.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations that include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, e.g., U.S. Pat. No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (e.g. Old World Monkey, Ape etc) and human constant region sequences.

"Antibody fragments" comprise a portion of an intact antibody, preferably comprising the antigen-binding or variable region thereof. Examples of antibody fragments include Fab, Fab', F(ab')2, and Fv fragments; diabodies; linear antibodies (Zapata et al., Protein Eng. 8(10): 1057-1062 [1995]); single-chain antibody molecules; and multispecific antibodies formed from antibody fragment(s).

An "intact" antibody is one that comprises an antigen-binding variable region as well as a light chain constant domain (C_{L}) and heavy chain constant domains, C_{H1}, C_{H2} and C_{H3}. The constant domains may be native sequence constant domains (e.g. human native sequence constant domains) or amino acid sequence variants thereof. Preferably, the intact antibody has one or more effector functions.

Antibody "effector functions" refer to those biological activities attributable to the Fc region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; down regulation of cell surface receptors (e.g. B cell receptor; BCR), etc.

"Antibody-dependent cell-mediated cytotoxicity" or "ADCC" refers to a form of cytotoxicity in which secreted Ig bound onto Fc receptors (FcRs) present on certain cytotoxic cells (e.g., Natural Killer (NK) cells, neutrophils, and macrophages) enable these cytotoxic effector cells to bind specifically to an antigen- bearing target cell and subsequently kill the target cell with cyrtotoxins. The antibodies "arm" the cytotoxic cells and are absolutely required for such killing. The primary cells for mediating ADCC, NK cells, express FcyRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-92 (1991). To assess ADCC activity of a molecule of interest, an in vitro ADCC assay, such as that described in U.S. Pat. No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. (USA) 95:652-656 (1998).

"Human effector cells" are leukocytes that express one or more FcRs and perform effector functions. Preferably, the cells express at least Fc.gamma.RIII and perform ADCC effector function. Examples of human leukocytes that mediate ADCC include peripheral blood mononuclear cells (PBMC), natural killer (NK) cells, monocytes, cytotoxic T cells and neutrophils; with PBMCs and NIC cells being preferred. The effector cells may be isolated from a native source thereof, e.g. from blood or PBMCs as described herein.

The terms "Fc receptor" or "FcR" are used to describe a receptor that binds to the Fc region of an antibody. The preferred FcR is a native sequence human FcR. Moreover, a preferred FcR is one that binds an IgG antibody (a gamma receptor) and includes receptors of the Fc.gamma.RI, Fc.gamma.RII, and Fc.gamma. RIII subclasses, including allelic variants and alternatively spliced forms of these receptors. Fc.gamma.RII receptors include Fc.gamma.RIIA (an "activating receptor") and Fc.gamma.RIIB (an "inhibiting receptor"), which have similar amino acid sequences that differ primarily in the cytoplasmic domains thereof. Activating receptor Fc.gamma.RIIA contains an immunoreceptor tyrosine-based activation motif (ITAM) in its cytoplasmic domain. Inhibiting receptor Fc.gamma.RIIB contains an immunoreceptor tyrosine-based inhibition motif (ITIM) in its cytoplasmic domain. (see review M. in Daron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

"Complement dependent cytotoxicity" or "CDC" refers to the ability of a molecule to lyse a target in the presence of complement. The complement activation pathway is initiated by the binding of the first component of the complement system (Clq) to a molecule (e.g. an antibody) complexed with a cognate antigen. To assess complement activation, a CDC assay, e.g. as described in Gazzano-Santoro et al., J. Immunol. Methods 202:163 (1996), may be performed.

An antibody, oligopeptide or other organic molecule which "induces cell death" is one which causes a viable cell to become nonviable. The cell is one which expresses a cancer cell specific antigen, preferably a cell that overexpresses the cancer antigen as compared to a normal cell of the same tissue type. Preferably, the cell is a cancer cell, e.g., a breast, ovarian, stomach, endometrial, salivary gland, lung, kidney, colon, thyroid, pancreatic or bladder cell. Cell death in vitro may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (i.e., in the absence of functional complement) and in the absence of immune effector cells. To determine whether the antibody, oligopeptide or other organic molecule is able to induce cell death, loss of membrane integrity as evaluated by uptake of propidium iodide (PI), trypan blue (see Moore et al. Cytotechnology 17:1-11 (1995)) or 7AAD can be assessed relative to untreated cells. Preferred cell death- inducing antibodies, oligopeptides or other organic molecules are those which induce PI uptake in the PI uptake assay in at least 20% of the target cells.

An antibody which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). Preferably the cell is a tumor cell, e.g. a breast, ovarian, stomach, endometrial, salivary gland, lung, kidney, colon, thyroid, pancreatic or bladder cell. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the antibody which induces apoptosis is one which results in about 2 to 50 fold, preferably about 5 to 50 fold, and most preferably about 10 to 50 fold, or greater than 50 fold, induction of annexin binding relative to untreated cell in an annexin binding assay using BT474 cells (see below).

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end. The constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

"Naked antibodies" are usually antibodies or fragments thereof that are not linked to or conjugated with any chemical, biological or radionuclide moieties, but may include natural modifications such as glycosylation.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a .beta.-sheet configuration, connected by three hypervariable regions, which form loops connecting, and in some cases forming part of, the .beta.-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, Md. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen binding. The hypervariable region generally comprises amino acid residues from a "complementarity determining region" or "CDR" (e.g. residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, Md. (1991)) and/or those residues from a "hypervariable loop" (e.g. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). "Framework Region" or "FR" residues are those variable domain residues other than the hypervariable region residues as herein defined.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-binding sites and is still capable of cross-linking antigen.

"Fv" is the minimum antibody fragment that contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')2 antibody fragments originally were produced as pairs of Fab' fragments which have hinge cysteines between them. Other chemical couplings of antibody fragments are also known.

The "light chains" of antibodies from any vertebrate species can be assigned to one of two clearly distinct types, called kappa and lambda, based on the amino acid sequences of their constant domains.

Depending on the amino acid sequence of the constant domain of their heavy chains, intact antibodies can be assigned to different "classes". There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called .alpha., .delta., .epsilon., .gamma., and .mu., respectively. The subunit structures and three-dimensional configurations of different classes of immunoglobulins are well known.

"Single-chain Fv" or "scFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains that enables the scFv to form the desired structure for antigen binding. For a review of scFv see Pluckthun in The Pharmacology of Monoclonal Antibodies, vol. 113, Rosenburg and Moore eds., Springer-Verlag, New York, pp. 269-315 (1994). Anti-ErbB2 antibody scFv fragments are described in WO93/16185; U.S. Pat. No. 5,571,894; and U.S. Pat. No. 5,587,458.

The term "diabodies" refers to small antibody fragments with two antigen-binding sites, which fragments comprise a variable heavy domain (V_{H}) connected to a variable light domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

"Multivalent antibodies" are those antibodies or antibody fragments comprising at least two binding sites, wherein the binding sites may both have specificity for the same epitope, or alternately, for two different epitopes.

"Humanized" forms of non-human (e.g., rodent) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody but which may be found for example in other human antibodies. These modifications ("superhumanization) are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

An "isolated" antibody is one that has been identified and separated and/or recovered from a component of its natural environment. Contaminant components of its natural environment are materials that would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coommassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody "which binds" an antigen of interest, is one capable of binding that antigen with sufficient affinity such that the antibody is useful as a therapeutic or diagnostic agent in targeting a cell expressing the antigen.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope. Preferably, an antibody that is specific for a target of interest binds with 1.5-fold, 2-fold, 5-fold, 10-fold, 100-fold, 10³-fold, 10⁴-fold, 10⁵-fold, 10⁶-fold or greater binding to the antigen on a target cell than for other non-specific epitopes. Optionally, the antibodies may demonstrate specificity to, or specific non-binding to any known target tumor cell antigen orthologs, including orthologs in primates, mice and/or rats. Optionally, the antigens may discriminate between the tumor cell antigen of interest and known homologs within the same species. Discrimination may be determined by a specificity of binding for one homolog over another where binding of the antibody to the homolog of interest binds with a 1.5-fold, 2-fold, 5-fold, 10-fold, 100-fold, 10³-fold, 10⁴-fold, 10⁵-fold, 10⁶-fold or greater binding as compared to binding of the antibody to the non-desired homolog. An antibody is said to specifically bind an epitope when the dissociation constant is less than 1 µM, preferably less than 100 nM and most preferably less than 10 nM.

The anti-tumor cell antigen antibodies of the present invention can bind to amino-acid-residues-specific-epitopes, carbohydrate-specific epitopes, or epitopes formed by both amino acid residues and carbohydrate portions of the molecule, as expressed by the target bearing tumor cells. If the anti-tumor cell antigen antibody is carries a cytotoxic or cytotstatic agent, it might be preferable that the antibody binds to an epitope that internalizes the antibody-target receptor complex. If the anti-target antibody is to work through ADCC and CDC, then it is preferable that the anti-target antibody remains on the surface of the target tumor cell until the antibody's Fc region binds to effector cells. Methods for determining whether an antibody bound to a cognate cell surface antigen remains on a cell surface or is internalized are well known in the art.

The term "epitope" refers to the specific binding sites or antigenic determinant on an antigen that the variable end of the antibody binds. Epitopes can be linear, i.e., be composed of a sequence of amino acid residues found in the primary pormin sequence. Epitopes can be conformational, such that an antibody recognizes a 3-D structure found on a folded porimin molecule as expressed on the surface of a porimin expressing cell such that the amino acids recognized are not necessarily contiguous in the primary sequence. Epitopes can also be a combination of linear and conformational elements. Further, carbohydrate portions of a molecule, as expressed by the target bearing tumor cells can also be epitopes.

The term "tumor cell antigen" refers to any protein, carbohydrate or other component capable of eliciting an immune response. The definition is meant to include, but is not limited to, using the whole tumor cell with all of its associated antigens as an antigen, as well as any component separated from the body of the cell, such as plasma membranes, proteins purified from the cell surface or membrane, or unique carbohydrate moieties associated with the cell surface. The definition also includes those antigens from the surface of the cell which require special treatment of the cells to access, "Marker" refers to a tumor cell antigen that is more highly expressed in a tumor cell as compared to the equivalent normal cell.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Pat. No. 4,275,149.

An "antagonist antibody" or "antagonistic antibody" is an antibody that blocks, inhibits or neutralizes a biological activity of a tumor cell antigen as disclosed herein. Methods for identifying an antagonistic antibody of a tumor cell antigen of interest can include contacting a tumor cell that expresses the tumor cell antigen of interest with the candidate antibody, and then measuring a change in the biological activity that is normally associated with that tumor cell antigen in the tumor cell. For example, a tumor cell antigen may be one that induces tumor cell proliferation, and an effective antagonistic antibody would one that interacts specifically with the tumor cell antigen and results in a decrease in cell proliferation. Preferred antagonist antibodies are those that result in a 30- 75% reduction in the biological activity of interest, and more preferred are those that result in a 75- 99.9% reduction or greater in the biological activity of interest.

An antibody that "blocks" ligand activation, or a "blocking antibody" is one type of antagonistic antibody that reduces or prevents activation of the ligand to which the antibody binds. Preferably, a blocking antibody is one that blocks 30- 75% of ligand activation, most preferably is 75- 99.9% or greater of ligand activation when a ligand activation assay measure side by side in the presence or absence of the putative blocking antibody. Prevention of ligand activation can be measured by observing such phenomena as a decrease in receptor phosphorylation or a loss in activation of other components of a signal transduction pathway downstream of the ligand of interest for example.

An "agonist antibody" is one that induces activation of the ligand to which the antibody binds. Preferably, an agonist antibody is one that increases ligand activation 2 fold-, 4 fold-, 10 fold-, 50 fold- or greater when the candidate antibody is compared side by side in a ligand activation assay with a non-specific antibody control. Ligand activation can be measured by observing such phenomena as an increase in receptor phosphorylation or an increase in activation of other components of a signal transduction pathway downstream of the ligand of interest for example.

An antibody having a "biological characteristic" of a designated antibody, is one that possesses one or more of the biological characteristics of that antibody that distinguish it from other antibodies that bind to the same antigen.

A "growth inhibitory agent" when used herein refers to a compound or composition which inhibits growth of a cell, especially a cancer cell either in vitro or in vivo. Thus, the growth inhibitory agent may be one that significantly reduces the percentage of cells in 5 phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (W B Saunders: Philadelphia, 1995), especially p. 13. Preferred growth inhibitory agents are those that block greater than 20% of cell growth in the presence of the growth inhibitor, or greater than 25%, greater than 30%, greater than 35%, greater than 40%, greater than 45%, more preferred are those that block greater than 50% of cell growth, or greater than 55%, greater than 60%, greater than 65%, greater than 70%, greater than 75%, greater than 80%, greater than 85%, greater than 90%, and most preferred are those that block 95%, or greater than 99.9 % of cell growth.

A "cytostatic agent" when used herein refers to a compound or composition which inhibits cell division without causing cell death. A cytostatic agent can inhibit cell progression through the cell cycle and cause division arrest at a specific point in the cell cycle such as prior to S phase for example. Preferred cytostatic agents are those which cause arrest in 30- 50 % of the cells, more preferred are those which cause arrest in 50- 95 % of the cells, and most preferred are those agents that cause arrest in 95 - 99.9 % or greater of the cells in the population.

The terms "treatment", "treating", "treat" and the like are used herein to generally refer to obtaining a desired pharmacologic and/or physiologic effect. "Treatment" refers to both therapeutic treatment and prophylactic or preventative measures. Those in need of treatment include those already with the disorder as well as those in which the disorder is to be prevented. Hence, the mammal to be treated herein may have been diagnosed as having the disorder or may be predisposed or susceptible to the disorder.

An "effective amount" of a polypeptide disclosed herein or an agonist or antagonist thereof is an amount sufficient to carry out a specifically stated purpose. An "effective amount" may be determined empirically and in a routine manner, in relation to the stated purpose.

"Mammal" for purposes of treatment refers to any animal classified as a mammal, including humans, domestic and farm animals, and zoo, sports, or pet animals, such as dogs, horses, cats, cows, etc. Preferably, the mammal is human.

A "disorder" is any condition that would benefit from treatment with the agonists/antagonists or targeting antibody or immunoconjugates of the invention. This includes chronic and acute disorders or diseases including those pathological conditions that predispose the mammal to the disorder in question. Non-limiting examples of disorders to be treated herein include benign and malignant tumors; leukemias and lymphoid malignancies; neuronal, glial, astrocytal, hypothalamic and other glandular, macrophagal, epithelial, stromal and blastocoelic disorders; and inflammatory, angiogenic and immunologic disorders.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to disease progression (TTP) and/or determining the response rate (RR).

By "hyperproliferative disease or disorder" is meant all neoplastic cell growth and proliferation, whether malignant or benign, incuding all transformed cells and tissues and all cancerous cells and tissues. Hyperproliferative diseases or disorders include, but are not limited to, precancerous lesions, abnormal cell growths, benign tumors, malignant tumors, and "cancer." Additional examples of hyperproliferative diseases, disorders, and/or conditions include, but are not limited to neoplasms, whether benign or malignant, located in the: prostate, colon, abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital tract.

The terms "cancer", "neoplasm", "tumor", "cancerous" and "carcinoma" refer to or describe the physiological condition in mammals that is typically characterized by unregulated cell growth. In general, cells of interest for detection or treatment in the present application include precancerous (e.g., benign), malignant, metastatic, and non-metastatic cells. Detection of cancerous cell is of particular interest. Examples of cancer include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

A cancer that "overexpresses" a tumor cell antigen is one that produces significantly higher levels of that antigen compared to a noncancerous cell of the same tissue type. Such overexpression may be caused by gene amplification or by increased transcription or translation. Overexpression of the antigen may be determined diagnostically by evaluating levels of the ligand (or nucleic acid encoding it) in the patient, e.g. in a tumor biopsy or by various diagnostic assays such as the IHC, FISH, southern blotting, PCR or in vivo assays described above.

The term "prognosis" is recognized in the art and encompasses predictions about the likely course of response to therapeutic intervention, and the likely course of disease or disease progression, particularly with respect to likelihood of disease remission, disease relapse, tumor recurrence, metastasis, and death. The ex vivo prognostic assays of the present invention can be used to predict the response of a candidate subject to a particular anti-tumor cell antigen therapeutic agent, or class of anti-tumor cell antigen therapeutic agents, or modulates ADCC. By "predicting the response of a candidate subject" is intended assessing the likelihood that a subject in question will experience a positive or negative outcome with a particular anti-tumor cell antigen therapeutic agent. For purposes of the present invention, "indicative of a positive treatment outcome" in the context of the ex vivo prognostic assays of the present invention is intended to mean an increased likelihood that the candidate subject will experience beneficial results in response to treatment with the anti-tumor cell antigen therapeutic agent under consideration, and thus treatment intervention with that anti-tumor cell antigen therapeutic agent would be warranted. In contrast, "indicative of a negative treatment outcome" is intended to mean an increased likelihood that the patient will not benefit from treatment intervention with the anti-tumor cell antigen therapeutic agent under consideration, and thus treatment intervention with that anti-tumor cell antigen therapeutic agent would not be warranted.

Beneficial results that can be achieved with treatment intervention with anti-tumor cell antigen therapeutic agents include any positive therapeutic response. By "positive therapeutic response" with respect to cancer treatment is intended an improvement in the disease in association with the anti-tumor activity of the anti-tumor cell antigen therapeutic agent and/or an improvement in the symptoms associated with the disease of interest. That is, an anti-proliferative effect, the prevention of further tumor outgrowths, a reduction in tumor size, a reduction in the number of cancer cells, and/or a decrease in one or more symptoms mediated by stimulation of tumor cell antigen-expressing cells can be observed. Thus, for example, a positive therapeutic response would refer to one or more of the following improvements in the disease: (1) a reduction in tumor size; (2) a reduction in the number of cancer (i.e., neoplastic) cells; (3) an increase in neoplastic cell death; (4) inhibition of neoplastic cell survival; (4) inhibition (i.e., slowing to some extent, preferably halting) of tumor growth; (5) inhibition (i.e., slowing to some extent, preferably halting) of cancer cell infiltration into peripheral organs; (6) inhibition (i.e., slowing to some extent, preferably halting) of tumor metastasis; (7) the prevention of further tumor outgrowths; (8) an increased patient survival rate; and (9) some extent of relief from one or more symptoms associated with the cancer. Positive therapeutic responses in any given malignancy can be determined by standardized response criteria specific to that malignancy.

Tumor response can be assessed for changes in tumor morphology (i.e., overall tumor burden, tumor size, and the like) using screening techniques such as magnetic resonance imaging (MRI) scan, x-radiographic imaging, computed tomographic (CT) scan, bone scan imaging, endoscopy, and tumor biopsy sampling including bone marrow aspiration (BMA) and counting of tumor cells in the circulation. In addition to these positive therapeutic responses, the subject undergoing therapy with the anti-tumor cell antigen therapeutic agent may experience the beneficial effect of an improvement in the symptoms associated with the disease. Thus for B cell tumors, the subject may experience a decrease in the so-called B symptoms, i.e., night sweats, fever, weight loss, and/or urticaria. For pre-malignant conditions, therapy with an anti-tumor cell antigen therapeutic agent may block and/or prolong the time before development of a related malignant condition.

In some embodiments, the ex vivo prognostic assays for use in the methods of the present invention comprise providing a test biological sample and a control biological sample from a candidate subject in need of prognosis for treatment intervention with an anti-tumor cell antigen therapeutic agent as noted herein, where the test and control biological samples comprise tumor cell antigen-expressing neoplastic cells that have been stimulated with a tumor cell ligand, either in vivo or ex vivo; contacting the test biological sample with an effective amount of the anti-tumor cell antigen therapeutic agent of interest; detecting the level of at least one biomarker in this test biological sample, where the biomarker is selected from the group consisting of a biomarker of cellular apoptosis, and a biomarker of cell survival, depending upon the mode of action of the anti-tumor cell antigen therapeutic agent of interest; and comparing the level of the biomarker(s) in the test biological sample to the level of the biomarker(s) in the control biological sample, which has not been contacted with the anti-tumor cell antigen therapeutic agent. Where the anti-tumor cell antigen therapeutic agent is an antagonist that blocks or interferes with signaling and also modulates ADCC, the ex vivo prognostic assays disclosed herein for any or all of these biomarkers of cell proliferation and survival, apoptosis, and signaling, can be used to assess the potential beneficial effect of the therapeutic agent, alone or in combination with assays for cytokine markers that are upregulated by signaling, and/or assays for one or more of the tumor cell antigen-related factors described herein, in order to identify a subject having a cancer or pre-malignant condition that would be responsive to treatment with that anti-tumor cell antigen therapeutic agent. Where the anti-tumor cell antigen therapeutic agent has its mode of action via modulating ADCC activity, for example, an anti-tumor cell antigen antibody, the ex vivo prognostic assay for one or more markers of apoptosis can be used to assess the potential beneficial effect of the therapeutic agent, alone or in combination with assays for one or more of the tumor cell antigen-related factors described herein, in order to identify a subject having a cancer or pre-malignant condition that would be responsive to treatment with that anti-tumor cell antigen therapeutic agent.

In accordance with the ex vivo prognostic assays of the invention, expression level of one or more biomarkers, and optionally one or more cytokine markers, in a test biological sample that is contacted with the anti-tumor cell antigen therapeutic agent of interest is compared to expression level for the biomarker(s), and optionally cytokine marker(s) in a control biological sample. By "test biological sample" is intended a biological sample comprising CD40-expressing neoplastic cells obtained from the candidate subject, and which will be contacted with the anti-tumor cell antigen therapeutic agent under consideration for treatment of the candidate subject. By "control biological sample" is intended a biological sample that is comparable to the test biological sample in that it also comprises approximately the same number and kind of tumor cell antigen-expressing neoplastic cells and has been obtained from the candidate subject in the same timeframe and in a manner equivalent to that used to obtain the test biological sample, and which will be subjected to the same experimental conditions as the test sample, but which will not be contacted with the anti-tumor cell antigen therapeutic agent of interest. The test biological sample and control biological sample can be provided from a single biological sample that has been obtained from the subject and divided into subsamples, one of which is designated the test biological sample and another of which is designated the control biological sample. Alternatively, the test biological sample and control biological sample can be provided from two or more biological samples, which can be pooled and then subdivided into subsamples as above, or which can individually represent the test and control biological samples.

The term "cytotoxic agent" as used herein refers to a substance that inhibits or prevents the function of cells and/or causes destruction of cells. These agents may be antimitotic, alkylating, antirnetabolite, angiogenesis-inhibiting, apoptotic, alkaloid, COS-2 inhibiting and antibiotic compounds and combinations thereof. The term is also intended to include radioactive isotopes (e.g. ²²⁵Ac, ¹⁸F, ⁶⁸Ga, ⁶⁷Ga, ⁹⁰Y, ⁸⁶Y, ¹¹¹In, ¹³¹I, ¹²⁵I, ¹²³I, ^{99m}TC, ^{94m}Tc, ⁸⁶Re, ¹⁸⁸Re, ¹⁷⁷Lu, ⁶²Cu, ⁶⁴Cu, ⁶⁷Cu, ²¹²Bi, ²¹³Bi, ³²P, ¹¹C, ¹³N, 15O, ⁷⁶Br, and ²¹¹At. Other radionuclides are also available as diagnostic and therapeutic agents, especially those in the energy range of 20- 10,000 keV), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of cancer. Examples of chemotherapeutic agents include alkylating agents such as thiotepa and cyclosphosphamide (CYTOXAN.TM.); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranirnustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK.RTM.; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxel (TAXOL.RTM., Bristol-Myers Squibb Oncology, Princeton, N.J.) and docetaxel (TAXOTERE.RTM., Rhne-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

An "anti-angiogenic agent" refers to a compound that blocks, or interferes with to some degree, the development of blood vessels. The anti-angiogenic factor may, for instance, be a small molecule or antibody that binds to a growth factor or growth factor receptor involved in promoting angiogenesis.

The term "cytokine" is a generic term for proteins released by one cell population that act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-.alpha. and -.beta.; mullerian-inhibiting substance; mouse gonadotropin-associated peptide; inhibin; activin; vascular endothelial growth factor; integrin; thrombopoietin (TPO); nerve growth factors such as NGF-.beta.; platelet-growth factor; transforming growth factors (TGFs) such as TGF-.alpha. and TGF-.beta.; insulin-like growth factor-I and -II; erythropoietin (EPO); osteoinductive factors; interferons such as interferon-.alpha., -.beta., and -.gamma.; colony stimulating factors (CSFs) such as tnacrophage-CSF (M-CSF); granulocyte-macrophage-CSF (GM-CSF); and granulocyte-CSF (G-CSF); interleukins (ILs) such as IL-1, IL-1.alpha., IL-2, IL-3, IL-4, IL-5, IL-6, IL-7, IL-8, IL-9, IL-10, IL-11, IL-12; a tumor necrosis factor such as TNF-.alpha. or TNF-.beta.; and other polypeptide factors including LIF and kit ligand (KL). As used herein, the term cytokine includes proteins from natural sources or from recombinant cell culture and biologically active equivalents of the native sequence cytokines.

Detection of the biomarker of interest at the protein or nucleotide level can be accomplished using any detection method known to those of skill in the art. By "detecting expression" or "detecting the level of" is intended determining the quantity or presence of a biomarker protein or gene in the biological sample. Thus, "detecting expression" encompasses instances where a biomarker is determined not to be expressed, not to be detectably expressed, expressed at a low level, expressed at a normal level, or overexpressed. In order to determine the effect of an anti-tumor cell antigen therapeutic, a test biological sample comprising tumor cell antigen-expressing neoplastic cells that is contacted with the anti-tumor cell antigen therapeutic agent for a sufficient time to allow the therapeutic agent to exert a cellular response, and then expression level of one or more biomarkers of interest in that test biological sample is compared to the expression level in the control biological sample that has not been contacted with the anti-tumor cell antigen therapeutic agent. In some embodiments, the control biological sample of neoplastic cells is contacted with a neutral substance or negative control. For example, in one embodiment, a non-specific immunoglobulin, for example IgG1, which does not bind to tumor cell antigen serves as the negative control. Detection can occur over a time course to allow for monitoring of changes in biomarkers over time. Detection can also occur with exposure to different concentrations of the anti-tumor cell antigen therapeutic agent to generate a "dose-response" curve for any given biomarker of interest.

The term "prodrug" as used in this application refers to a precursor or derivative form of a pharmaceutically active substance that is less cytotoxic to tumor cells compared to the parent drug and is capable of being enzymatically activated or converted into the more active parent form. See, e.g., Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, .beta.-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

A "liposome" is a small vesicle composed of various types of lipids, phospholipids and/or surfactant that is useful for delivery of a drug (such as the anti-tumor cell antigen antibodies disclosed herein and, optionally, a chemotherapeutic agent) to a mammal. The components of the liposome are commonly arranged in a bilayer formation, similar to the lipid arrangement of biological membranes.

The term "package insert" is used to refer to instructions customarily included in commercial packages of therapeutic products, that contain information about the indications, usage, dosage, administration, contraindications and/or warnings concerning the use of such therapeutic products.

An "isolated" nucleic acid molecule is a nucleic acid molecule that is identified and separated from at least one contaminant nucleic acid molecule with which it is ordinarily associated in the natural source of the antibody nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the antibody where, for example, the nucleic acid molecule is in a chromosomal location different from that of natural cells.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

### II. Screening for agonists or antagonists of tumor cell antigens

To assay for antagonists, the tumor cell antigen may be added to a cell along with the compound to be screened for a particular activity and the ability of the compound to inhibit the activity of interest in the presence of the tumor cell antigen indicates that the compound is an antagonist to the tumor cell antigen. Alternatively, antagonists may be detected by combining the tumor cell antigen and a potential antagonist with membrane-bound tumor cell antigen receptors or recombinant receptors under appropriate conditions for a competitive inhibition assay. The tumor cell antigen can be labeled, such as by radioactivity, such that the number of tumor cell antigen molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2) : Chapter 5 (1991). Preferably, expression cloning is employed wherein polyadenylated RNA is prepared from a cell responsive to the tumor cell antigen and a cDNA library created from this RNA is divided into pools and used to transfect COS cells or other cells that are not responsive to the tumor cell antigen. Transfected cells that are grown on glass slides are exposed to labeled tumor cell antigen. The tumor cell antigen can be labeled by a variety of means including iodination or inclusion of a recognition site for a site-specific protein kinase. Following fixation and incubation, the slides are subjected to autoradiographic analysis. Positive pools are identified and sub-pools are prepared and re- transfected using an interactive sub-pooling and re-screening process, eventually yielding a single clone that encodes the putative receptor.

As an alternative approach for receptor identification, labeled tumor cell antigen can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro sequencing would be used to design a set of degenerate oligonucleotide probes to screen a cDNA library to identify the gene encoding the putative receptor.

In another assay for antagonists, mammalian cells or a membrane preparation expressing the receptor would be incubated with labeled tumor cell antigen in the presence of the candidate compound. The ability of the compound to enhance or block this interaction could then be measured.

More specific examples of potential antagonists include an oligonucleotide that binds to the fusions of immunoglobulin with tumor cell antigen, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the tumor cell antigen that recognizes the receptor but imparts no effect, thereby competitively inhibiting the action of the tumor cell antigen.

Another potential tumor cell antigen antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5'coding portion of the polynucleotide sequence, which encodes the mature tumor cell antigens herein, is used to design an antisense RNA oligonucleotide of from about 10 to 40 base pairs in length. A DNA oligonucleotide is designed to be complementary to a region of the gene involved in transcription (triple helix--see Lee et al., Nucl. Acids Res., 6:3073 (1979); Cooney et al., Science, 241: 456 (1988); Dervan et al., Science, 251:1360 (1991)), thereby preventing transcription and the production of the tumor cell antigen. The antisense RNA oligonucleotide hybridizes to the mRNA in vivo and blocks translation of the mRNA molecule into the tumor cell antigen (antisense--Okano, Neurochem., 56:560 (1991); Oligodeoxynucleotides as Antisense Inhibitors of Gene Expression (CRC Press: Boca Raton, Fla., 1988). The oligonucleotides described above can also be delivered to cells such that the antisense RNA or DNA may be expressed in vivo to inhibit production of the tumor cell antigen. When antisense DNA is used, oligodeoxyribonucleotides derived from the translation-initiation site, e. g., between about -10 and +10 positions of the target gene nucleotide sequence, are preferred.

Another method of tumor cell antigen antagonism is the use of siRNA technology wherein single stranded small inteferring RNAs (siRNAs: 19- 29 nucleotides in length) are used to silence genes wherein the siRNAs serve to guide cleavage of the target antigen's mRNA (see Martinez et al, Cell (2002) 110(5): 563- 74).

Potential antagonists include small molecules that bind to the active site, the receptor binding site, or growth factor or other relevant binding site of the tumor cell antigen, thereby blocking the normal biological activity of the tumor cell antigen. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence- specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994), and PCT publication No. WO 97133551 (published Sep. 18, 1997).

Nucleic acid molecules in triple-helix formation used to Inhibit transcription should be single-stranded and composed of deoxynucleotides. The base composition of these oligonucleotides is designed such that it promotes triple-helix formation via Hoogsteen base- pairing rules, which generally require sizeable stretches of purines or pyrimidines on one strand of a duplex. For further details see, e.g., PCT publication No. WO 97/33551, supra.

These small molecules can be identified by any one or more of the screening assays discussed hereinabove and/or by any other screening techniques well known for those skilled in the art.

### III. Production of Anti-tumor cell antigen Antibodies

A description follows as to exemplary techniques for the production of the antibodies used in accordance with the present invention. The tumor cell antigen to be used for production of antibodies may be, e.g., a soluble form of the extracellular domain of the tumor cell antigen or a portion thereof, containing the desired epitope. Alternatively, cells expressing the tumor cell antigen at their cell surface can be used to generate antibodies. In another embodiment, DNA-based immunization using the gene encoding the tumor cell antigen may be use to generate antibodies (see Ramos J.D.A. et al Allergy, Volume 59, Number 5, May 2004, pp. 539-547(9)). Other forms of the tumor cell antigen useful for generating antibodies will be apparent to those skilled in the arts

### (i) Polyclonal Antibodies

The anti-tumor cell antigen antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the tumor cell antigen or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants that may be employed include Freund's complete adjuvant and MPL-TDM adjuvant (monophosphoryl Lipid A, synthetic trehalose dicorynomycolate). The immunization protocol may be selected by one skilled in the art without undue experimentation.

### (ii) Monoclonal Antibodies

Monoclonal antibodies are obtained from a population of substantially homogeneous antibodies, i.e., the individual antibodies comprising the population are identical except for possible naturally occurring mutations that may be present in minor amounts. Thus, the modifier "monoclonal" indicates the character of the antibody as not being a mixture of discrete antibodies.

Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized in vitro.

The immunizing agent will typically include the tumor cell antigen polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPR™), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, Calif. and the American Type Culture Collection, Manassas, Va. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcet Dekker, Inc., New York, (1987) pp. 51-63].

The culture medium in which the hybridoma cells are cultured can then be assayed for the presence of monoclonal antibodies directed against tumor cell antigens. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Pat. No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Pat. No. 4,816,567; Morrison et al., supra]or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown in vivo as ascites in a mammal. Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPMI-1640 medium. In addition, the hybridoma cells may be grown in vivo as ascites tumors in an animal.

The binding affinity of the monoclonal antibody can, for example, be determined by the Scatchard analysis of Munson et al., Anal. Biochem., 107:220 (1980).

The monoclonal antibodies secreted by the subclones are suitably separated from the culture medium, ascites fluid, or serum by conventional antibody purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Pluckthun, Immunol. Revs., 130:151-188 (1992).

In a further embodiment, monoclonal antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy chain and light chain constant domains in place of the homologous murine sequences (U.S. Pat. No. 4,816,567; and Morrison, et al., Proc. Natl Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (iii) Humanized Antibodies

The anti-tumor cell antigen antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues that are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies have been described in the art. Generally, a humanized antibody has one or more amino acid residues introduced into it from a source that is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al, Science, 239:1534-1536 (1988)), by substituting hypervariable region sequences for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence that is closest to that of the rodent is then accepted as the human framework region (FR) for the humanized antibody (Sims et al., J. Immunol., 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

An alternative method of antibody humanization relies on identifying those amino acids in an antibody variable domain that may be modified without diminishing the native affinity of the domain for antigen while reducing the antibody's immunogenicity with respect to heterologous species. The amino acids that are modified are those that are not critical for antigen binding, but may be exposed to immunogenicity-stimulating factors (see for example WO931179).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

Various forms of the humanized antibody or affinity matured antibody are contemplated. For example, the humanized antibody or affinity matured antibody may be an antibody fragment, such as a Fab, which is optionally conjugated with one or more cytotoxic agent(s) in order to generate an immunoconjugate. Alternatively, the humanized antibody or affinity matured antibody may be an intact antibody, such as an intact IgG1 antibody.

### (iv) Human Antibodies

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggermann et al., Year in Immuno., 7:33 (1993); and U.S. Pat. Nos. 5,591,669, 5,589,369 and 5,545,807.

Alternatively, phage display technology (McCafferty et al., Nature 348:552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particles. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats; for their review see, e.g., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffth et al., EMBO J. 12:725-734 (1993). See, also, U.S. Pat. Nos. 5,565,332 and 5,573,905.

As discussed above, human antibodies may also be generated by in vitro activated B cells (see U.S. Pat. Nos. 5,567,610 and 5,229,275).

### (v) Antibody Fragments

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; U.S. Pat. No. 5,571,894; and U.S. Pat. No. 5,587,458. The antibody fragment may also be a "linear antibody", e.g., as described in U.S. Pat. No. 5,641,870 for example. Such linear antibody fragments may be monospecific or bispecific.

Binding domain-immunoglobulin fusion proteins are also contemplated. In these constructs, fusion proteins are constructed that comprise the binding domain for an antigen of interest fused to an antibody hinge region and immunoglobulin CH2 and CH3 domains that are capable of ADCC and/or CDC while existing predominantly as monomers due to an impaired ability to form disulfide-linked multimers (see US20030118592). These fusion proteins can be produced recombinantly at high levels.

### (vi) Bispecific Antibodies

Bispecific antibodies are antibodies that have binding specificities for at least two different epitopes. Exemplary bispecific antibodies may bind to two different epitopes of the tumor antigen protein. Alternatively, an anti-tumor cell antigen arm may be combined with an arm that binds to a triggering molecule on a leukocyte such as a T-cell receptor molecule (e.g. CD2 or CD3), or Fc receptors for IgG (Fc.gamma.R), such as Fc.gamma.RI (CD64), Fc.gamma.RII (CD32) and Fc.gamma.RIII (CD16) so as to focus cellular defense mechanisms to the tumor antigen-expressing cell. Bispecific antibodies may also be used to localize cytotoxic agents to cells that express tumor antigens. These antibodies possess a tumor antigen-binding arm and an arm that binds the cytotoxic agent (e.g. saporin, anti-interferon-.alpha., vinca alkaloid, ricin A chain, methotrexate or radioactive isotope hapten). Bispecific antibodies can be prepared as full length antibodies or antibody fragments (e.g. F(ab')₂ bispecific antibodies).

Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

Antibody variable domains with the desired binding specificities (antibody-antigen combining sites) are fused to immunoglobulin constant domain sequences. The fusion preferably is with an immunoglobulin heavy chain constant domain, comprising at least part of the hinge, C_{H2}, and C_{H3} regions. It is preferred to have the first heavy-chain constant region (C_{H1}) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. Heteroconjugate antibodies are composed of two covalently joined antibodies. Such antibodies have, for example, been proposed to target immune system cells to unwanted cells (U.S. Pat. No. 4,676,980), and for treatment of HIV infection (WO 91/00360, WO 92/200373, and EP 03089). It is contemplated that the antibodies may be prepared in vitro using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Pat. No. 4,676,980.

Bispecific antibodies can be prepared as full length antibodies or antibody fragments (e.g. F(ab')2 bispecific antibodies). Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab' TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab'-SH fragments may be directly recovered from *E. coli*, which can be chemically coupled to form bispecific antibodies. Shalaby et al., J. Exp. Med., 175: 217-225 (1992) describe the production of a fully humanized bispecific antibody F(ab')₂ molecule. Each Fab' fragment was separately secreted from *E. coli* and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecifc antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (V_{H}) connected to a light-chain variable domain (V_{L}) by a linker that is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

Exemplary bispecific antibodies may bind to two different epitopes on a given tumor cell antigen polypeptide herein. Alternatively, an anti-tumor cell antigen arm may be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular tumor cell antigen. Bispecific antibodies may also be used to localize cytotoxic agents to cells which express a particular tumor cell antigen. These antibodies possess a tumor cell antigen-binding arm and an arm which binds a cytotoxic agent or a radionuclide chelator, such as EOTUBE, DPTA, DOTA, or TETA. Another bispecific antibody of interest binds the tumor cell antigen and further binds tissue factor (TF).

### (vii) Other Amino Acid Sequence Modifications

Amino acid sequence modification(s) of the anti-tumor cell antigen antibodies described herein are contemplated. For example, it may be desirable to improve the binding affinity and/or other biological properties of the antibody. Amino acid sequence variants of the anti-tumor cell antigen antibody are prepared by introducing appropriate nucleotide changes into the anti-tumor cell antigen antibody nucleic acid, or by peptide synthesis. Such modifications include, for example, deletions from, and/or insertions into and/or substitutions of, residues within the amino acid sequences of the anti-tumor cell antigen antibody. Any combination of deletion, insertion, and substitution is made to arrive at the final construct, provided that the final construct possesses the desired characteristics. The amino acid changes also may alter post-translational processes of the anti-tumor cell antigen antibody, such as changing the number or position of glycosylation sites.

A useful method for identification of certain residues or regions of the anti-tumor cell antigen antibody that are preferred locations for mutagenesis is called "alanine scanning mutagenesis" as described by Cunningham and Wells Science, 244:1081-1085 (1989). Here, a residue or group of target residues are identified (e.g., charged residues such as arg, asp, his, lys, and glu) and replaced by a neutral or negatively charged amino acid (most preferably alanine or polyalanine) to affect the interaction of the amino acids with tumor cell antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed anti-tumor cell antibody variants are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an anti-tumor cell antigen antibody with an N-terminal methionyl residue or the antibody fused to a cytotoxic polypeptide. Other insertional variants of the anti-tumor cell antigen antibody molecule include the fusion to the N- or C-terminus of the anti-tumor cell antigen antibody to an enzyme (e.g. for ADEPT) or a polypeptide that increases the serum half-life of the antibody.

Another type of variant is an amino acid substitution variant. These variants have at least one amino acid residue in the anti-tumor cell antigen antibody molecule replaced by a different residue. The sites of greatest interest for substitutional mutagenesis include the hypervariable regions, but FR alterations are also contemplated. Conservative substitutions are shown in Table 1 under the heading of "preferred substitutions". If such substitutions result in a change in biological activity, then more substantial changes, denominated "exemplary substitutions" in Table 1, or as further described below in reference to amino acid classes, may be introduced and the products screened.

**TABLE 1**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; asp, lys; arg | gln |
| Asp (D) | glu; asn | glu |
| Cys (C) | ser; ala | ser |
| Gln (Q) | asn, glu | asn |
| Glu (E) | asp; gln | asp |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | tyr |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
1) hydrophobic: norleucine, met, ala, val, leu, ile;
2) neutral hydrophilic: cys, ser, thr;
3) acidic: asp, glu;
4) basic: asn, gln, his, lys, arg;
5) residues that influence chain orientation: gly, pro; and
6) aromatic: trp, tyr, phe.

Non-conservative substitutions will entail exchanging a member of one of these classes for another class.

Such substituted residues also may be introduced into the conservative substitution sites or, more preferably, into the remaining (non-conserved) sites.

Any cysteine residue not involved in maintaining the proper conformation of the anti-tumor cell antigen antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

A particularly preferred type of substitutional variant involves substituting one or more hypervariable region residues of a parent antibody (e.g. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (e.g. 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g. binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and human tumor cell antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antibody alters the original glycosylation pattern of the antibody. By altering is meant deleting one or more carbohydrate moieties found in the antibody, and/or adding one or more glycosylation sites that are not present in the antibody.

Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Nucleic acid molecules encoding amino acid sequence variants of the anti-tumor cell antigen antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the anti-tumor cell antigen antibody.

To increase the serum half life of the antibody, one may incorporate a salvage receptor binding epitope into the antibody (especially an antibody fragment) as described in U.S. Pat. No. 5,739,277, for example. As used herein, the term "salvage receptor binding epitope" refers to an epitope of the Fc region of an IgG molecule (e.g., IgG₁, IgG₂, IgG₃, or IgG₄) that is responsible for increasing the in vivo serum half-life of the IgG molecule.

### (viii) Affinity Maturation

It may be necessary to increase the affinity of antibodies of the invention, and there are many methods known in the art to accomplish this task. For example, focused, step-by-step mutagenesis can be carried out on using phage-expressed antibody libraries wherein all six heavy and light chain CDRs containing single mutations are expressed and screened for increased antigen affinity (see Wu et al, (1998) Proc Natl Acad Sci USA 95(11):6037-42). Another focused approach is termed "hot-spot mutagenesis", where randomly introduce mutations are introduced at sites that are most likely to generate improved affinity (see Ho et al, (2005) J. Biol. Chem. 280:607-17). Focused approaches such as these have an advantage in that only small libraries are needed for screening, but have a disadvantage in that later, extensive screening of all the variant combinations is required to obtain the best clones.

A second general approach for carrying out antibody affinity maturation relies on use of one of the several different types of display technologies. In these techniques, millions of antibody variants are displayed and selected under conditions that favor those variants with improved affinity. Display systems are often paired with some method for introducing random mutants such as error-prone PCR mutagenesis. Display technologies that may be used include, but are not limited to, ribosome-, yeast-, phage-, microbead-, protein-DNA linkage-, bacterial- and retroviral-display. Using these techniques, 1000X improvements in antibody affinity have been reported, generating antibodies with affinities as low as 48 fM. For review, please see Hoogenboom (2005) Nature Biotechnology 23:1105-16.

An alternate approach, termed "look-through mutagenesis" is a multidimensional mutagenesis approach that simultaneously assesses and optimizes combinatorial mutations by examining the distribution of chemistries within a CDR domain and addressing the synergistic contribution of amino acid side chain chemistry (see Rajpal et al (2005) Proc Natl Acad Sci USA 102(24): 8466-71).

### (ix) Effector Function Engineering

It may be desirable to modify the antibody of the invention with respect to effector function, e.g. so as to enhance antigen-dependent cell-mediated cyotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region (For review: Weiner and Carter (2005) Nature Biotechnology 23(5): 556-557). The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al. Anti-Cancer Drug Design 3:219-230 (1989). Antibodies can be produced with modified glycosylation within the Fc region. For example, lowering the fucose content in the carbohydrate chains may improve the antibody's intrinsic ADCC activity (see for example BioWa's Potillegent^{™} ADCC Enhancing Technology, described in W00061739). Alternately, antibodies can be produced in cell lines that add bisected non-fucosylated oligosaccharide chains (see US 6,602,684). Both these technologies produce antibodies with an increased affinity for the FcgammaIIIa receptor on effector cells which results in increased ADCC efficiency. The Fc region can also be engineered to alter the serum half life of the antibodies of the invention. Abdegs are engineered IgGs with an increased affinity for the FcRn salvage receptor, and so have shorter half life than conventional IgGs (see Vaccaro et al, (2005) Nature Biotechnology 23(10): 1283- 1288). To increase serum half life, specific mutations can be introduced into the Fc region that appear to decrease the affinity with FcRn (see Hinton et al, (2004) J Biol Chem 297(8): 6213-6216). Antibodies of the invention can also be modified to use other mechanisms to alter serum half life, such as including a serum albumin binding domain (dAb) (see WO0S035572 for example). Engineered Fc domains (see for example XmAB™, W005077981) may also be incorporated into the antibodies of the invention to lead to improved ADCC activity, altered serum half life or increased antibody protein stability.

### (x) Immunoconjugates

The invention also pertains to immunoconjugates comprising an antibody conjugated to a cytotoxic agent such as a chemotherapeutic agent, toxin (e.g. an enzymatically active toxin or an enzymatically active toxin of bacterial, fungal, plant or animal origin, including fragments and/or variants thereof), prodrug or to another agent such as an immunomodulator, a hormone or hormone antagonist, and enzyme or enzyme inhibitor, a photoactive therapeutic agent such as a chromagen or dye, an angiogenesis inhibitor, an alternate antibody or fragment thereof, or a radioactive isotope (i.e., a radioconjugate) (for review, see Schrama et al, (2006) Nature Reviews 5: 147- 159).

Chemotherapeutic agents useful in the generation of such immunoconjugates have been described above. Conjugates of an antibody and one or more small molecule toxins, such as a calicheamicin, a maytansine (U.S. Pat. No. 5,208,020), a trichothene, the duocarmycins (also known as 'Ultra Potent Toxins'; see generally Lillo et al, (2004) Chemistry and Biology 11; p. 897- 906) and CC-1065 are also contemplated herein.

In one preferred embodiment of the invention, the antibody is conjugated to one or more maytansine molecules (e.g. about 1 to about 10 maytansine molecules per antibody molecule). Maytansine may, for example, be converted to May-SS-Me which may be reduced to May-SH3 and reacted with modified antibody (Chari et al. Cancer Research 52: 127-131 (1992)) to generate a maytansinoid-antibody immunoconjugate. Alternately, the drug selected may be the highly potent maytansine derivative DM1 (N2'-deacetyl-N2'-(3-mercapto-1-oxopropyl)-maytansine) (see for example WO02/098883 published Dec. 12, 2002) which has an IC50 of approximately 10⁻¹¹ M (review, see Payne (2003) Cancer Cell 3:207- 212) or DM4 (N²'-deacetyl-N²'(4-methyl- -4-mercapto-1-oxopentyl)-maytansine) (see for example W02004/103272 published Dec. 2, 2004)

Another immunoconjugate of interest comprises an anti-tumor cell antigen antibody conjugated to one or more calicheamicin molecules. The calicheamicin family of antibiotics are capable of producing double-stranded DNA breaks at sub-picomolar concentrations. Structural analogues of calicheamicin which may be used include, but are not limited to, .gamma..sub.1.sup.I, .alpha..sub.2.sup.I, .alpha..sub.3.sup.I, N-acetyl-.gamma..sub.1.sup.I, PSAG and .theta..sup.I.sub.1 (Hinman et al. Cancer Research 53: 3336-3342 (1993) and Lode et al. Cancer Research 58: 2925-2928 (1998)). See, also, U.S. Pat. Nos. 5,714,586; 5,712,374; 5,264,586; and 5,773,001 expressly incorporated herein by reference.

Still another approach involves conjugating the tumor cell antigen antibody to a prodrug, capable of being release in its active form by enzymes overproduced in many cancers. For example, antibody conjugates can be made with a prodrug form of doxorubicin wherein the active component is released from the conjugate by plasmin. Plasmin is known to be over produced in many cancerous tissues (see Decy et al, (2004) FASEB Journal 18(3): 565-567).

Enzymatically active toxins and fragments thereof which can be used include diphtheria A chain, nonbinding active fragments of diphtheria toxin, exotoxin A chain (from *Pseudomonas* aeruginosa),Pseudomonas endotoxin, ricin A chain, abrin A chain, modeccin A chain, alphasarcin, *Aleurites fordii* proteins*,* dianthin proteins, *Phytolaca americana* proteins (PAPI, PAPII, and PAP-S), Ribonuclease (Rnase), Deoxyribonuclease (Dnase), pokeweed antiviral protein, momordica charantia inhibitor, curcin, crotin, sapaonaria officinalis inhibitor, gelonin, mitogellin, restrictocin, phenomycin, neomycin and the tricothecenes. See, for example, WO 93/21232 published Oct. 28, 1993. Particularly preferred are toxins that have low intrinsic immunogenicity and a mechanism of action (e.g. a cytotoxic mechanism versus a cytostatic mechanism) that reduces the opportunity for the cancerous cells to become resistant to the toxin.

Conjugates made between the antibodies of the invention and immunomodulators are contemplated. For example, immunostimulatory oligonucleotides can be used. These molecules are potent immunogens that can elicit antigen-specific antibody responses (see Datta et al, (2003) Ann N.Y. Acad. Sci 1002: 105-111). Additional immunomodulatory compounds can include stem cell growth factor such as "S1 factor", lymphotoxins such as tumor necrosis factor (TNF), hematopoietic factor such as an interleukin, colony stimulating factor (CSF) such as granulocyte-colony stimulating factor (G-CSF) or granulocyte macrophage-stimulating factor (GM-CSF), interferon (IFN) such as interferon alpha, beta or gamma, erythropoietin, and thrombopoietin.

A variety of radioactive isotopes are available for the production of radioconjugated anti-tumor cell antigen antibodies. Therapeutic radioconjugates can be made using P-32, P-33, Sc-47, Fe-59, Cu-64, Cu-67, Se-75, As-77, Sr-89, Y-90, Mo-99, Rh-105, Pd-109, Ag-Il I , I-125,1-131, Pr-142, Pr-143, Pm-149, Sm-153, Th-161, Ho-166, Er-169, Lu-177, Re-186, Re-188, Re-189, Ir-194, Au-198, Au-199, Pb-211, Pb-212, and Bi-213, Co-58, Ga-67, Br-80m, Tc-99m, Rh- 103m, Pt-109, In-ill, Sb-I 19,1-125, Ho-161, Os-189m, Ir-192, Dy-152, At-211, Bi-212, Ra-223, Rn-219, Po-215, Bi-211, Ac-225, Fr-221, At-217, Bi-213, Fm-255 and combinations thereof. Additionally, boron, gadolinium or uranium atoms can be used, wherein the boron atom is preferably B-10, the gadolinium atom is Gd-157 and the uranium atom is U-235.

Preferably, the therapeutic radionuclide conjugate has a radionuclide with an energy between 20 and 10,000 keV. The radionuclide can be an Auger emitter, with an energy of less than 1000 keV, a P emitter with an energy between 20 and 5000 keV, or an alpha or'a' emitter with an energy between 2000 and 10,000 keV.

Diagnostic radioconjugates may contain a radionuclide that is a gamma- beta- or positron-emitting isotope, where the radionuclide has an energy between 20 and 10,000 keV. The radionuclide may be selected from the group of 18F, 51Mn, 52mMn, 52Fe, 55Co, 62Cu, 64Cu, 68Ga, 72As, 75Br, 76Br, 82mRb, 83Sr, 86y, 89Zr, 94mTc, IIn, 120i, 124i, 51Cr, 57Co, 58Co, 59Fe, 67CU, 67Ga, 75Se, 97Ru, 99mTc, IllIn, 114mIn, 123i, 125i, 131i, 169, 197Hg, and 21TI.

Additional types of diagnostic immunoconjugates are contemplated. The antibody or fragments of the invention may be linked to diagnostic agents that are photoactive or contrast agents. Photoactive compounds can comprise compounds such as chromagens or dyes. Contrast agents may be for example a paramagnetic ion, wherein the ion comprises a metal selected from the group of chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) and erbium (III). The contrast agent may also be a radio-opaque compound used in X-ray techniques or computed tomography, such as an iodine, iridium, barium, gallium and thallium compound. Radio-opaque compounds may be selected from the group of barium, diatrizoate, ethiodized oil, gallium citrate, iocarmic acid, iocetamic acid, iodamide, iodipamide, iodoxamic acid, iogulamide, iohexol, iopamidol, iopanoic acid, ioprocemic acid, iosefamic acid, ioseric acid, iosulamide meglumine, iosemetic acid, iotasul, iotetric acid, iothalamic acid, iotroxic acid, ioxaglic acid, ioxotrizoic acid, ipodate, meglumine, metrizamide, metrizoate, propyliodone, and thallous chloride. Alternatively, the diagnostic immunoconjugates may contain ultrasound-enhancing agents such as a gas filled liposome that is conjugated to an antibody of the invention. Diagnostic immunoconjugates may be used for a variety of procedures including, but not limited to, intraoperative, endoscopic or intravascular methods of tumor or cancer diagnosis and detection.

Conjugates of the antibody and cytotoxic agent may be made using a variety of bifunctional protein coupling agents such as N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al. Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, dimethyl linker or disulfide-containing linker (Chari et al. Cancer Research 52: 127-131 (1992)) may be used. Agents may be additionally be linked to the antibodies of the invention through a carbohydrate moiety.

Alternatively, a fusion protein comprising the anti-tumor cell antigen antibody and cytotoxic agent may be made, e.g. by recombinant techniques or peptide synthesis.

In yet another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (e.g. avidin) which is conjugated to a cytotoxic agent (e.g. a radionucleotide).

The anti-tumor cell antigen antibodies may additionally be conjugated to a cytotoxic molecule which is only released inside a target cell lysozome. For example, the drug monomethyl auristatin E (MMAE) can be conjugated via a valine-citrulline linkage which will be cleaved by the proteolytic lysozomal enzyme cathepsin B following internalization of the antibody conjugate (see for example WO03/026577 published April 3, 2003). Alternatively, the MMAE can be attached to the antibody using an acid-labile linker containing a hydrazone functionality as the cleavable moiety (see for example WO02/088172 published Nov. 11, 2002).

### (xi) Antibody Dependent Enzyme Mediated Prodrug Therapy (ADEPT)

The antibodies of the present invention may also be used in ADEPT by conjugating the antibody to a prodrug-activating enzyme which converts a prodrug (e.g. a peptidyl chemotherapeutic agent, see WO81/01145) to an active anti-cancer drug. See, for example, WO 88/07378 and U.S. Pat. No. 4,975,278.

The enzyme component of the immunoconjugate useful for ADEPT includes any enzyme capable of acting on a prodrug in such a way so as to covert it into its more active, cytotoxic form.

Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as .beta.-galactosidase and neuraminidase useful for converting glycosylated prodrugs into free drugs; .beta.-lactamase useful for converting drugs derivatized with .beta.-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, e.g., Massey, Nature 328: 457-458 (1987)). Antibody-abzyme conjugates can be prepared as described herein for delivery of the abryme to a tumor cell population.

The enzymes of this invention can be covalently bound to the anti-tumor cell antigen antibodies by techniques well known in the art such as the use of the heterobifunctional crosslinking reagents discussed above. Alternatively, fusion proteins comprising at least the antigen binding region of an antibody of the invention linked to at least a functionally active portion of an enzyme of the invention can be constructed using recombinant DNA techniques well known in the art (see, e.g., Neuberger et al., Nature, 312: 604-608 (1984).

### (xii) Other Antibody Modifications

Other modifications of the antibody are contemplated herein. For example, the antibody may be linked to one of a variety of nonproteinaceous polymers, e.g., polyethylene glycol, polypropylene glycol, polyoxyalkylenes, or copolymers of polyethylene glycol and polypropylene glycol. The antibody also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Oslo, A., Ed., (1980).

The anti-tumor cell antigen antibodies disclosed herein may also be formulated as immunoliposomes. Liposomes containing the antibody are prepared by methods known in the art, such as described in Epstein et al., Proc. Natl. Acad. Sci. USA, 82:3688 (1985); Hwang et al., Proc. Natl Acad. Sci. USA, 77:4030 (1980); U.S. Pat. Nos. 4,485,045 and 4,544,545; and WO97/38731 published Oct. 23, 1997. Liposomes with enhanced circulation time are disclosed in U.S. Pat. No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al. J. Biol. Chem. 257: 286-288 (1982) via a disulfide interchange reaction. A chemotherapeutic agent is optionally contained within the liposome. See Gabizon et al. J. National Cancer Inst. 81(19)1484 (1989).

Diagnostic or therapeutic fusion proteins may be constructed comprising the antibodies of the invention. These fusion proteins may comprise at least two anti-tumor cell antigen binding domains derived from the antibodies of the invention, or may contain one anti-tumor cell antigen binding domain fused to a binding domain which binds to an alternate epitope of interest. This alternate epitope may be a carcinoma-associated epitope. These fusions may also be immunoconjugates, and may be comprised of full-length antibodies or fragments thereof.

### (IV) Screening for Antibodies with the Desired Properties

Techniques for generating antibodies have been described above. One may further select antibodies with certain biological characteristics, as desired.

To identify an antibody that promotes cell death, in vitro assays can be performed using cancer cells that express the tumor cell antigen and the antibodies of the invention. One assay that is known in the art is the CellTiter 96 Aqueous Non-radioactive Cell proliferation assay (Promega cat# G5421). In this assay, solutions of a novel tetrazolium compound [3-(4,5-dimethylthiazol-2-yl)-5-(3-carboxymethoxyphenyl)-2-(4-sulfophenyl)-2H- tetrazolium, inner salt; MTS(a)] and an electron coupling reagent (phenazine methosulfate) PMS are utilyzed. MTS is bioreduced by cells into a formazan product that is soluble in tissue culture medium. The absorbance of the formazan product at 490nm can be measured directly from 96-well assay plates without additional processing. The conversion of MTS into the aqueous soluble formazan product is accomplished by dehydrogenase enzymes found in metabolically active cells. The quantity of formazan product as measured by the amount of 490nm absorbance is directly proportional to the number of living cells in culture. Another preferred assay is the CytoTox 96 assay (Promega cat# G1780) that quantitatively measures released lactate dehydrogenase (LDH) in culture supernatants with a coupled enzymatic assay that results in the conversion of a tetrazolium salt (INT) into a red formazan product. The amount of color formed is proportional to the number of lysed cells. Visible wavelength absorbance data are collected using a standard 96-well plate reader.

Identification of an antibody that acts in a cytostatic manner rather than an cytotoxic manner can be accomplished by measuring viability of a treated target cell culture in comparison with a non-treated control culture. Viability can be detected using methods known in the art such as the CellTiter-Blue® Cell Viability Assay or the CellTiter-Glo® Luminescent Cell Viability Assay (Promega, catalog numbers G8080 and G5750 respectively). An antibody will be considered as potentially cytostatic if treatment causes a decrease in cell number in comparison to the control culture without any evidence of cell death as measured by the means described above.

To identify an antibody that promotes ADCC, an in vitro screening assay can be performed following one of the assays known in the art. One preferred assay is the In Vitro ADCC Assay: To prepare chromium 51 -labeled target cells, tumor cell lines are grown in tissue culture plates and harvested using sterile 10 mM EDTA in PBS. The detached cells are washed twice with cell culture medium. Cells (5×10⁶) are labeled with 200 µCi of chromium 51 (New England Nuctear/DuPont) at 37° C. for one hour with occasional mixing. Labeled cells were washed three times with cell culture medium, then are resuspended to a concentration of 1×10⁵ cells/mL. Cells are used either without opsonization, or are opsonized prior to the assay by incubation with test antibody at 100 ng/mL and 1.25 ng/mL in PBMC assay or 20 ng/mL and 1 ng/mL in NK assay. Peripheral blood mononuclear cells are prepared by collecting blood on heparin from normal healthy donors and diluted with an equal volume of phosphate buffered saline (PBS). The blood is then layered over LYMPHOCYTE SEPARATION MEDIUM® (LSM: Organon Teknika) and centrifuged according to the manufacturer's instructions. Mononuclear cells are collected from the LSM-plasma interface and are washed three times with PBS. Effector cells are suspended in cell culture medium to a final concentration of 1×10⁷ cells/mL. After purification through LSM, natural killer (NK) cells are isolated from PBMCs by negative selection using an NK cell isolation kit and a magnetic column (Miltenyi Biotech) according to the manufacturer's instructions. Isolated NK cells are collected, washed and resuspended in cell culture medium to a concentration of 2×10⁶ cells/mL. The identity of the NK cells is confirmed by flow cytometric analysis. Varying effector:target ratios are prepared by serially diluting the effector (either PBMC or NK) cells two-fold along the rows of a microtiter plate (100 µL final volume) in cell culture medium. The concentration of effector cells ranges from 1.0×10⁷/mL to 2.0×10⁴ /mL for PBMC and from 2.0×10⁶/mL to 3.9×10³/mL for NK. After titration of effector cells, 100 µL of chromium 51 -labeled target cells (opsonized or nonoponsonized) at 1×10⁵ cells/mL are added to each well of the plate. This results in an initial effector:target ratio of 100:1 for PBMC and 20:1 for NK cells. All assays are run in duplicate, and each plate contains controls for both spontaneous lysis (no effector cells) and total lysis (target cells plus 100 µL 1% sodium dodecyl sulfate, 1 N sodium hydroxide). The plates are incubated at 37° C. for 18 hours, after which the cell culture supernatants are harvested using a supernatant collection system (Skatron Instrument, Inc.) and counted in a Minaxi auto-gamma 5000 series gamma counter (Packard) for one minute. Results are then expressed as percent cytotoxicity using the formula: % Cytotoxicity=(sample cpm-spontaneous lysis)/(total lysis-spontaneous lysis)×100.

To identify antibodies capable of promoting ADCC in a high throughput format, (pending application 60/756,301, incorporated herein by reference) one can perform the real time ADCC assay using the ACEA Biosciences RT-CES® (Real Time Cell Electronic Sensor) system as follows: Cells are seeded in E-Plate micro-titer plates (ACEA Biosciences) wherein the interaction of cells with the microelectrode surface leads to the generation of a cell-electrode impedance response, which indicates the status of the cells in terms of morphology, quality of adhesion and number. Human PBMC are prepared fresh from human blood (from healthy volunteer donors) and then added to the target cells at a optimal ratio determined experimentally, for example 25:1 effector: target. Test antibody is then added and the cell plate is put back into the ACEA System for continuous monitoring of Cell Index for the next 48 hours. A drop in the recorded cell index after additional of PBMC and test antibody is reflective of killing of the target cells by ADCC mediated by the test antibody. Controls to be performed can include running the assay in the absence of effector PBMC cells or use of an irrelevant isotype control antibody.

To identify an antibody that promotes CDC, the skilled artisan may perform one of the assays known in the art. One preferred assay is the In Vitro CDC assay: In vitro, the CDC activity can be measured by incubating tumor cell antigen expressing cells with human (or alternate source) complement-containing serum in the absence or presence of different concentrations of test antibody. Cytotoxicity is then measured by quantifying live cells using ALAMAR BLUE® (Gazzano-Santoro et al., J. Immunol. Methods 202 163-171 (1997)). Control assays are performed without antibody, and with antibody, but using heat inactivated serum and/or using cells which do not express the tumor cell antigen in question. Alternatively, red blood cells can be coated with tumor antigen or peptides derived from tumor antigen, and then CDC may be assayed by observing red cell lysis (see for example Karjalainen and Mantyjarvi, Acta Pathol Microbiol Scand [C]. 1981 Oct;89(5):315-9).

To identify growth inhibitory anti-tumor cell antigen antibodies, one may screen for antibodies that inhibit the growth of cancer cells that overexpress the tumor cell antigen. In one embodiment, the growth inhibitory antibody of choice is able to inhibit growth of tumor cell antigen expressing cells in cell culture by about 20-100% and preferably by about 50-100% at an antibody concentration of about 0.5 to 30 µg/mL.

To select for antibodies that induce cell death, loss of membrane integrity as indicated by, e.g., PI, trypan blue or 7AAD uptake may be assessed relative to control. The preferred assay is the PI uptake assay using tumor antigen expressing cells. According to this assay, tumor cell antigen expressing cells are cultured in Dulbecco's Modified Eagle Medium (D-MEM):Ham's F-12 (50:50) supplemented with 10% heat-inactivated FBS (Hyclone) and 2 mM L-glutarnine. (Thus, the assay is performed in the absence of complement and immune effector cells). The tumor cells are seeded at a density of 3 × 10⁶ per dish in 100 × 20 mm dishes and allowed to attach overnight. The medium is then removed and replaced with fresh medium alone or medium containing 10 µg/mL of the appropriate monoclonal antibody. The cells are incubated for a 3 day time period. Following each treatment, monolayers are washed with PBS and detached by trypsinization. Cells are then centrifuged at 1200 rpm for 5 minutes at 4.degree. C., the pellet resuspended in 3 mL ice cold Ca²⁺ binding buffer (10 mM Hepes, pH 7.4,140 mM NaCl, 2.5 mM CaCl₂) and aliquoted into 35 mm strainer-capped 12 × 75 tubes (1 mL per tube, 3 tubes per treatment group) for removal of cell clumps. Tubes then receive PI (10 µg/mL). Samples may be analyzed using a FACSCAN™ flow cytometer and FACSCONVERT™. CellQuest software (Becton Dickinson). Those antibodies that induce statistically significant levels of cell death as determined by PI uptake may be selected as cell death-inducing antibodies.

In order to select for antibodies that induce apoptosis, an annexin binding assay using BT474 cells is available. Cell surface phosphatidylserine (PS) can be detected using any of the commercially available Annexin V staining reagents, which are based on the high affinity of annexin V for PS. The BT474 cells are cultured and seeded in dishes as discussed in the preceding paragraph. The medium is then removed and replaced with fresh medium alone or medium containing 10 µg/mL of the monoclonal antibody. Following a three day incubation period, monolayers are washed with PBS and detached by trypsinization. Cells are then centrifuged, resuspended in Ca²⁺ binding buffer and aliquoted into tubes as discussed above for the cell death assay. Tubes then receive labeled annexin (e.g. annexin V-FTIC) (1 µg/mL). Samples may be analyzed using a FACSCAN™. flow cytometer and FACSCONVERT™. CellQuest software (Becton Dickinson). Those antibodies that induce statistically significant levels of annexin binding relative to control are selected as apoptosis-inducing antibodies. Alternatively, see for example, the Annexin V staining reagents commercially available from Roche Applied Science. By conjugating FITC to Annexin V it is possible to identify and quantitate apoptotic cells on a single-cell basis by flow cytometry. Staining cells simultaneously with FITC-Annexin V (green fluorescence) and the non-vital dye propidium iodide (red fluorescence) can provide for the discrimination of intact cells (FITC-PI-), early apoptotic (FITC+PI-), and late apoptotic or necrotic cells (FITC+PI+).

In addition to the annexin binding assay, a DNA staining assay using BT474 cells is available. In order to perform this assay, BT474 cells that have been treated with the antibody of interest as described in the preceding two paragraphs are incubated with 9 µg/ml HOECHST 33342™ for 2 hr at 37° C., then analyzed on an EPICS ELITE.TM. flow cytometer (Coulter Corporation) using MODFIT™. software (Verity Software House). Antibodies that induce a change in the percentage of apoptotic cells which is 2 fold or greater (and preferably 3 fold or greater) than untreated cells (up to 100% apoptotic cells) may be selected as pro-apoptotic antibodies using this assay.

Further, elevated apoptosis within a biological sample can be confirmed with nucleic acid-based methods that detect the DNA fragmentation that is characteristic of apoptosis. When resolved using electrophoresis on agarose gels, apoptotic DNA initially has a characteristic "ladder" pattern, as opposed to a smear of nucleic acids that is observed, for example, in necrosis or other non-specific DNA degradation. A common histochemical technique to detect DNA fragmentation uses end-labeled DNA. Kits for such are commercially available, such as the APOLERT DNA fragmentation kit (Clontech Laboratories, Inc., Palo Alto, California). This assay is based on terminal deoxynucleotidyltransferase (Tdt)-mediated dUTP nick-end labeling (TUNEL), where Tdt catalyzes the incorporation of fluorescein-dUTP at the free 3'-hydroxyl ends of fragmented DNA in cells undergoing apoptosis.

A number of assay kits for biomarkers of caspases are commercially available. For example, the Homogeneous Caspases Assay (Roche Applied Sciences, Indianapolis, Indiana), is a fluorimetric assay for the quantitative in vitro determination of caspase activity in microplates. The assay is particularly useful for high-throughput screening, allowing, for example, for 100 tests on 96-well plates, and 400 tests on 384-well plates (Cat. No. 3 005 372). This assay allows for detection of several caspases, including Caspase-2, Caspase-3, Caspase-7, and to a lesser extent, Caspase-6, Caspase-8, Caspase-9, and Caspase-10, in biological samples, including, for example, serum or plasma. The Cell Death Detection ELISAPLUS assay (Cat. No. 1 774 425; Roche Applied Sciences, Indianapolis, Indiana) is based on a quantitative sandwich-enzyme-immunoassay principle, using mouse monoclonal antibodies directed against DNA and histones, respectively. This assay allows the specific detection and quantitation of mono- and oligonucleosomes that are released into the cytoplasm of cells that die from apoptosis. It can be used for a variety of samples, including cell lysates, serum, culture supernatant, and the like.

The antibodies of the invention can also be screened in vivo for apoptotic activity using 18F-annexin as a PET imaging agent. In this procedure, Annexin V is radiolabeled with 18F and given to the test animal following dosage with the antibody under investigation. One of the earliest events to occur in the apoptotic process in the eversion of phosphatidylserine from the inner side of the cell membrane to the outer cell surface, where it is accessible to annexin. The animals are then subjected to PET imaging (see Yagle et al, J Nucl Med. 2005 Apr;46(4):658-66). Animals can also be sacrificed and individual organs or tumors removed and analyzed for apoptotic markers following standard protocols.

To screen for antibodies that bind to an epitope on the tumor cell antigen bound by an antibody of interest, a routine cross-blocking assay such as that described in Antibodies, A Laboratory Manual, Cold Spring Harbor Laboratory, Ed Harlow and David Lane (1988), can be performed. Alternatively, or additionally, epitope mapping can be performed by methods known in the art.

The antibodies of the invention can be screened for the ability to either rapidly internalized upon binding to the tumor-cell antigen in question, or for the ability to remain on the cell surface following binding. In some embodiments, for example in the construction of some types of immunoconjugates, the ability of an antibody to be internalized may be desired if internalization is required to release the toxin moiety. Alternatively, if the antibody is being used to promote ADCC or CDC, it may be more desirable for the antibody to remain on the cell surface. A screening method can be used to differentiate these type behaviors. For example, a tumor cell antigen bearing cell may be used where the cells are incubated with human IgGi (control antibody) or one of the antibodies of the invention at a concentration of approximately 1 µg/mL on ice (with 0.1% sodium azide to block internalization) or 37°C (without sodium azide) for 3 hours. The cells are then washed with cold staining buffer (PBS+1%BSA+0.1% sodium azide), and are stained with goat anti-human IgG-FITC for 30 minutes on ice. Geometric mean fluorescent intensity (MFI) is recorded by FACS Calibur. If no difference in MFI is observed between cells incubated with the antibody of the invention on ice in the presence of sodium azide and cells observed at 37°C in the absence of sodium azide, the antibody will be suspected to be one that remains bound to the cell surface, rather than being internalized. If however, a decrease in surface stainable antibody is found when the cells are incubated at 37°C in the absence of sodium azide, the antibody will be suspected to be one which is capable of internalization.

### V. Vectors, Host Cells and Recombinant Methods

The invention also provides isolated nucleic acid encoding the humanized anti-tumor cell antigen antibody, vectors and host cells comprising the nucleic acid, and recombinant techniques for the production of the antibody.

For recombinant production of the antibody, the nucleic acid encoding it is isolated and inserted into a replicable vector for further cloning (amplification of the DNA) or for expression. DNA encoding the monoclonal antibody is readily isolated and sequenced using conventional procedures (e.g., by using oligonucleotide probes that are capable of binding specifically to genes encoding the heavy and light chains of the antibody). Many vectors are available. The vector components generally include, but are not limited to, one or more of the following: a signal sequence, an origin of replication, one or more marker genes, an enhancer element, a promoter, and a transcription termination sequence.

### (i) Signal Sequence Component

The anti-tumor cell antigen antibody of this invention may be produced recombinantly not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed (i.e., cleaved by a signal peptidase) by the host cell. For prokaryotic host cells that do not recognize and process the native anti-tumor cell antigen antibody signal sequence, the signal sequence is substituted by a prokaryotic signal sequence selected, for example, from the group of the alkaline phosphatase, penicillinase, Ipp, or heat-stable enterotoxin II leaders. For yeast secretion the native signal sequence may be substituted by, e.g., the yeast invertase leader, .alpha. factor leader (including Saccharomyces and Kluyveromyces .alpha.-factor leaders), or acid phosphatase leader, the *C albicans* glucoamylase leader, or the signal described in WO 90/13646. In mammalian cell expression, mammalian signal sequences as well as viral secretory leaders, for example, the herpes simplex gD signal, are available.

The DNA for such precursor region is ligated in reading frame to DNA encoding the anti-tumor cell antigen antibody.

### (ii) Origin of Replication Component

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2.mu. plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

### (iii) Selection Gene Component

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for Bacilli.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the anti-tumor cell antigen antibody nucleic acid, such as DHFR, thymidine kinase, metallothionein-I and -II, preferably primate metallothionein genes, adenosine deaminase, ornithine decarboxylase, etc.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity.

Alternatively, host cells (particularly wild-type hosts that contain endogenous DHFR) transformed or co-transformed with DNA sequences encoding anti-tumor cell antigen antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Pat. No. 4,965,199.

A suitable selection gene for use in yeast is the trp1 gene present in the yeast plasmid YRp7 (Stinchcomb et al., Nature, 282:39 (1979)). The trp1 gene provides a selection marker for a mutant strain of yeast lacking the ability to grow in tryptophan, for example, ATCC No. 44076 or PEP4-1. Jones, Genetics, 85:12 (1977). The presence of the trp1 lesion in the yeast host cell genome then provides an effective environment for detecting transformation by growth in the absence of tryptophan. Similarly, Leu2-deficient yeast strains (ATCC 20,622 or 38,626) are complemented by known plasmids bearing the Leu2 gene.

In addition, vectors derived from the 1.6 .mu.m circular plasmid pKD1 can be used for transformation of Kluyveromyces yeasts. Alternatively, an expression system for large-scale production of recombinant calf chymosin was reported for *K. lactis.* Van den Berg, Bio/Technology, 8:135 (1990). Stable multi-copy expression vectors for secretion of mature recombinant human serum albumin by industrial strains of Kluyveromyces have also been disclosed. Fleer et al., Bio/Technology, 9:968-975 (1991).

### (iv) Promoter Component

Expression and cloning vectors usually contain a promoter that is recognized by the host organism and is operably linked to the anti-tumor cell antigen antibody nucleic acid. Promoters suitable for use with prokaryotic hosts include the phoA promoter, .beta.-lactamase and lactose promoter systems, alkaline phosphatase, a tryptophan (trp) promoter system, and hybrid promoters such as the tac promoter. However, other known bacterial promoters are suitable. Promoters for use in bacterial systems also will contain a Shine-Dalgarno (S.D.) sequence operably linked to the DNA encoding the anti-tumor cell antigen antibody.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Anti-tumor cell antigen antibody transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, e.g., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Pat. No. 4,419,446. A modification of this system is described in U.S. Pat. No. 4,601,978. See also Reyes et al., Nature 297:598-601 (1982) on expression of human .beta.-interferon cDNA in mouse cells under the control of a thymidine kinase promoter from herpes simplex virus. Alternatively, the rous sarcoma virus long terminal repeat can be used as the promoter.

### (v) Enhancer Element Component

Transcription of a DNA encoding the anti-tumor cell antigen antibody of this invention by higher eukaryotes is often increased by inserting an enhancer sequence into the vector. Many enhancer sequences are now known from mammalian genes (globin, elastase, albumin, .alpha.-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the anti-tumor cell antigen antibody-encoding sequence, but is preferably located at a site 5' from the promoter.

### (vi) Transcription Termination Component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding anti-tumor cell antigen antibody. One useful transcription termination component is the bovine growth hormone polyadenylation region. See WO94/11026 and the expression vector disclosed therein.

### (vii) Selection and Transformation of Host Cells

Host cells are transfected or transformed with expression or cloning vectors described herein for antibody production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 Jun. 1989. For mammalian cells without such cell walls, the calcium phosphate precipitation method of Graham and van der Eb, Virology, 52:456-457 (1978) can be employed. General aspects of mammalian cell host system transfections have been described in U.S. Pat. No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, e.g., *E. coli,* Enterobacter, Erwinia, Klebsiella, Proteus, Salmonella, e.g., *Salmonella typhimurium,* Serratia, e.g., *Serratia marcescans,* and Shigella, as well as Bacilli such as *B. subtilis and B. licheniformis* (e.g*., B. licheniformis* 41P disclosed in DD 266,710 published 12 Apr. 1989), Pseudomonas such as *P. aeruginosa,* and Streptomyces. One preferred *E. coli* cloning host is E. coli 294 (ATCC 31,446), although other strains such as *E. coli* B*, E. coli*X1776 (ATCC 31,537), and *E. coli* W3110 (ATCC 27,325) are suitable. These examples are illustrative rather than limiting.

In addition to prokaryotes, eukaryotic microbes such as filamentous fungi or yeast are suitable cloning or expression hosts for antibody-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Pat. No. 4,943,529; Fleer et al., Bio/Technology, 9:968-975 (1991)) such as, e.g., *K. lactis* (MW98-8C*,* CBS683, CBS4574; Louvencourt et al., J. Bacteriol., 154(2):737-742 [1983]), *K. fragilis* (ATCC 12,424), K. *bulgaricus*(ATCC 16,045), *K. wickeramii* (ATCC 24,178), *K. waltii* (ATCC 56,500), *K. drosophilarum* (ATCC 36,906; Van den Berg et al., Bio/Technology, 8:135(1990)), *K. thermotolerans,* and *K. marxianus; yarrowia* (EP 402,226); *Pichiapastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces such* as *Schwanniomyces occidentalis* (P 394,538 published 31 Oct. 1990); and filamentous fungi such as, e.g., *Neurospora, Penicillium, Tolypocladium* (WO 91/00357 published 10 Jan. 1991), and *Aspergillus* hosts such as A. *nidulans* (Ballance et al., Biochem. Biophys Res. Commun., 112:284-289 [1983]; Tilburn et al., Gene, 26:205-221 [1983]; Yelton et al., Proc. Natl. Acad. Sci. USA, 81: 1470-1474 [1984]) and A. niger (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated antibodies are derived from multicellular organisms. Examples of invertebrate cells include insect cells such as *Drosophila* S2 and *Spodoptera* Sf9, as well as plant cells. Examples of useful mammalian host cell lines include Chinese hamster ovary (CHO) and COS cells. More specific examples include monkey kidney CVI line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR(CHO, Urlaub and Chasin, Proc. Natl. Acad. Sci. USA, 77:4216(1980)); mouse sertoli cells (IM4, Mather, Biol. Reprod., 23:243-251(1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for anti-tumor cell antigen antibody production and cultured in conventional nutrient media modified as appropriate for inducing promoters, selecting transformants, or amplifying the genes encoding the desired sequences.

### (viii) Culturing the Host Cells

The host cells used to produce the anti-tumor cell antigen antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Pat. No. Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN.TM. drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (ix) Detecting Gene Amplification/Expression

Gene amplification and/or expression may be measured in a sample directly, for example, by conventional Southern blotting, Northern blotting to quantitate the transcription of mRNA [Thomas, Proc. Nad. Acad. Sci. USA. 77:5201-5205 (1980)], dot blotting (DNA analysis), or in situ hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product Antibodies useful for immunohistochemical staining and/or assay of sample fluids may be either monoclonal or polyclonal, and may be prepared in any mammal. Conveniently, the antibodies may be prepared against a native sequence tumor cell antigen polypeptide or against a synthetic peptide based on the DNA sequences provided herein or against exogenous sequence fused to tumor cell antigen DNA and encoding a specific antibody epitope.

### (x) Purification of Anti-tumor Cell Antigen Antibody

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology 10:163-167 (1992) describe a procedure for isolating antibodies which are secreted to the periplasmic space of *E*. *coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human .gamma.1, .gamma.2, or .gamma.4 heavy chains (Lindmark et al., J. Immunol. Meth. 62:1-13 (1983)). Protein G is recommended for all mouse isotypes and for human .gamma.3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C_{H3} domain, the Bakerbond ABX™ resin (J. T. Baker, Phillipsburg, N.J.) is useful for purification. Other techniques for protein purification such as fractionation on an ionexchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (e.g.,from about 0-0.25M salt).

### VI. Pharmaceutical Formulations

Therapeutic formulations of the agonist/antagonist or antibody molecules used in accordance with the present invention are prepared for storage by mixing the molecule having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ioris such as sodium; metal complexes (e.g. Zn-protein complexes); and/or non-ionic surfactants such as TWEEN™, PLURONICS™ or polyethylene glycol (PEG). Preferred lyophilized anti-tumor cell antigen antibody formulations are described in WO 97/04801, expressly incorporated herein by reference.

The formulation herein may also contain more than one active compound as necessary for the particular indication being treated, preferably those with complementary activities that do not adversely affect each other. For example, it may be desirable to further provide antibodies which bind to alternate family member proteins or to different epitopes on the selected tumor cell antigen in the one formulation. Suitable second antibodies can include antibodies against CEA, EGP-1, EGP-2 (e.g., 17-IA), MUC-1, MUC-2, MUC-3, MUC-4, PAM-4, KC4, TAG-72, EGFR, HER2/neu, BrE3, Le-Y, A3, Ep-CAM, Tn, and Thomson-Friedenreich antigens, tumor necrosis antigens, ferritin, acidic isoferritin, tenascin, an oncogene, an oncogene product, IL-6, IGF-1, IGFR-1, tumor angiogenesis antigens, such as vascular endothelium growth factor (VEGF), placental growth factor (PIGF), ED-B fibronectin, and other vascular growth factors, Ga 733, or a combination thereof. Alternately, the second antibody may be one that binds to the same tumor cell antigen, but uses an alternate epitope. In a further embodiment, the second antibody may bind to an alternate tumor cell antigen, where the tumor cell antigen is selected from the group of KIAA1815, LOC157378, FLJ20421, DSCD75, GPR160, GPCR41, and SLC1A5. It is contemplated that all the second antibodies may be immunoconjugates or naked antibodies, and may be multivalent antibodies. These antibody combinations may be administered before, concurrently or after dosage with the antibodies of the invention.

Alternatively, or additionally, the composition may further comprise a chemotherapeutic agent, cytotoxic agent, cytokine, growth inhibitory agent, anti-hormonal agent, anti-angiogenic agent, and/or cardioprotectant. Such molecules are suitably present in combination in amounts that are effective for the purpose intended. These agents may be administered before, concurrently or after dosage with the antibodies of the invention.

The formulations to be used for in vivo administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

The route of administration is in accord with known methods, e.g. injection or infusion by intravenous, intraperitoneal, intracerebral, intramuscular, intraocular, intraarterial or intralesional routes, topical administration, or by sustained release systems.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Dosages and desired drug concentrations of pharmaceutical compositions of the present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" In Toxicokinetics and New Drug Development, Yacobi et al., Eds., Pergamon Press, New York 1989, pp. 42-96.

When in vivo administration of an anti-tumor cell antigen antibody is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. No. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

Where sustained-release administration of an anti-tumor cell antigen antibody is desired in a formulation with release characteristics suitable for the treatment of any disease or disorder requiring administration of the antibody, microencapsulation of the antibody is contemplated. Microencapsulation of recombinant proteins for sustained release has been successfully performed with human growth hormone (rhGH), interferon-(rhIFN-), interleukin-2, and MN rgp120. Johnson et al., Nat. Med., 2:795-799 (1996); Yasuda, Biomed. Ther., 27:1221-1223 (1993); Horaet al., Bio/Technology. 8:755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439462; WO 97/03692, WO 96/40072, WO 96/07399; and U.S. Pat. No. 5,654,010.

The sustained-release formulations of these proteins were developed using poly-lactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. Lewis, "Controlled release of bioactive agents from lactide/glycolide polymer," in: M. Chasin and R. Langer (Eds.), Biodegradable Polymers as Drug Delivery Systems (Marcel Dekker: New York, 1990), pp. 1-41.

### VII. Treatment with the Anti-tumor Cell Antigen Antibodies

It is contemplated that, according to the present invention, the anti-tumor cell antigen agonist/antagonist or antibody molecules may be used to treat various diseases or disorders. Exemplary conditions or disorders include benign or malignant tumors; leukemias and lymphoid malignancies; other disorders such as neuronal, glial, astrocytal, hypothalamic, glandular, macrophagal, epithelial, stromal, blastocoelic, inflammatory, angiogenic and immunologic disorders.

Generally, the disease or disorder to be treated is cancer. Examples of cancer to be treated herein include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

The cancer will generally comprise tumor antigen-expressing cells, such that the anti-tumor antigen antibody herein is able to bind to the cancer. Methods for detecting expression of the biomarkers of the invention, and optionally cytokine markers, within the test and control biological samples comprise any methods that determine the quantity or the presence of these markers either at the nucleic acid or protein level. Such methods are well known in the art and include but are not limited to western blots, northern blots, ELISA, immunoprecipitation, immunofluorescence, flow cytometry, immunohistochemistry, nucleic acid hybridization techniques, nucleic acid reverse transcription methods, and nucleic acid amplification methods. In particular embodiments, expression of a biomarker is detected on a protein level using, for example, antibodies that are directed against specific biomarker proteins. These antibodies can be used in various methods such as Western blot, ELISA, multiplexing technologies, immunoprecipitation, or immunohistochemistry techniques. In some embodiments, detection of cytokine markers is accomplished by electrochemiluminescence (ECL). Any of these detection methods for biomarkers and optionally cytokine markers can be combined with assessment of clinical information, conventional prognostic methods, expression of other tumor cell antigen-related factors, particularly expression of cell-surface tumor cell antigen and circulating levels of potential soluble tumor cell antigen, and expression of, or presence of, clinically useful and characteristic prognostic markers known in the art, including, but not limited to, those noted herein for cancer patients. In this manner, the disclosed methods may permit the more accurate determination of candidate subjects whose cancer or pre-malignant condition would benefit from therapeutic intervention with an anti-tumor cell antigen therapeutic agent described herein.

Thus, in some embodiments, a candidate subject having a hyperproliferative, cancerous or pre-malignant disorder or condition that is associated with tumor cell antigen-expressing neoplastic cells is tested for responsiveness to an anti-tumor cell antigen therapeutic agent of interest using the ex vivo prognostic assays described herein, wherein effects of the therapeutic agent on one or more tumor cell antigen-mediated activities is assessed. Where further refinement of the ex vivo prognostic assay is desirable, the candidate subject can be examined for the level of expression of, or absence of expression of, one or more tumor cell antigen-related factors identified herein above, one or more clinically useful prognostic markers. In this manner, a biological sample comprising tumor cell antigen-expressing neoplastic cells can be collected from a candidate subject and assessed for the level of expression of, or absence of expression of, the tumor cell antigen-related factor(s) and/or clinically useful prognostic marker(s) of interest. Any biological sample comprising tumor cell antigen-expressing neoplastic cells as noted herein above can be collected for these prognostic assays. Further, any detection method known to those of skill in the art can be used to detect the level of expression, or absence of expression, of the tumor cell antigen-related factor(s) and/or clinically useful prognostic marker(s) of interest, as noted elsewhere herein.

Where the expression level of one or more tumor cell antigen-related factors is to be assessed in order to identify a subject having a cancer or pre-malignant condition that will be responsive to treatment with an anti-tumor cell antigen therapeutic agent, a biological sample is collected from the subject, and the level of expression in that sample is compared to the level of expression of that factor (or factors) in a control or reference standard. For expression level of cell-surface tumor cell antigen, any biological sample comprising tumor cell antigen-expressing neoplastic cells can be used as noted herein above. For circulating levels of soluble tumor cell antigen, a blood sample or sample comprising a blood component such as plasma or serum can be obtained from the candidate subject. By "control" or "reference standard" is intended a standard that is of the same biological source (i.e., tissue or bodily fluid) and which distinguishes subjects having the cancer or pre-malignant condition from healthy subjects that are not afflicted with the disease. A skilled artisan can provide a reference standard by taking a measurement of expression levels of these tumor cell antigen-related factors (i.e., cell-surface tumor cell antigen or soluble tumor cell antigen) in healthy subjects that do not have the disease and subjects that do have the disease, controlling for age, sex, race, and the like, and comparing the expression levels to determine the standard level of expression to be expected in a healthy subject. In some embodiments, the expression level in the candidate subject having the cancer or pre-malignant condition is at least 20%, 30%, 40%, 50%, 60%, 70%, 80%, 100%, 150%, 200%, 250%, 300% greater than the expression level in the reference standard. It is recognized that the applicability of treatment with an anti-tumor cell antigen therapeutic agent can be assessed by detecting the level of expression of one or more of these tumor cell antigen-related factors, wherein an increased level of expression in a biological sample relative to the reference standard is sufficient to establish that the subject has a cancer or pre-malignant condition that will be responsive to treatment with the anti-tumor cell antigen therapeutic agent of interest without having to do additional screening for ex vivo effects of the anti-tumor cell antigen therapeutic agent on tumor cell antigen-mediated activities such as cell survival and proliferation, and/or ADCC activity.

The presence of the tumor cell antigens of interest described herein within a biological sample obtained from a candidate subject may also be determined at the nucleic acid level. Nucleic acid-based techniques for assessing expression are well known in the art and include, for example, determining the level of biomarker mRNA in a biological sample. Many expression detection methods use isolated RNA. Any RNA isolation technique that does not select against the isolation of mRNA can be utilized for the purification of RNA (see, e.g., Ausubel et al., ed. (1987-1999) Current Protocols in Molecular Biology (John Wiley & Sons, New York). Additionally, large numbers of tissue samples can readily be processed using techniques well known to those of skill in the art, such as, for example, the single-step RNA isolation process disclosed in U.S. Patent No. 4,843,155.

Thus, in some embodiments, the detection of a biomarker or other protein of interest is assayed at the nucleic acid level using nucleic acid probes. The term "nucleic acid probe" refers to any molecule that is capable of selectively binding to a specifically intended target nucleic acid molecule, for example, a nucleotide transcript. Probes can be synthesized by one of skill in the art, or derived from appropriate biological preparations. Probes may be specifically designed to be labeled, for example, with a radioactive label, a fluorescent label, an enzyme, a chemiluminescent tag, a colorimetric tag, or other labels or tags that are discussed above or that are known in the art. Examples of molecules that can be utilized as probes include, but are not limited to, RNA and DNA.

For example, isolated mRNA can be used in hybridization or amplification assays that include, but are not limited to, Southern or Northern analyses, polymerase chain reaction analyses and probe arrays. One method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7,15, 30, 50, 100, 250 or 500 nucleotides in length and sufficient to specifically hybridize under stringent conditions to an mRNA or genomic DNA encoding a biomarker, CD40-related factor, or clinically useful prognostic marker described herein above. Hybridization of an mRNA with the probe indicates that the biomarker or other target protein of interest is being expressed.

In one embodiment, the mRNA is immobilized on a solid surface and contacted with a probe, for example by running the isolated mRNA on an agarose gel and transferring the mRNA from the gel to a membrane, such as nitrocellulose. In an alternative embodiment, the probe(s) are immobilized on a solid surface and the mRNA is contacted with the probe(s), for example, in a gene chip array. A skilled artisan can readily adapt known mRNA detection methods for use in detecting the level of mRNA encoding the biomarkers or other proteins of interest. An alternative method for determining the level of a mRNA of interest in a sample involves the process of nucleic acid amplification, e.g., by RT-PCR (see, for example, U.S. Patent No. 4,683,202), ligase chain reaction (Barany (1991) Proc. Natl. Acad. Sci. USA 88:189-193), self-sustained sequence replication (Guatelli et al. (1990) Proc. Natl. Acad. Sci. USA 87:1874-1878), transcriptional amplification system (Kwoh et al. (1989) Proc. Natl. Acad. Sci. USA 86:1173-1177), Q-Beta Replicase (Lizardi et al. (1988) Bio/Technology 6:1197), rolling circle replication (U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. In particular aspects of the invention, biomarker expression, or expression of a tumor cell antigen-related factor or other clinically useful prognostic marker, is assessed by quantitative fluorogenic RT-PCR (i.e., the TaqMan® System).

Expression levels of an RNA of interest may be monitored using a membrane blot (such as used in hybridization analysis such as Northern, dot, and the like), or microwells, sample tubes, gels, beads or fibers (or any solid support comprising bound nucleic acids). See U.S. Patent Nos. 5,770,722, 5,874,219, 5,744,305, 5,677,195 and 5,445,934, which are incorporated herein by reference. The detection of expression may also comprise using nucleic acid probes in solution. In one embodiment of the invention, microarrays are used to detect expression of one or more biomarkers, tumor cell antigen-related factors, and/or clinically useful prognostic markers. Microarrays are particularly well suited for this purpose because of the reproducibility between different experiments. DNA microarrays provide one method for the simultaneous measurement of the expression levels of large numbers of genes. Each array consists of a reproducible pattern of capture probes attached to a solid support. Labeled RNA or DNA is hybridized to complementary probes on the array and then detected by laser scanning. Hybridization intensities for each probe on the array are determined and converted to a quantitative value representing relative gene expression levels. See, U.S. Patent Nos. 6,040,138, 5,800,992 and 6,020,135, 6,033,860, and 6,344,316, which are incorporated herein by reference. High-density oligonucleotide arrays are particularly useful for determining the gene expression profile for a large number of RNA's in a sample.

Techniques for the synthesis of these arrays using mechanical synthesis methods are described in, e.g., U.S. Patent No. 5,384,261, incorporated herein by reference in its entirety. Although a planar array surface is preferred, the array may be fabricated on a surface of virtually any shape or even a multiplicity of surfaces. Arrays may be peptides or nucleic acids on beads, gels, polymeric surfaces, fibers such as fiber optics, glass or any other appropriate substrate, see U.S. Patent Nos. 5,770,358, 5,789,162, 5,708,153, 6,040,193 and 5,800,992, each of which is hereby incorporated in its entirety for all purposes. Arrays may be packaged in such a manner as to allow for diagnostics or other manipulation of an all-inclusive device. See, for example, U.S. Patent Nos. 5,856,174 and 5,922,591, herein incorporated by reference.

In one approach, total mRNA isolated from the sample is converted to labeled cRNA and then hybridized to an oligonucleotide array. Each sample is hybridized to a separate array. Relative transcript levels may be calculated by reference to appropriate controls present on the array and in the sample.

While the cancer may be characterized by overexpression of the tumor antigen, the present application further provides a method for treating cancer which is not considered to be a tumor antigen-overexpressing cancer. To determine tumor antigen expression in the cancer, various diagnostic/prognostic assays are available. In one embodiment, tumor antigen overexpression may be analyzed by IHC. Paraffin embedded tissue sections from a tumor biopsy may be subjected to the IHC assay and accorded a tumor antigen protein staining intensity criteria as follows:
Score 0
   no staining is observed or membrane staining is observed in less than 10% of tumor cells.
Score 1+
   a faint/barely perceptible membrane staining is detected in more than 10% of the tumor cells. The cells are only stained in part of their membrane.
Score 2+
   a weak to moderate complete membrane staining is observed in more than 10% of the tumor cells.
Score 3+
   a moderate to strong complete membrane staining is observed in more than 10% of the tumor cells.

Those tumors with 0 or 1+ scores for tumor antigen overexpression assessment may be characterized as not overexpressing the tumor antigen, whereas those tumors with 2+ or 3+ scores may be characterized as overexpressing the tumor antigen.

Alternatively, or additionally, FISH assays such as the INFORM™ (sold by Ventana, Ariz.) or PATHVISION™ (Vysis, Ill.) may be carried out on formalin-fixed, paraffin-embedded tumor tissue to determine the extent (if any) of tumor antigen overexpression in the tumor.

Moreover, tumor antigen overexpression or amplification may be evaluated using an in vivo diagnostic assay, e.g. by administering a molecule (such as an antibody) which binds the molecule to be detected and is tagged with a detectable label (e.g. a radioactive isotope) and externally scanning the patient for localization of the label.

In a preferred embodiment, tumor antigens are selected for targeting by comparison of the expression level of the antigen in comparison with neighboring healthy tissue or with pooled normal tissue. Preferred candidate tumor antigens include those with at least a 3 fold (300%) increased expression relative to surrounding normal tissue, and wherein this 3 fold increase is seen in comparison with a majority of pooled, commercially available normal tissue samples. Screening for preferred tumor antigens can be carried out using laser capture microscopy to dissect cancerous tissues from normal adjacent ones, followed by expressional microarray analysis utilizing standard commercially available chips such as the standard Affimetrix chip U133 (cat# 900370) (see for example, Yang et al, (2005) Oncogene, 10-31). Alternatively, custom chips containing nucleic acid samples derived from pools of patient tissue samples grouped by cancer type can be made and probed to analyze expression profiles (see for example Makino et al, Dis Esophagus. 2005;18(1):37-40.):

Where the cancer to be treated is hormone independent cancer, expression of the hormone (e.g. androgen) and/or its cognate receptor in the tumor may be assessed using any of the various assays available, e.g. as described above. Alternatively, or additionally, the patient may be diagnosed as having hormone independent cancer in that they no longer respond to anti-androgen therapy.

In certain embodiments, an immunoconjugate comprising the anti-tumor antigen antibody conjugated with a cytotoxic agent is administered to the patient. Preferably, the immunoconjugate and/or tumor cell antigen protein to which it is bound is/are internalized by the cell, resulting in increased therapeutic efficacy of the immunoconjugate in killing the cancer cell to which it binds. In a preferred embodiment, the cytotoxic agent targets or interferes with nucleic acid in the cancer cell. Examples of such cytotoxic agents include maytansinoids, calicheamicins, ribonucleases and DNA endonucleases.

The anti-tumor cell antigen antibodies or immunoconjugates are administered to a human patient in accord with known methods, such as intravenous administration, e.g., as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody is preferred.

Other therapeutic regimens may be combined with the administration of the anti-tumor cell antigen antibody. The combined administration includes coadministration, using separate formulations or a single pharmaceutical formulation, and consecutive administration in either order, wherein preferably there is a time period while both (or all) active agents simultaneously exert their biological activities (review see Lin et al (2005) Clinical Cancer Research 11:129-138).

In one embodiment, the treatment of the present invention involves the combined administration of an anti-tumor cell antigen antibody (or antibodies) and one or more chemotherapeutic agents or growth inhibitory agents, including coadministration of cocktails of different chemotherapeutic agents. Preferred chemotherapeutic agents include taxanes (such as paclitaxel and docetaxel) and/or anthracycline antibiotics. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M. C. Perry, Williams & Wilkins, Baltimore, Md. (1992).

The antibody may be combined with an anti-hormonal compound; e.g., an anti-estrogen compound such as tamoxifen; an anti-progesterone such as onapristone (see, EP 616 812); or an anti-androgen such as flutamide, in dosages known for such molecules. Where the cancer to be treated is hormone independent cancer, the patient may previously have been subjected to anti-hormonal therapy and, after the cancer becomes hormone independent, the anti-tumor cell antigen antibody (and optionally other agents as described herein) may be administered to the patient.

In an alternate embodiment, immunoconjugate antibodies may be used in combination with a naked antibody wherein the non-conjugated antibody is the same antibody, or a variant of the antibody, as that used in the immunoconjugate. Naked antibody can be administered first to either bind and block low affinity or non-specific binding sites. Alternatively, if the naked antibody has ADCC or CDC activity, it can be administered to clear as much of the target tumor cells as possible by one of these activities. Then, the immunoconjugate antibody can be administered to kill any remaining tumor cells. In this way, the immunoconjugate can be given in smaller doses and may reduce any side effects that may be associated with the toxin portion of the conjugate.

Suitable dosages for any of the above coadministered agents are those presently used and may be lowered due to the combined action (synergy) of the agent and anti-tumor cell antigen antibody.

For the prevention or treatment of disease, the appropriate dosage of antibody will depend on the type of disease to be treated, as defined above, the severity and course of the disease, whether the antibody is administered for preventive or therapeutic purposes, whether the antibody is conjugated to a drug or radioisotope, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg (e.g. 0.1-20 mg/kg) of antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. The preferred dosage of the antibody will be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, e.g. about six doses of the anti-tumor cell antigen antibody). Dose ranges can be is in the range from about 0.01 mg/kg to about 40 mg/kg, from about 0.01 mg/kg to about 30 mg/kg, from about 0.1 mg/kg to about 30 mg/kg, from about 1 mg/kg to about 30 mg/kg, from about 3 mg/kg to about 30 mg/kg, from about 3 mg/kg to about 25 mg/kg, from about 3 mg/kg to about 20 mg/kg, from about 5 mg/kg to about 15 mg/kg, or from about 7 mg/kg to about 12 mg/kg. It is recognized that the method of treatment may comprise a single administration of a therapeutically effective dose or multiple administrations of a therapeutically effective dose. Additionally, an initial higher loading dose, followed by one or more lower doses may be administered. An exemplary dosing regimen comprises administering an initial loading dose of about 4 mg/kg, followed by a weekly maintenance dose of about 2 mg/kg of the anti-tumor cell antigen antibody. However, other dosage regimens may be useful. The progress of this therapy is easily monitored by conventional techniques and assays.

The current application contemplates a method for delivering a diagnostic/detection or therapeutic agent to a target cell. The diagnostic/detection agent may be an agent previously described, such as a photoactive agent, a radionuclide or a contrast agent. The therapeutic agent may also be an agent previously described, such as a radionuclide, an immunomodulator, a hormone or hormone antagonist, an enzyme or enzyme inhibitor, a photoactive therapeutic agent, a cytotoxic agent and a combination thereof. The method contemplates providing the composition of the invention and administering it to a patient in need thereof. The antibodies or immunoconjugates of the invention may be given first, and following clearance from the bloodstream, a carrier molecule may be administering comprising the diagnostic/detection or therapeutic agent or combinatipn thereof wherein the carrier molecule will bind to the antibodies of the invention. The carrier molecule may bind to one or more sites on the antibodies or immunoconjugates of the invention.

Aside from administration of the antibody protein to the patient, the present application contemplates administration of the antibody by gene therapy. Such administration of nucleic acid encoding the antibody is encompassed by the expression "administering a therapeutically effective amount of an antibody". See, for example, WO96/07321 published Mar. 14, 1996 concerning the use of gene therapy to generate intracellular antibodies.

There are two major approaches to getting the nucleic acid (optionally contained in a vector) into the patient's cells; in vivo and ex vivo. For in vivo delivery the nucleic acid is injected directly into the patient, usually at the site where the antibody is required. For ex vivo treatment, the patient's cells are removed, the nucleic acid is introduced into these isolated cells and the modified cells are administered to the patient either directly or, for example, encapsulated within porous membranes which are implanted into the patient (see, e.g. U.S. Pat. Nos. 4,892,538 and 5,283,187). There are a variety of techniques available for introducing nucleic acids into viable cells. The techniques vary depending upon whether the nucleic acid is transferred into cultured cells in vitro, or in vivo in the cells of the intended host. Techniques suitable for the transfer of nucleic acid into mammalian cells in vitro include the use of liposomes, electroporation, microinjection, cell fusion, DEAE-dextran, the calcium phosphate precipitation method, etc. A commonly used vector for ex vivo delivery of the gene is a retrovirus.

The currently preferred in vivo nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, and proteins that target intracellular localization and enhance intracellular half-life. The technique of receptor-mediated endocytosis is described, for example, by Wu et al., J. Biol. Chem. 262:4429-4432 (1987); and Wagner et al., Proc. Natl. Acad. Sci. USA 87:3410-3414 (1990). For review of the currently known gene marking and gene therapy protocols see Anderson et al., Science 256:808-813 (1992). See also WO 93/25673 and the references cited therein.

### VIII. Safety studies

The antibodies of the invention will be examined for safety and toxicological characteristics. Guidelines for these types of studies can be found in the document issued by the USDA CBER division, "Points to Consider in the Manufacture and Testing of Monoclonal Antibody Products for Human Use" (see http://www.fda.gov/cber/gdlns/ptc_mab.pdf) incorporated herein by reference. In general, the candidate antibodies should be screened in preclinical studies using a number of human tissue samples and/or isolated human cell types to assess non-target tissue binding and cross reactivity. Following a satisfactory outcome from these human tissue studies, a panel of tissue samples or isolated cells from a variety of animal species can be screened to identify a suitable species for use in general toxicological studies. If no cross reactive animal species is identified, other types of models may be deemed appropriate. These other models can include studies such as xenograft models, where human tumor cells are implanted into a rodent host, or the use of a surrogate monoclonal antibody which recognizes the corresponding tumor-cell antigen in the animal species chosen for the toxicological studies. It should be appreciated that the data from these types of alternate models will be first approximations and proceeding into higher species should be done with caution.

For a candidate naked antibody, studies looking at simple tolerability can be performed. In these studies the candidate therapeutic antibody an attempt to characterize the therapeutic index of the candidate molecule is made by observing any dose-dependent pharmacodynamic effects. A broad range of doses should be use (for example from 0.1 mg/kg to 100 mg/kg). Differences between tumor cell antigen number, affinity of the candidate antibody for the cross reactive animal target and differences in cellular response following binding of the antibody should be considered in estimating therapeutic index. Pharmacodynamic and pharmacokinetic studies should also be carried out in an appropriate animal model to help guild initial dose considerations when the candidate antibody is tested in humans.

For candidate immunoconjugates, stability studies of the conjugate must be performed in vivo. Optimally, pharmacodynamic and pharmacokinetic studies should be carried out on the individual components of the immunoconjugate to determine the consequences of any breakdown products from the candidate immunoconjugate. Pharmacodynamic and pharmacokinetic studies should also be carried out as above in an appropriate animal model to help guild initial dose considerations. Additional consideration must be given to safety study design when the drug will be given in combination with pretreatment with naked antibody. Safety studies must be carried out with the naked antibody alone, and studies must be designed with the immunoconjugate keeping in mind that the ultimate doses of immunoconjugate will be lower in this type of treatment regimen.

For radio-immunoconjugates, animal tissue distribution studies should be carried out to determine biodistribution data. In addition, an accounting of metabolic degradation of the total dose of administered radioactivity should be performed with both early and late time points being taken. Radio-immunoconjugates can be tested for stability in vitro using serum or plasma, and methods should be developed to measure the percentages of free radionuclide, radio-immunoconjugate and labeled, non- antibody compounds.

### IX. Articles of Manufacture

In another embodiment of the invention, an article of manufacture containing materials useful for the treatment of the disorders described above is provided. The article of manufacture comprises a container and a label or package insert on or associated with the container. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is an anti-tumor cell antigen antibody. The label or package insert indicates that the composition is used for treating the condition of choice, such as cancer. In one embodiment, the label or package inserts indicates that the composition comprising the antibody which binds the tumor cell antigen can be used to treat cancer which expresses a tumor cell antigen. The label or package insert may also indicate that the composition can be used to treat cancer, wherein the cancer is not characterized by overexpression of the tumor cell antigen. For example, whereas the present package insert for HERCEPTIN.RTM. indicates that the antibody is used to treat patients with metastatic breast cancer whose tumors overexpress the ErbB2 protein, the package insert herein may indicate that the antibody or composition is used to treat cancer regardless of the extent of the tumor cell antigen overexpression. In other embodiments, the package insert may indicate that the antibody or composition can be used to treat breast cancer (e.g. metastatic breast cancer); hormone independent cancer; prostate cancer, (e.g. androgen independent prostate cancer); lung cancer (e.g. non-small cell lung cancer); colon, rectal or colorectal cancer; or any of the other diseases or disorders disclosed herein. Moreover, the article of manufacture may comprise (a) a first container with a composition contained therein, wherein the composition comprises a first antibody which binds the tumor cell antigen and inhibits growth of cancer cells which overexpress the tumor cell antigen; and (b) a second container with a composition contained therein, wherein the composition comprises a second antibody which binds the tumor cell antigen. The article of manufacture in this embodiment of the invention may further comprises a package insert indicating that the first and second antibody compositions can be used to treat cancer. Moreover, the package insert may instruct the user of the composition

(comprising an antibody which binds the tumor cell antigen) to combine therapy with the antibody and any of the adjunct therapies described in the preceding section (e.g. a chemotherapeutic agent, an anti-angiogenic agent, an anti-hormonal compound, and/or a cytokine). Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### X. Non-Therapeutic Uses for the Anti-tumor Cell Antigen Antibody

The antibodies (e.g. the anti-tumor cell antigen antibodies) of the invention have further non-therapeutic applications.

For example, the antibodies may be used as affinity purification agents. In this process, the antibodies are immobilized on a solid phase such a Sephadex resin or filter paper, using methods well known in the art. The immobilized antibody is contacted with a sample containing the tumor cell antigen protein (or fragment thereof) to be purified, and thereafter the support is washed with a suitable solvent that will remove substantially all the material in the sample except the tumor cell antigen protein, which is bound to the immobilized antibody. Finally, the support is washed with another suitable solvent, such as glycine buffer, pH 5.0, that will release the tumor cell antigen protein from the antibody.

Anti-tumor cell antigen antibodies may also be useful in diagnostic assays for the tumor cell antigen protein, e.g., detecting its expression in specific cells, tissues, or serum.

For diagnostic applications, the antibody typically will be labeled with a detectable moiety. Numerous labels are available which can be generally grouped into the following categories:
(a) Radionuclides such as those previously discussed. The antibody can be labeled with the radioisotope using the techniques described in Current Protocols in Immunology, Volumes 1 and 2, Coligen et al., Ed. Wiley-Interscience, New York, N.Y., Pubs. (1991) for example and radioactivity can be measured using scintillation counting.
(b) Fluorescent labels such as rare earth chelates (europium chelates) or fluorescein and its derivatives, rhodamine and its derivatives, dansyl, Lissamine, phycoerythrin and Texas Red are available. The fluorescent labels can be conjugated to the antibody using the techniques disclosed in Current Protocols in Immunology, supra, for example. Fluorescence can be quantified using a fluorimeter.
(c) Various enzyme-substrate labels are available and U.S. Pat. No. 4,275,149 provides a review of some of these. The enzyme generally catalyzes a chemical alteration of the chromogenic substrate which can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (e.g., firefly luciferase and bacterial luciferase; U.S. Pat. No. 4,737,456), luciferin, 2,3-dihydrophthalazinediones, malate dehydrogenase, urease, peroxidase such as horseradish peroxidase (HRPO), alkaline phosphatase, beta.-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed J. Langone & H. Van Vunakis), Academic press, New York, 73:147-166 (1981).

Examples of enzyme-substrate combinations include, for example:
(i) Horseradish peroxidase (HRPO) with hydrogen peroxidase as a substrate, wherein the hydrogen peroxidase oxidizes a dye precursor (e.g., orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) .beta.-D-galactosidase (.beta.-D-Gal) with a chromogenic substrate (e.g., p-nitrophenyl-.beta.-D-galactosidase) or fluorogenic substrate 4-methylumbelliferyl-.beta.-D-galactosidase.

Numerous other enzyme-substrate combinations are available to those skilled in the art. For a general review of these, see U.S. Pat. Nos. 4,275,149 and 4,318,980.

Sometimes, the label is indirectly conjugated with the antibody. The skilled artisan will be aware of various techniques for achieving this. For example, the antibody can be conjugated with biotin and any of the three broad categories of labels mentioned above can be conjugated with avidin, or vice versa. Biotin binds selectively to avidin and thus, the label can be conjugated with the antibody in this indirect manner. Alternatively, to achieve indirect conjugation of the label with the antibody, the antibody is conjugated with a small hapten (e.g., digoxin) and one of the different types of labels mentioned above is conjugated with an anti-hapten antibody (e.g., anti-digoxin antibody). Thus, indirect conjugation of the label with the antibody can be achieved.

In another embodiment of the invention, the anti-tumor cell antigen antibody need not be labeled, and the presence thereof can be detected using a labeled antibody which binds to the tumor cell antigen antibody.

The antibodies of the present invention may be employed in any known assay method, such as competitive binding assays, direct and indirect sandwich assays, and immunoprecipitation assays. Zola, Monoclonal Antibodies: A Manual of Techniques, pp. 147-158 (CRC Press, Inc. 1987).

For immunohistochemistry, the tumor sample may be fresh or frozen or may be embedded in paraffin and fixed with a preservative such as formalin, for example.

The antibodies may also be used for in vivo diagnostic assays. Generally, the antibody is labeled with a radionuclide so that the tumor can be localized using immunoscintiography. As a matter of convenience, the antibodies of the present invention can be provided in a kit, ie., a packaged combination of reagents in predetermined amounts with instructions for performing the diagnostic assay. Where the antibody is labeled with an enzyme, the kit will include substrates and cofactors required by the enzyme (e.g., a substrate precursor which provides the detectable chromophore or fluorophore). In addition, other additives may be included such as stabilizers, buffers (e.g., a block buffer or lysis buffer) and the like. The relative amounts of the various reagents may be varied widely to provide for concentrations in solution of the reagents which substantially optimize the sensitivity of the assay. Particularly, the reagents may be provided as dry powders, usually lyophilized, including excipients which on dissolution will provide a reagent solution having the appropriate concentration.

Further details of the invention are illustrated by the following non-limiting Examples. The disclosures of all citations in the specification are expressly incorporated herein by reference.

### XI. EXAMPLES

### EXAMPLE 1

Selection of Tumor Associated Antigens for targeting

### A. Laser dissection of tumorous cells and adjacent normals and production of RNA from dissected cells.

Normal and cancerous tissues were collected from patients using laser capture microdissection (LCM), and RNA was prepared from these tissues, using techniques which are well known in the art (see, e.g., Ohyama et al. (2000) Biotechniques 29:530-6; Curran et al. (2000) Mol. Pathol. 53:64-8; Suarez-Quian et al. (1999) Biotech'iques 26:328-35; Simone et al. (1998) Trends Gerzet 14:272-6; Conia et al. (1997) J. Clin. Lab. Anal. 11:28-38; Emmert-Buck et al. (1996) Science 274:998-1001). Because LCM provides for the isolation of specific cell types to provide a substantially homogenous cell sample, this provided for a similarly pure RNA sample.

### B. Microarray analysis

Production of cDNA: Total RNA produced from the dissected cells was then used to produce cDNA using an Affymetrix Two-cycle cDNA Synthesis Kit (cat# 900432). 8 µL of total RNA was used with 1 µL T7-(dT) 24 primer (50 pmol/ µL) in an 11 µL reaction which was heated to 70° C for 12 minutes. The mixture was then cooled to room temperature for five minutes. 9 µL master mix (4 µL 5x 1^{st} strand cDNA buffer, 2 µL 0.1 M DTT, 1 µL 10 mM dNTP mix, 2 µL Superscript II (600 U/ µL)) was added and the mixture was incubated for 2.5 hours at 42° C (total volume of the mixture was 20 µL). Following Cooling on ice, the 2^{nd} strand synthesis was completed as follows: 20 µL mixture from above was mixed with 130 µL second strand master mix (91 µL water, 30 µL 5x Second Strand Reaction Buffer, 3 µL 10 mM dNTP mix, 1 µL 10 U/ µL e. coli DNA ligase, 4 µL 10 U/ µL E. coli DNA polymerase I, 1 µL 2 U/ µL e. coli Rnase H) and was incubated for 2 hours at 16°C for 10 minutes. Following cooling on ice, the dsDNA was purified from the reaction mixture. Briefly, a QiaQuick PCT Purification Kit was used (Qiagen, cat# 28104), and 5 volumes of buffer PB was added to 1 volume of the cDNA mixture. The cDNA was then purified on a QIAquick spin column according to manufacture's directions, yielding a final volume of 60 µL.

Production of biotin-labeled cRNA. The cDNA produced and purified above was then used to make biotin labeled RNA as follows: The 60 µL of cDNA recovered from the QIAQuick column was reduced to a volume of 22 µL in a medium heated speed vacuum. This was then used with an ENZO BioArray High Yield RNA Transcription Kit (cat# 4265520). Briefly, a master mix containing 4 µL 10x HY Reaction buffer, 4 µL 10x Biotin-Labeled Ribonucleotides, 4 µL DTT, 4 µL Rnase Inhibitor Mix, and 2 µL T7 RNA Polymerase was added to the 22 µL of purified cDNA, and left tp incubate at 37 °C for 4 to 6 hours. The reaction was then purified using a Qiagen RNeasy Kit (cat# 74104) according to manufacturer's directions.

Fragmentation of cRNA. 15 to 20 µg of cRNA from above was mixed with 8 µL of 5x Fragmentation Buffer (200mM Tris-acetate, pH 8.1, 500mM Potassium acetate, 150mM Magnesium acetate) and water to a final volume of 40 µL. The mixture was incubated at 94 °C for 35 minutes. Typically, this fragmentation protocol yields a distribution of RNA fragments that range in size from 35 to 200 bases. Fragmentation was confirmed using TAE agarose electrophoresis.

Array Hybridization. The fragmented cRNA from above was then used to make a hybridization cocktail. Briefly, the 40 µL from above was mixed with 1 mg/mL human Cot DNA and a suitable control oligonucleotide. Additionally, 3 mg of Herring Sperm DNA (10 mg/mL) was added along with 150 µL 2x Hybridization buffer (100 mM MES, 1 M NaCl, 20 mM EDTA, 0.01 % Tween-20) and water to a final volume of 300 µL. 200 µL of this solution was then loaded onto the U133 array (Affymetrix cat # 900370) and incubated at 45 °C with a constant speed of 45 rpm overnight. The hybridization buffer was then removed and the array was washed and stained with 200 µL Non-stringent wash buffer (6X SSPE, 0.01% Tween-20) and using a GeneChip Fluidics Station 450 (Affymetrix, cat# 00-0079) according to manufacturer's protocol.

Scanning array. The array from above was then scanned using a GeneChip Scanner 3000 (Affymetrix cat# 00-0217) according to manufacturer's protocol.

Selection of potential tumor cell antigen targets. The tumor antigens were selected for targeting by comparison of the expression level of the antigen in the tumor cells (either primary tumors or metastases) versus neighboring healthy tissue or with pooled normal tissue. Tumor antigens selected showed at least a 3 fold (300%) increased expression relative to surrounding normal tissue, where this 3 fold increase is seen in comparison with a majority of pooled, commercially available normal tissue samples (Reference standard mix or RSM, pools are made for each tissue type). Table 2 below presents the fold increase data from the array analysis for KIAA1815, LOC157378, FU20421, DSCD75, GPR160, GPCR41, and SLC1A5, where the numbers represent the percent of patient samples analyzed that showed a 2-, 3- or 5-fold increase in expression in comparison to normal tissues.

Table 2- Microarray results for KIAA1815, LOC157378, FL320421, DSCD75, GPR160, GPCR41, and SLC1A5:

**Table 2a- FLJ23390**

| Patient Type | Number of patients | 2-fold increase | 3-fold increase | 5-fold increase |
|---|---|---|---|---|
| Colon Primary vrs Normal RSM | 25 | 20 | 4 | 0 |
| Colon metastasis vrs Normal RSM | 33 | 0 | 0 | 0 |
| Breast Primary vrs Normal RSM | 20 | 100 | 75 | 25 |
| Prostate Primary vrs Normal RSM | 22 | 0 | 0 | 0 |
| Prostate Primary vrs Normal | 17 | 6 | 6 | 0 |

**Table 2b- LOC157378**

| Patient Type | Number of patients | 2-fold increase | 3-fold increase | 5-fold increase |
|---|---|---|---|---|
| Colon Primary vrs Normal RSM | 26 | 50 | 23 | 9 |
| Colon metastasis vrs Normal RSM | 32 | 50 | 44 | 22 |
| Breast Primary vrs Normal RSM | 49 | 24 | 6 | 2 |
| Prostate Primary vrs Normal RSM | 21 | 14 | 0 | 0 |
| Prostate Primary vrs Normal | 15 | 40 | 7 | 0 |

**Table 2c- FU20421**

| Patient Type | Number of patients | 2-fold increase | 3-fold increase | 5-fold increase |
|---|---|---|---|---|
| Colon Primary vrs Normal RSM | 26 | 35 | 15 | 0 |
| Colon metastasis vrs Normal RSM | 33 | 24 | 3 | 0 |
| Breast Primary vrs Normal RSM | 50 | 70 | 40 | 14 |
| Prostate Primary vrs Normal RSM | 22 | 14 | 5 | 0 |
| Prostate Primary vrs Normal | 17 | 24 | 0 | 0 |

**Table 2d- DCSD75**

| Patient Type | Number of patients | 2-fold increase | 3-fold increase | 5-fold increase |
|---|---|---|---|---|
| Colon Primary vrs Normal RSM | 27 | 52 | 37 | 7 |
| Colon metastasis vrs Normal RSM | 33 | 76 | 36 | 9 |
| Breast Primary vrs Normal RSM | 49 | 22 | 2 | 0 |
| Prostate Primary vrs Normal RSM | 22 | 5 | 0 | 0 |
| Prostate Primary vrs Normal | 16 | 12 | 0 | 0 |

**Table 2e- GPR160**

| Patient Type | Number of patients | 2-fold increase | 3-fold increase | 5-fold increase |
|---|---|---|---|---|
| Colon Primary vrs Normal RSM | 26 | 23 | 8 | 0 |
| Colon metastasis vrs Normal RSM | 33 | 12 | 0 | 0 |
| Breast Primary vrs Normal RSM | 46 | 24 | 9 | 0 |
| Prostate Primary vrs Normal RSM | 22 | 36 | 9 | 5 |
| Prostate Primary vrs Normal | 17 | 59 | 24 | 6 |

**Table 2f- GPCR41**

| Patient Type | Number of patients | 2-fold increase | 3-fold increase | 5-fold increase |
|---|---|---|---|---|
| Colon Primary vrs Normal RSM | 23 | 48 | 17 | 4 |
| Colon metastasis vrs Normal RSM | 30 | 77 | 50 | 13 |
| Breast Primary vrs Normal RSM | 43 | 63 | 40 | 23 |
| Prostate Primary vrs Normal RSM | 0 | 0 | 0 | 0 |
| Prostate Primary vrs Normal | 3 | 33 | 33 | 33 |

**Table 2g- SLC1A5**

| Patient Type | Number of patients | 2-fold increase | 3-fold increase | 5-fold increase |
|---|---|---|---|---|
| Colon Primary vrs Normal RSM | 26 | 15 | 4 | 0 |
| Colon metastasis vrs Normal RSM | 31 | 13 | 0 | 0 |
| Breast Primary vrs Normal RSM | 39 | 18 | 3 | 0 |
| Prostate Primary vrs Normal RSM | 1 | 100 | 0 | 0 |
| Prostate Primary vrs Normal | 6 | 67 | 50 | 33 |

### EXAMPLE 2- Expression analysis of cancerous versus normal tissue

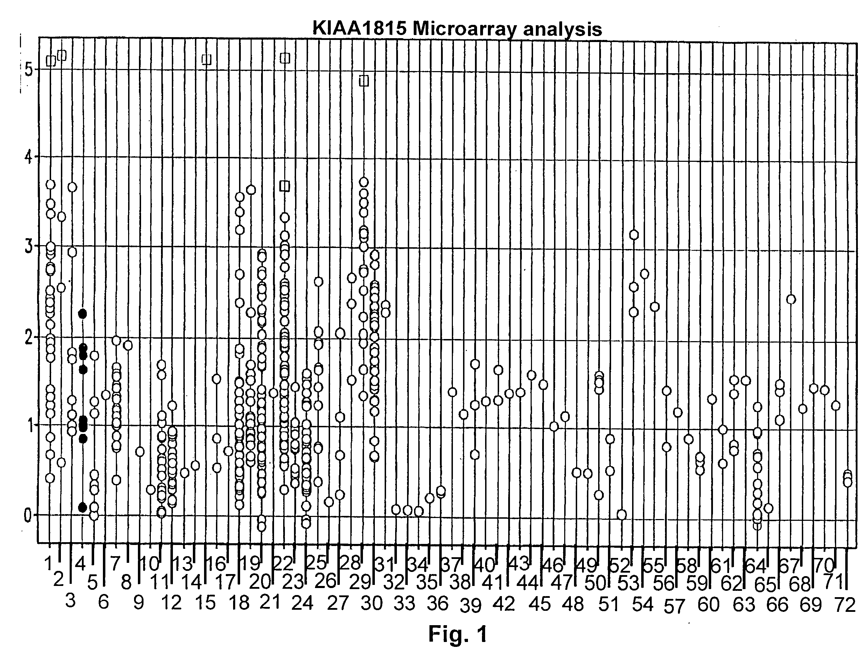

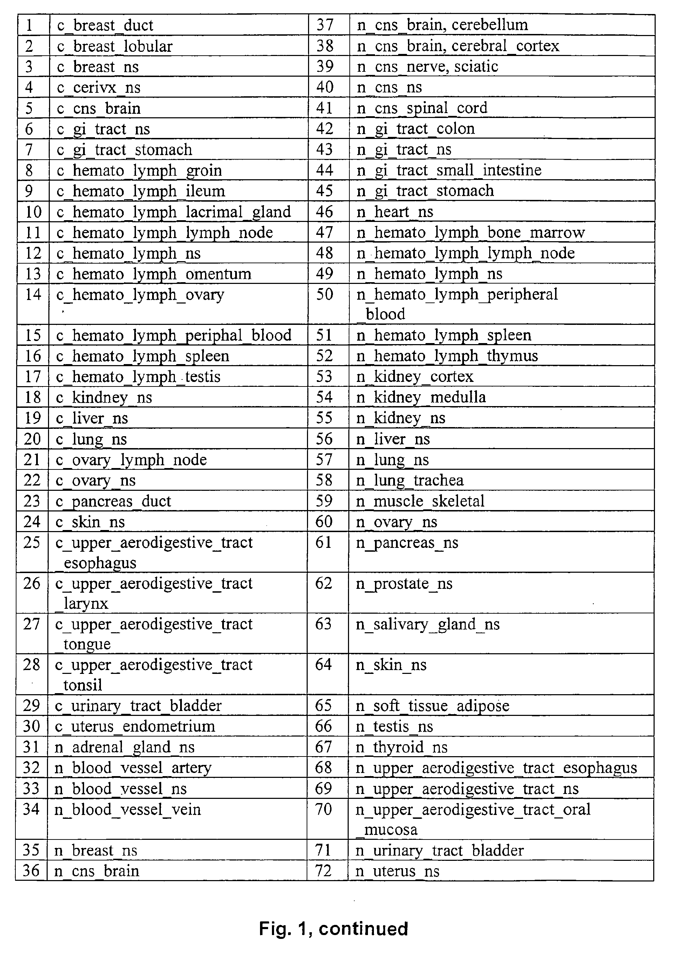

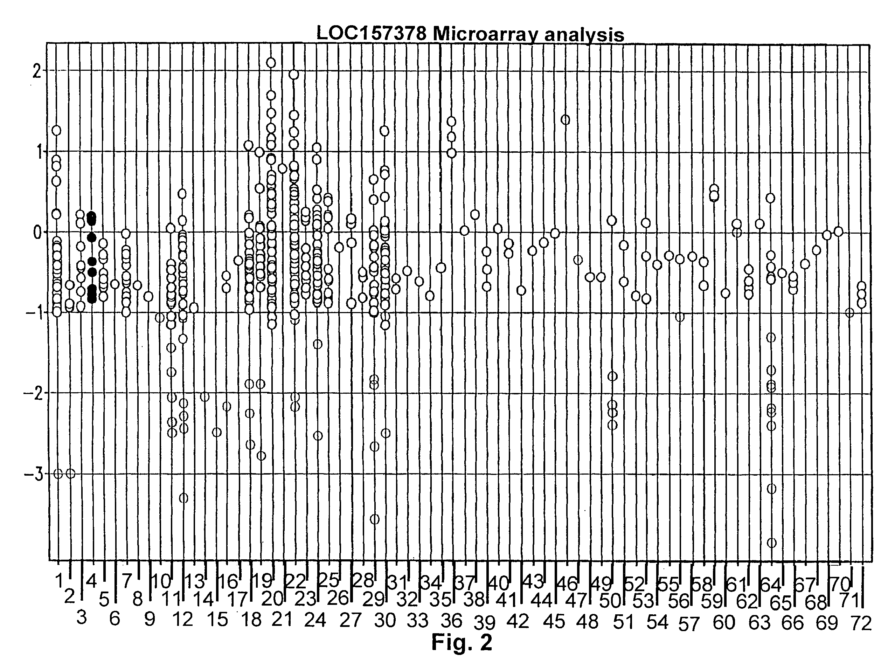

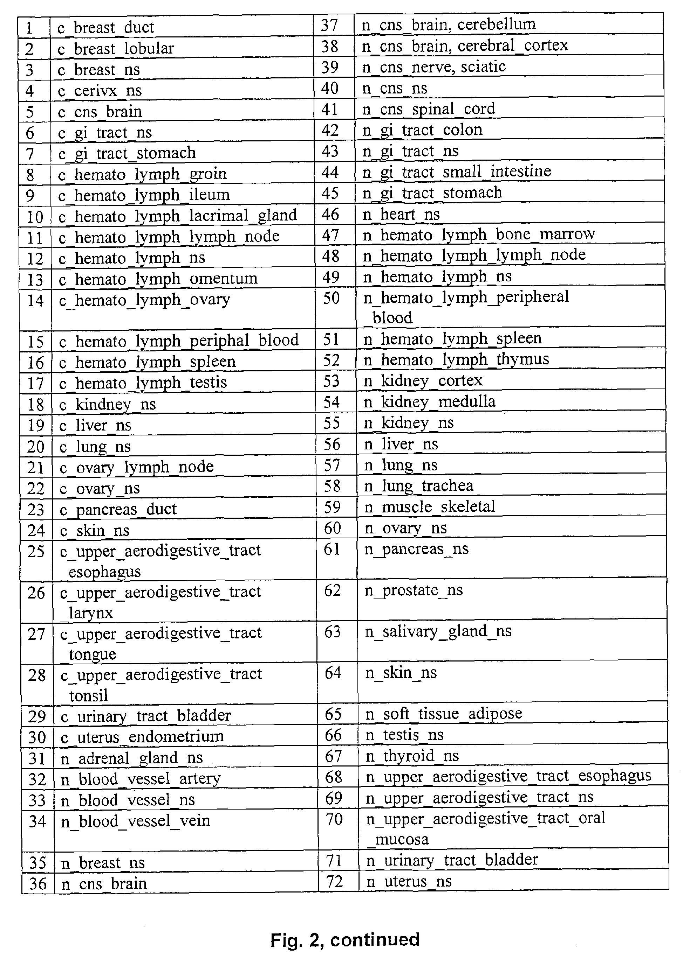

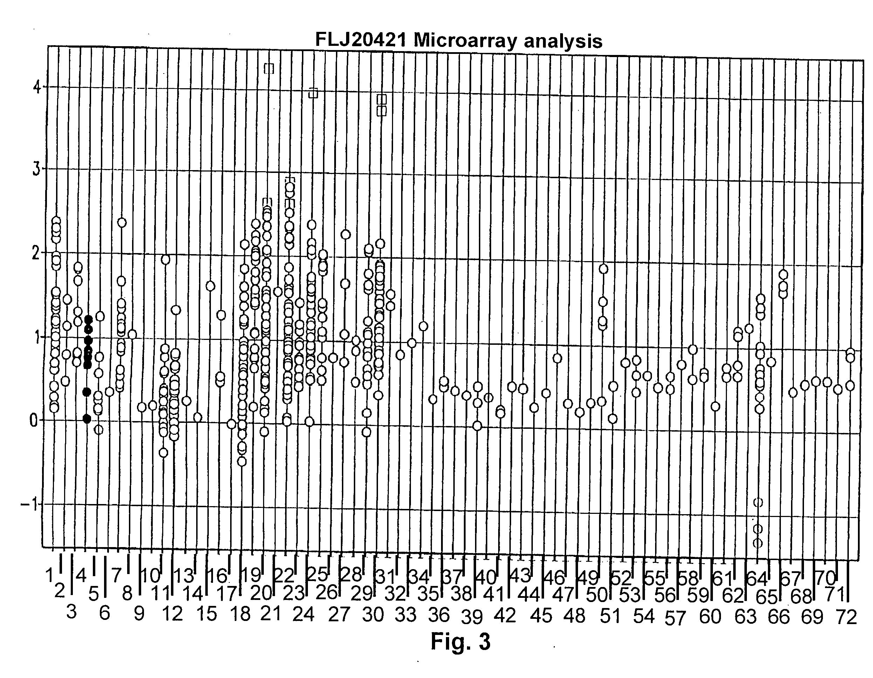

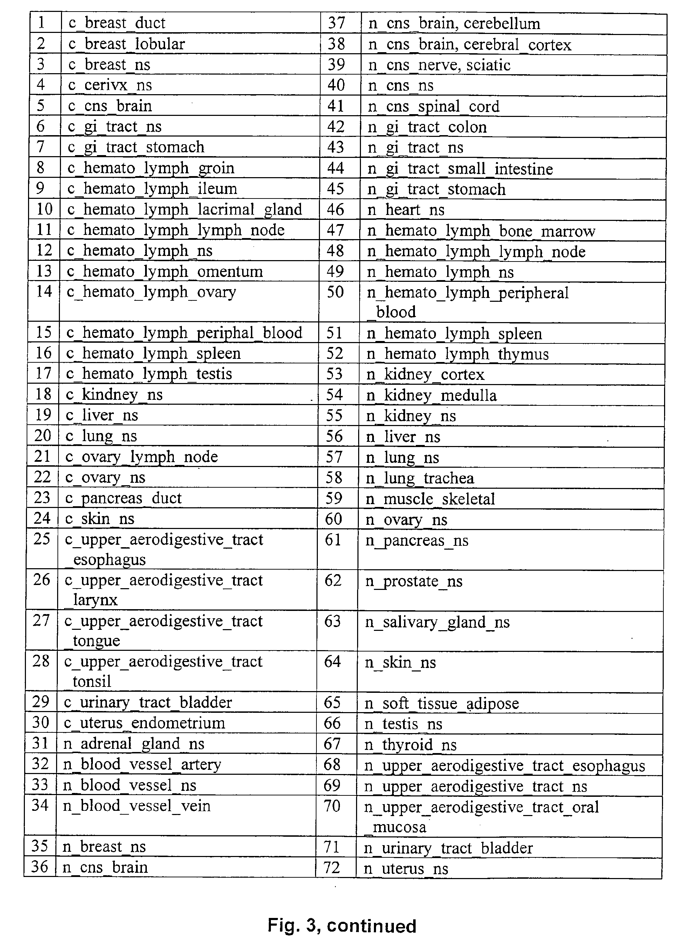

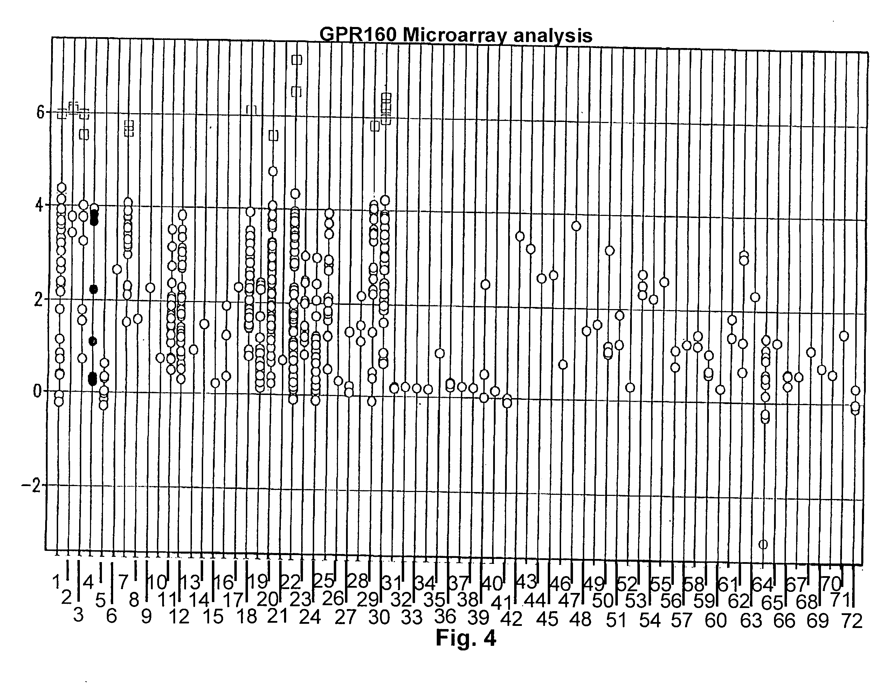

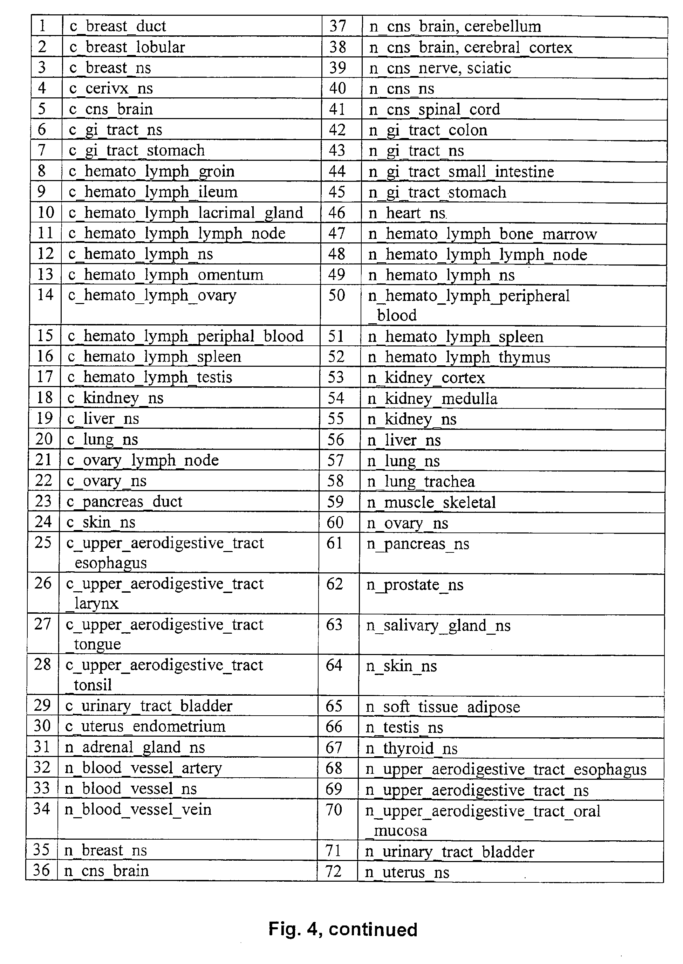

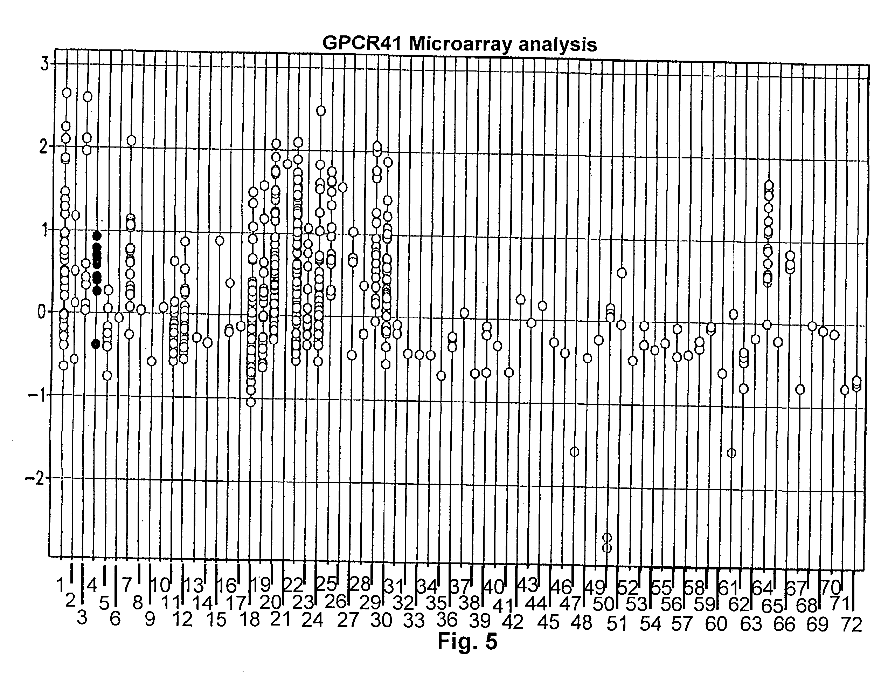

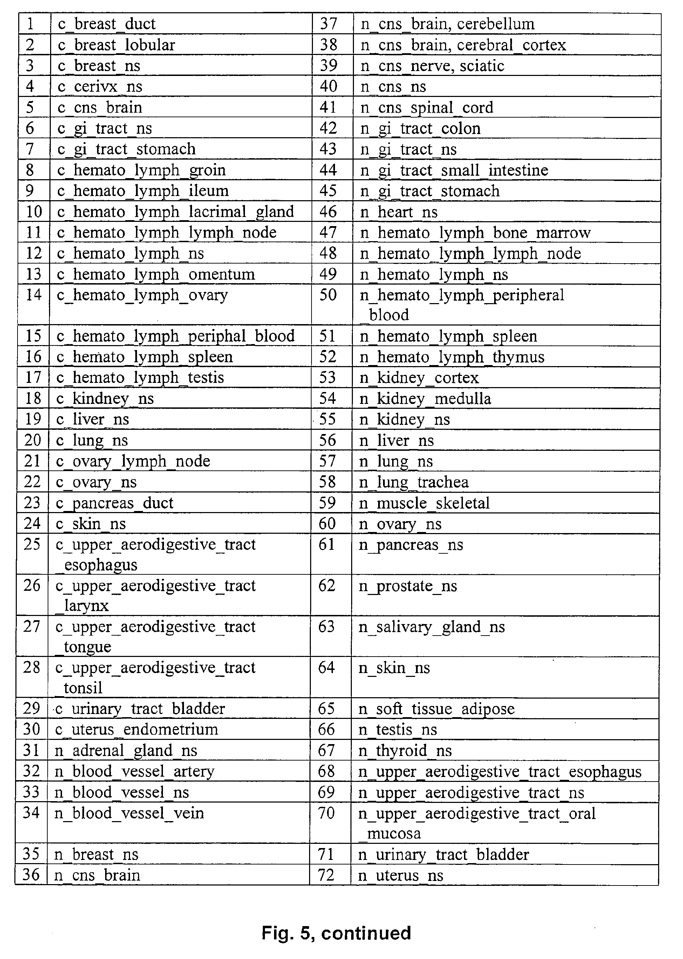

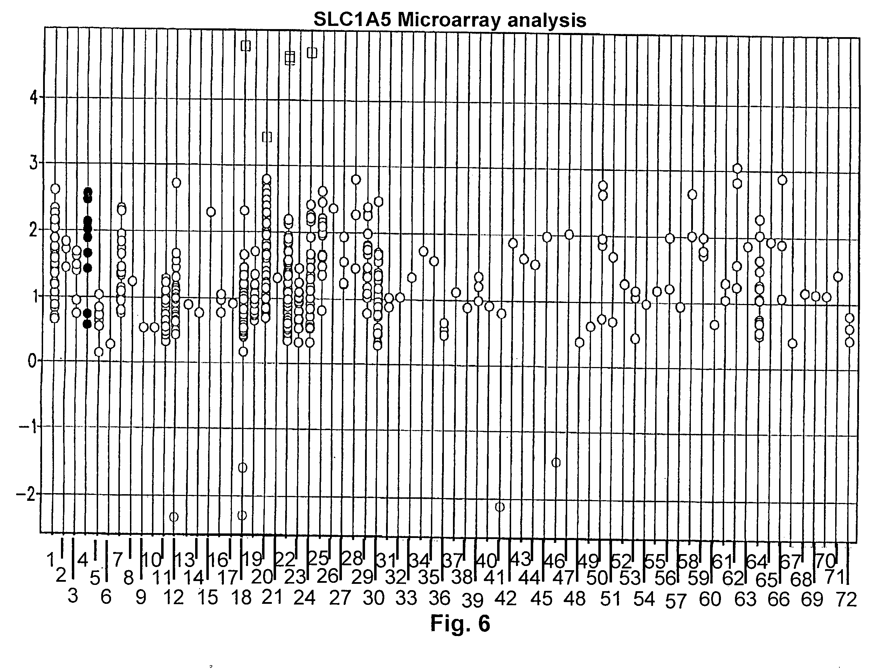

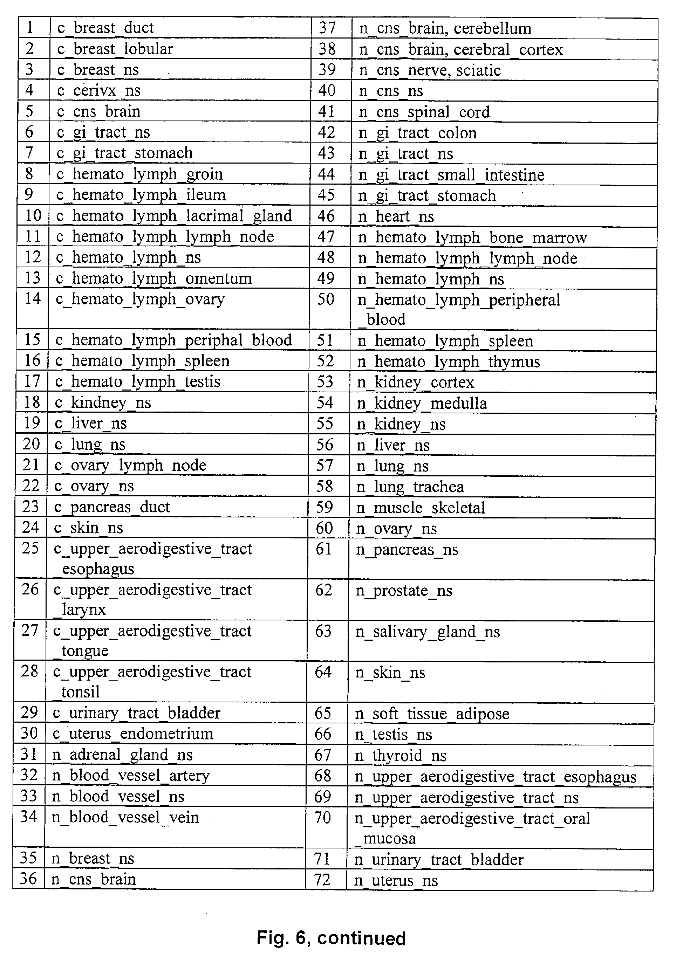

The microarray procedure used for Example 1 was also used to look broadly at expression in a number of cancerous tumor types and a number of tissues from normal samples. The production of cDNA, RNA and the hybridization conditions were all those used in Example 1, with the exception that a U133 Plus 2.0 array was used (Affymetrix cat# 900470). DecisionSite software (Spotfire, Somerville, MA) and in-house developed Pipeline Pilot (SciTegic, San Diego, CA, in-house developer Josef Ringgenberg) protocols were was used to generate the graphical depictions shown in Figures 1- 6. In the graphical depictions, normal and cancerous tissue types are laid out along the horizontal axis. Cancerous tissues are labeled with a `c', for example, "c_breast_duct" which represents a breast cancer tissue sample and normal tissues are similarly represented with a 'n'. The tissue types are further labeled with respect to the type and subtype of the tissue, if known. For example "c_breast_duct" is a cancerous tissue from a breast cancer that was localized in a breast duct. If the subtype was not clear during surgical removal or was unknown, the label says, 'ns' for 'non-specified'. Each spot on the vertical axes represents a tissue sample from a single patient, and the height of each spot on the vertical axes (log 2 based) represents relative expression level of the probeset. Filled circles represent samples with expression levels in the linear detection range. Open circles represent an upper limit on gene expression in samples where the gene was below the probeset's detection limit. Open squares represent a lower limit on gene expression in samples where the probeset was saturated. Briefly, before performing an analysis, each probeset is calibrated by analyzing the behavior of its constituent probes across a large, diverse set of samples. This calibration determines the relative sensitivity of each probe, and the range of intensities within which the probeset response is linear between probes. Intensities below this range are called "undetected" while those above it are called "saturated". Because of variation in the hybridization and labeling efficiency between samples, each chip is normalized after applying the calibrations.

### EXAMPLE 3- Analysis of extracellular domain of tumor cell antigens.

Following identification of the tumor cell antigens above, the topology of the proteins was analyzed to identify theoretical extracellular domains. Two analysis algorithms were used that predict transmembrane domains and the results were compared. The first was TMpred (see K. Hofmann & W. Stoffel (1993) TMbase - A database of membrane spanning proteins segments Biol. Chem. Hoppe-Seyler 374,166). A second analysis program that was used was TMHMM (A. Krogh, B. Larsson, G. von Heijne, and E. L. L. Sonnhammer. Predicting transmembrane protein topology with a hidden Markov model: Application to complete genomes. Journal of Molecular Biology, 305(3):567-580, January 2001). The results for each of the tumor cell antigens are listed below in Table 3. In general, the results from the two sets of analyses agreed fairly well, although in some instances the algorithms could not predict with certainty one topological orientation over another. In those cases, both orientations are listed. Table 3 shows the position of the putative transmembrane helixes (TM helix) as well as the theoretical portions of the proteins that are located either on the outside or the inside of the plasma membrane. In addition, SignalP 3.0 (Bednsten et al, (2004) J Mol Biol. 2004 Jul 16;340(4):783-95) was also used to predict the presence of signal peptides and to predict where those peptides would be cleaved from the full-length protein. The portions of the proteins on the outside of the cell could serve as targets for antibody interaction.

**Table 3a- KIAA1815: accession number NP_079172, 902 aas.**

| **TMHMM** | | **IMpred** | |
|---|---|---|---|
| **location** | **position** | **location** | **position** |
| outside | 1 - 408 | outside | 1-74 |
| TM helix | 409 - 431 | TM helix | 75 - 92 |
| inside | 432 - 450 | inside | 93-398 |
| TM helix | 451 - 473 | TM helix | 399 - 422 |
| outside | 474 - 487 | outside | 422 |
| TM helix | 488 - 510 | TM helix | 418 - 436 |
| inside | 511 - 521 | inside | 437-462 |
| TM helix | 522 - 544 | TM helix | 463 - 487 |
| outside | 545 - 548 | outside | 488 - 489 |
| TM helix | 549 - 568 | TM helix | 490- 512 |
| inside | 569 - 579 | inside | 513 - 537 |
| TM helix | 580 - 602 | TM helix | 538 - 564 |
| outside | 603 - -621 | outside | 565 - 581 |
| TM helix | 622 - 644 | TM helix | 582 - 602 |
| inside | 645 - 650 | inside | 603 - 623 |
| TM helix | 651 - 673 | TM helix | 624 - 641 |
| outside | 674 - 904 | outside | 642 - 653 |
| | | TM helix | 654 - 674 |
| | | inside | 675- 904 |

| | | | |
|---|---|---|---|
| SignalP 3.0 predicted no signal peptide | | | |

**Table 3b- BC017881: accession number NP_919267, 240 aas**

| **TMHMM** | | **IMpred #1** | | **IMpred #2** | |
|---|---|---|---|---|---|
| **location** | **position** | **location** | **position** | **location** | **position** |
| inside | 1 - 116 | inside | 1- 117 | outside | 1- 117 |
| TM helix | 117 - 139 | TM helix | 118 - 142 | TM helix | 118 - 143 |
| outside | 140 - 142 | outside | 143 - 148 | inside | 144 |
| TM helix | 143 - 165 | TM helix | 149 - 167 | TM helix | 145 - 164 |
| inside | 166 - 206 | inside | 168 - 211 | outside | 165- 211 |
| TM helix | 207 - 229 | TM helix | 212 - 230 | TM helix | 212 - 232 |
| outside | 230 - 240 | outside | 231 - 240 | inside | 233 - 240 |

| | | | | | |
|---|---|---|---|---|---|
| SignalP 3.0 predicted a signal peptide from aa 1- 48. | | | | | |

**Table 3c- FLJ20421: accession number AAH67814, 359 aas.**

| **TMHMM** | | **IMpred #1** | | **IMpred #2** | |
|---|---|---|---|---|---|
| **location** | **position** | **location** | **position** | **location** | **position** |
| inside | 1-6 | inside | 1-9 | outside | 1-7 |
| TM helix | 7-29 | TM helix | 10-28 | TM helix | 8-28 |
| outside | 30 - 359 | outside | 29 - 359 | inside | 29 - 359 |

| | | | | | |
|---|---|---|---|---|---|
| SignalP 3.0 predicted a signal peptide from aa 1- 50. | | | | | |

**Table 3d- DSCD75: accession number AAF65450, 208 aas**

| **TMHMM** | | **IMpred #1** | | **IMpred #2** | |
|---|---|---|---|---|---|
| **location** | **position** | **location** | **position** | **location** | **position** |
| inside | 1 | inside | 0 | outside | 0 |
| TM helix | 2-24 | TM helix | 1-19 | TM helix- | 1-19 |
| outside | 25 - 208 | outside | 20 - 208 | inside | 20 - 208 |

| | | | | | |
|---|---|---|---|---|---|
| SignalP 3.0 predicted a signal peptide from aa 1- 30. | | | | | |

**Table 3e- GPR160: accession number NP_055188, 338 aas**

| **TMHMM** | | **IMpred** | |
|---|---|---|---|
| **location** | **position** | **location** | **position** |
| outside | 1 - 21 | outside | 1- 25 |
| TM helix | 22-44 | TM helix | 26-43 |
| inside | 45 - 56 | inside | 44-56 |
| TM helix | 57 - 79 | TM helix | 57 - 79 |
| outside | 80 - 93 | outside | 80 - 93 |
| TM helix | 94 - 116 | TM helix | 94 - 121 |
| inside | 117 - 136 | inside | 122 -136 |
| TM helix | 137 - 156 | TM helix | 137 - 156 |
| outside | 157 - 175 | outside | 157 - 182 |
| TM helix | 176 - 198 | TM helix | 183 - 199 |
| inside | 199 -239 | inside | 200 - 243 |
| TM helix | 240 - 262 | TM helix | 244 - 265 |
| outside | 263 - 276 | outside | 266 - 276 |
| TM helix | 277 - 294 | TM helix | 277 - 294 |
| inside | 295 - 338 | | |

| | | | |
|---|---|---|---|
| SignalP 3.0 predicted a signal peptide from aa 1- 38. | | | |

**Table 3f- GPCR41: accession number AAH02917, 445 aas**

| **TMHMM** | | **IMpred** | |
|---|---|---|---|
| **location** | **position** | **location** | **position** |
| inside | 1-8 | inside | 1-8 |
| TM helix | 9 - 31 | TM helix | 9-30 |
| outside | 32-45 | outside | 31-49 |
| TM helix | 46-68 | TM helix | 50-68 |
| inside | 69-80 | inside | 69 - 111 |
| TM helix | 81 - 103 | TM helix | 112 - 133 |
| outside | 104 - 112 | outside | 134 - 145 |
| TM helix | 113 - 135 | TM helix | 146 - 167 |
| inside | 136 - 146 | inside | 168 - 194 |
| TM helix | 147 - 169 | TM helix | 195 - 211 |
| outside | 170 - 195 | outside | 212 - 270 |
| TM helix | 196 - 218 | TM helix | 271 - 297 |
| inside | 219 - 275 | inside | 298 - 311 |
| TM helix | 276 - 298 | TM helix | 312 - 339 |
| outside | 299 - 307 | outside | 339 |
| TM helix | 308 - 330 | TM helix | 335 - 359 |
| inside | 331 - 336 | inside | 360 - 370 |
| TM helix | 337 - 359 | TM helix | 371 - 389 |
| outside | 360 - 368 | outside | 390 - 403 |
| TM helix | 369 - 391 | TM helix | 404 - 424 |
| inside | 392 - 403 | inside | 425 - 445 |
| TM helix | 404 - 426 | | |
| outside | 427 - 445 | | |

| | | | |
|---|---|---|---|
| SignalP 3.0 predicted a signal peptide from aa 1-25. | | | |

**Table 3g- SLC1A5: accession number AAH00062, 541 aas**

| **TMHMM** | | **IMpred** | |
|---|---|---|---|
| **location** | **position** | **location** | **position** |
| inside | 1- 52 | inside | 1 - 50 |
| TM helix | 53 - 75 | TM helix | **51** - 72 |
| outside | 76 - 94 | outside | 73 - 98 |
| TM helix | 95 - 117 | TM helix | 99 - 119 |
| inside | 118 - 129 | inside | 120 - 126 |
| TM helix | 130 - 152 | TM helix | 127 - 145 |
| outside | 153 - 227 | outside | 146 - 227 |
| TM helix | 228 - 245 | TM helix | 228 - 296 |
| inside | 246 - 264 | inside | 247 - 264 |
| TM helix | 265 - 287 | TM helix | 265 - 284 |
| outside | 288 - 301 | outside | 285 - 304 |
| TM helix | 302 - 324 | TM helix | 305 - 329 |
| inside | 325 - 336 | inside | 330 - 335 |
| TM helix | 337 - 359 | TM helix | 336 - 361 |
| outside | 360 - 378 | outside | 362 - 376 |
| TM helix | 379 - 401 | TM helix | 377 - 404 |
| inside | 402 - 413 | inside | 405 - 413 |
| TM helix | 414 - 436 | TM helix | 414 - 432 |
| outside | 437 - 541 | outside | 433 - 541 |

| | | | |
|---|---|---|---|
| SignalP 3.0 predicted a signal peptide from aa 1- 35 | | | |

### EXAMPLE 4- Production of monoclonal antibodies to KIAA1815, LOC157378, FU20421, DSCD75, GPR160, GPCR41, and SLC1A5.

### A. PCR Cloning of KIAA1815, LOC157378, FLJ20421, DSCD75, GPR160, GPCR41, and SLC1A5.

RNA will be isolated from a population of human cancer cells expressing the cancer cell antigen essentially as described by Chirgwin et al., Biochemistry (1979) 17:5294. In brief, the cells will be washed twice with phosphate buffered saline (PBS) and lysed in 5 M guanidinium thiocyanate in the presence of 0.7 M 2-mercaptoethanol. The cell lysate will be layered on a discontinuous CsCl gradient (Chirgwin et al.) and centrifuged for 16 hours at 26,000 rpm in a Beckman SW28 rotor. The RNA will be recovered by dissolving the pellet in DEPC-treated H2O. The RNA will be precipitated with ethanol once, resuspended in DEPC-treated H2O, and stored at -70° C.

Total RNA (10 µg/reaction) will be converted to cDNA using random hexamer priming in 50 µl reaction buffer containing 500 units MLV-RT (Bethesda Research Laboratories, Bethesda, Md.), 5 µM random hexamer (Pharmacia, Piscataway, N.J.), 1 mM DTT, dNTP mix (0.5 mM each), 10 mM Tris-HCL pH 8.3, 50 mM KCl, 2.5 mM MgCl2 and 0.1 mg/ml BSA (bovine serum albumin). After incubation at 37° C. for 1 hour, the samples will be boiled for 3 minutes and stored at -70° C. The DNA encoding the KIAA1815, LOC157378, FLJ20421, DSCD75, GPR160, GPCR41, and SLC1A5 molecules will be generated by PCR using primers which contained sequences having homology to known KIAA1815, LOC157378, FU20421, DSCD75, GPR160, GPCR41, and SLC1A5 sequence, where the primers also encoded restriction sites useful for cloning. These primers will be based on the published cDNA coding sequences for KIAA1815, LOC157378, FLJ20421, DSCD75, GPR160, GPCR41, and SLC1A5. All primers will start with a C-G clamp at the 5' end followed by a restriction site for cloning.

For PCR amplification, 1 µl of cDNA will be mixed with 1 µl (10 picomoles) of a forward primer, 1 µl (10 picomoles) of a backward primer, and 47 µl of PCR mix. The PCR mix will consist of 1.25 units Taq polymerase (Perkin-Elmer/Cetus, Norwalk, Conn.), dNTP mix (0.2 mM each), 10 mM Tris-HCL pH 8.3, 50 mM KCl, 2.5 mM MgCl2 and 0.1 mg/ml BSA. The 50 µl of PCR mixture will be overlaid with 70 µl mineral oil and subjected to 25 cycles of amplification in a Perkin-Elmer/Cetus thermocycler (denaturation at 95° C. for 30 seconds, primer annealing at 55° C. for 30 seconds and extension at 72° C. for 1.5 minutes). PCR products will be obtained after 25 amplification cycles.

The amplification products will be isolated by size-fractionation. Before expression in baculovirus, the DNA sequence of each fragment will be confirmed by sequencing analysis to prevent the introduction of PCR-induced mutations.

The amplified fragments will be ligated to a suitable expression vector (for example pAcCB vector for use in a baculovirus expression system). The ligation products will be transformed into bacterial strain DH5α (Gibco/BRL, Gaithersburg Md.) and recombinant pAcC8 vectors will be selected on the basis of ampicillin resistance. Recombinant plasmids will be isolated from bacterial clones (Maniatis et al., Molecular Cloning: A Laboratory Manual (Cold Spring Harbor, N.Y.: Cold Spring Harbor Laboratories), 1982; Ausubel et al., Current Protocols in Molecular Biology (Media, Pa.: John Wiley and Sons)) and the presence of the insert of interest will be verified using polymerase chain reactions (see above). Large scale plasmid preparation will be performed by standard procedures (Ausubel et al.; Maniatis et al.; Sambrook et al., Molecular Cloning: A Laboratory Manual (Cold Spring Harbor, N.Y.: Cold Spring Harbor Laboratories), 1989).

### B. Baculovirus Expression of Human KIAA1815, LOC157378, FIJ20421, DSCD75, GPR160, GPCR41, and SLC1A5.

Sequences encoding human KIAA1815, LOC157378, FLJ20421, DSCD75, GPR160, GPCR41, and SLC1A5 will be individually recombined into the *Autographa californica* baculovirus (AcNPV) using the transfer vectors comprising the full-length DNA molecule of interest. The plasmids will be cotransfected with wild-type baculoviral DNA (2-10 pfu) (AcNPV; Summers et al., A Manual of Methods for Baculovirus Vectors and Insect Cell Culture Procedures, Texas Agricultural Experimental Station Bulletin No. 1555 (1987)) into Sf9 (*Spodoptera frugiperda*) cells at a density of 10⁶ cells/mL (Summers et al.). Recombinant baculovirus-infected Sf9 cells will be identified and clonally purified (Summers et al.). For cell surface expression of recombinant proteins the cells will be harvested after 48 hours of culture.

### C. Host Animal Immunization

Female BALB/c mice will be injected intraperitoneally at day 0 and day 14 with 5×10⁶ Sf9 cells infected with the virus containing the gene of interest or a control virus lacking any insert. At day 21, 100 µl of serum will be obtained to test for the presence of specific antibodies. After a rest period of at least two weeks, the mice will receive a final injection with Sf9 5×10⁶ cells infected with recombinant or control. Three days after this last injection, the spleen cells will be used for cell fusion.

### D. Generation of Hybridoma Clones

Splenocytes from immunized BALB/c mice will be fused with SP2/0 murine myeloma cells at a ratio of 10:1 using 50% polyethylene glycol as previously described by de Boer et al., J. Immunol. Meth. (1988) 113:143. The fused cells will be resuspended in complete IMDM medium supplemented with hypoxanthine (0.1 mM), aminopterin (0.01 mM), thymidine (0.016 mM) and 0.5 ng/ml hIL-6 (Genzyme, Cambridge, Mass.). The fused cells will be then distributed between the wells of 96-well tissue culture plates, so that each well contains on average a single growing hybridoma.

After 10-14 days the supernatants of the hybridoma populations will be screened for specific antibody production. Positive hybridoma cells will be cloned three times by limiting dilution in IMDM/FBS containing 0.5 ng/mL hIL-6.

### E. Use of phage display or transgenic animal technology

As an alternate approach to the use of antibody generation through hybridoma technology, antibodies of the invention may be generated using display technology or through the use of transgenic animals.

There are several kinds of display technology currently in use for purification of human antibodies (see Hoogenboom (2005) Nature Biotechnology 23(9): 1105- 1116). Phage display is one specific type of display technology that can be used to generate the human antibodies of the invention. The first panning round can be done using 1 - 20 µg of purified tumor cell antigen to coat wells of an ELISA plate. The phage library is added (for methodology associated with construction of a phage library and more detailed protocol information, see for example P.M. O'Brien and R. Aitken, Antibody Phage Display Humana Press, 2001) generally at a concentration of 1 x 10¹⁰ to 1x 10¹¹ pfu per well. Following incubation, the wells are washed and bound phage is eluted using salt or a low pH glycine solution. The eluted phage is then amplified by infecting bacteria, and the amplified and purified phage particles are then used for subsequent panning rounds. If desired, other panning techniques can be performed such as cell-based panning against cells that express the tumor cell antigen. Alternatively, liquid-based panning protocols can be carried out. Often lower concentrations of purified antigen can be used in later panning rounds to drive the selection of higher affinity antibodies. The number of panning rounds to be carried out varies according to the target and the library. Panning techniques such as panning on solid phase and panning on whole cells can also be used in alternate rounds. Once an antibody clone has been identified that produces an antibody with the desired qualities, the genes encoding that antibody can be isolated from the phage and expressed in a variety of bacterial, fungal or mammalian cell based expression systems.

For generation of human antibodies using transgenic mice, animals can be injected either with purified tumor cell antigen or with whole cells expressing that antigen. For example, mice can be given 8 injections overall in the following manner: On day 0, 10⁷ cells expressing the tumor cell antigen of interest are injected into footpads of the transgenic mice. On days 3, 7, 10, and 14, the mice can be given boosting injections, each injection containing 10⁷ cells expressing the tumor cell antigen of interest plus 10 µg of a CpG polynucleotide. On days 17, 21, and 27, the mice can be given additional boosting injections containing purified tumor cell antigen protein. Whole blood from the immunized transgenic mice can be harvested on day 31 and hybridomas prepared via standard techniques. The resulting hybridoma supernatants are screened as described above in `D'.

### F. Humanization/other modification

If robust ADCC or CDC activity is desired in the antibodies of the invention, the antibodies must contain human or humanized constant regions. Variable domains from the antibodies of the invention can be fused to human constant domains if the antibodies are derived from a non-human species. Following fusion to the human constant domain, the variable domain may additionally be humanized to lessen potential immunogenicity. Additionally, other genetic or recombinant modifications can be performed at this point, such as the construction of fusion proteins, affinity maturation, or additional effector function engineering to name a few examples.

### G. Purification of monoclonal antibodies

Antibodies of interest will be purified from the hybridoma cultures using standard Protein A chromatography, using techniques that are well known in the art. According to methods well known in the art, hybridoma supernatant will be harvested and any cells are removed by filtration after termination. The filtrate will be loaded onto a Protein A column (in multiple passes, if needed). The column will be washed and then the expressed and secreted immunoglobulin polypeptides will be eluted from the column. For preparation of antibody product, the Protein A pool will be held at a low pH (pH 3 for a minimum of 30 minutes and a maximum of one hour) as a viral inactivation step. An adsorptive cation exchange step will next be used to further purify the product. The eluate from the adsorptive separation column will be passed through a virus retaining filter to provide further clearance of potential viral particles. The filtrate will be further purified by passing through an anion exchange column in which the product does not bind. Finally, the purification process will be concluded by transferring the product into the formulation buffer through diafiltration. The retentate will be adjusted to a protein concentration of at least 1 mg/mL and a stabilizer is added.

### H. Characterization of binding affinity

The antibodies of the invention can be analyzed for their affinity for the targets of interest using BIACore microchip analysis. For example, a BIACore 2000 analyzer can be used in concert with a CM5 sensor chip (BIACore; Piscataway, NJ). Purified antigen protein will be immobilized to the sensor chip surface according to manufacturer's instructions, using a continuous flow of HBS-EP buffer (10mM HEPES, 0.15M NaCl, 3.4mM EDTA, 0.005% P-20, pH 7.4). Carboxyl groups on the sensor chip surfaces will be activated by injecting 60 uL of a mixture containing 0.2 M N-ethyl-N'- (dimethylaminopropyl)carbodiimide (EDC) and 0.05 M N- hydroxysuccinimide (NHS). Specific surfaces will be obtained by injecting recombinant tumor cell antigen diluted in 10mM acetate, pH 4.5 (BlACore, Inc.; Piscataway, NJ) at a concentration of 10 ug/mL to obtain a moderate surface density of 2,000 resonance units (RU). In certain embodiments, other concentrations of tumor cell antigen, such as 25 g/mL, may also be used. Excess reactive groups on the chip surfaces will be deactivated by injecting 60 uL of 1 M ethanolamine. A blank, mock-coupled reference surface will be prepared on each sensor chip. For mock-coupling, activation and inactivation steps are carried out without protein.

Monoclonal antibody candidates will be diluted into sample buffer (1X PBS + 0.005% P-20 + 0.1mg/mL BSA (fraction V, IgG free; Sigma, Inc.) filtered and degassed) to a concentration of 25nM and injected over the tumor cell antigen surface for two minutes at a flow rate of 80pUmin. For all analyses, the instrument running buffer will be 1X PBS (no calcium chloride, no magnesium chloride; Gibco Inc.) + 0.005% P-20 (filtered and degassed), and the temperature is set to 25°C. Antibody binding curves are compared qualitatively for binding signal intensity, as well as for dissociation rates.

### I. Conjugation

If the antibodies of the invention are to be used as partners in an immunoconjugate, the purified antibodies can now be conjugated to another moiety. For example, the antibody can be conjugated to a prodrug-activating enzyme for use in ADEPT technology, to radionuclides, toxins or other immunomodulatory agents.

EXAMPLE 5- In vitro screening of anti-Tumor cell antigen antibodies

### A. Screen for ADCC activity.

In Vitro ADCC Assay: To prepare chromium 51 -labeled target cells, tumor cell lines will be grown in tissue culture plates and harvested using sterile 10 mM EDTA in PBS. The detached cells will be washed twice with cell culture medium. Cells (5×10⁶) will be labeled with 200 µCi of chromium 51 (New England Nuclear/DuPont) at 37° C. for one hour with occasional mixing. Labeled cells will be washed three times with cell culture medium, then resuspended to a concentration of 1×10⁵ cells/mL. Cells will be used either without opsonization, or are opsonized prior to the assay by incubation with test antibody at 100 ng/mL and 1.25 ng/mL in PBMC assay or 20 ng/mL and 1 ng/mL in NK assay. Peripheral blood mononuclear cells will be prepared by collecting blood on heparin from normal healthy donors and diluted with an equal volume of phosphate buffered saline (PBS). The blood will be then layered over LYMPHOCYTE SEPARATION MEDIUM® (LSM: Organon Teknika) and centrifuged according to the manufacturer's instructions. Mononuclear cells will be collected from the LSM-plasma interface and washed three times with PBS. Effector cells will be suspended in cell culture medium to a final concentration of 1×10⁷ cells/mL. After purification through LSM, natural killer (NK) cells will be isolated from PBMCs by negative selection using an NK cell isolation kit and a magnetic column (Miltenyi Biotech) according to the manufacturer's instructions. Isolated NK cells will be collected, washed and resuspended in cell culture medium to a concentration of 2×10⁶ cells/mL. The identity of the NK cells will be confirmed by flow cytometric analysis. Varying effector:target ratios will be prepared by serially diluting the effector (either PBMC or NK) cells two-fold along the rows of a microtiter plate (100 µL final volume) in cell culture medium. The concentration of effector cells ranges from 1.0×10⁷/mL to 2.0×10⁴ /mL for PBMC and from 2.0×10⁶/mL to 3.9×10³/mL for NK. After titration of effector cells, 100 µL of chromium 51 -labeled target cells (opsonized or nonoponsonized) at 1×10⁵ cells/mL will be added to each well of the plate. This results in an initial effector:target ratio of 100:1 for PBMC and 20:1 for NK cells. All assays will be run in duplicate, and each plate contains controls for both spontaneous lysis (no effector cells) and total lysis (target cells plus 100 µL 1% sodium dodecyl sulfate, 1 N sodium hydroxide). The plates will be incubated at 37° C. for 18 hours, after which the cell culture supernatants will be harvested using a supernatant collection system (Skatron Instrument, Inc.) and counted in a Minaxi auto-gamma 5000 series gamma counter (Packard) for one minute. Results will be then expressed as percent cytotoxicity using the formula: % Cytotoxicity=(sample cpm-spontaneous lysis)/(total lysis-spontaneous lysis)×100.

### B. Screen for apoptotic activity.

This assay relies on the fact that annexin V has a high affinity for phosphostidylserine (PS), whose exposure to the outside of the cell is an early hallmark of the apoptotic process. Tumor cell antigen expressing cells will be cultured in Dulbecco's Modified Eagle Medium (D-MEM):Ham's F-12 (50:50) supplemented with 10% heat-inactivated FBS (Hyclone) and 2 mM L-glutarnine. The tumor antigen expressing tumor cells will be seeded at a density of 3 x 10⁶ per dish in 100 x 20 mm dishes and allowed to attach overnight. Cell surface PS will be detected using any of the commercially available Annexin V staining reagents, which can are based on the high affinity of annexin V for PS. The cell medium will be removed and replaced with fresh medium alone or medium containing 10 µg/mL of the monoclonal antibody. Following a three-day incubation period, monolayers will be washed with PBS and detached by trypsinization. Cells will be then centrifuged, resuspended in Ca²⁺ binding buffer and aliquoted into tubes. Tubes then receive labeled annexin (e.g. annexin V-FTIC) (1 µg/mL). Samples may be analyzed using a FACSCAN™, flow cytometer and FACSCONVERT™. CellQuest software (Becton Dickinson). Those antibodies that induce statistically significant levels of annexin binding relative to control will be selected as apoptosis-inducing antibodies.

### EXAMPLE 6- In vivo characterization of antibodies of interest.

Antibodies will be tested in vivo for the ability to prevent tumor formation and/or growth, inhibit growth or reduce size of established tumors, inhibit metastasis, or treat established metastatic lesions. Relevant tumor models for each tumor antigen will be selected based on tumor antigen expression in tumor cell lines, as evaluated by RNA or protein expression. Representative tumor cell lines for the tumor cell antigens described include, but are not restricted to, the following:
KIAA1815:
   T47D human breast
   COLO205 human colon
   HT29 human colon
   HCT116 human colon
LOC157378:
   A549 human nsclc
FLJ20421:
   MDA-MB-435
DSCD74:
   KM12 human colon
   COL0205
GPR160:
   T47D human breast
   MCF7 human breast
GPCR41:
   COLO205 human colon
   PC3-M-luc human prostate
   MDA-MB-435 human breast
SLC1A5
   COL0205 human colon
   SW620 human colon

### A. Tumor formation or growth

Immunocompromised mice at the age of 4-7 weeks old, average weight 20 g will be used for these studies.

### i) Treating colorectal cancer with anti-tumor cell antigen antibodies

Human colorectal cancer cell lines such as COL0205, KM-12 or SW620 will be implanted subcutaneously in athymic nude mice as described in Sheng et al. J. Clin. Invest. 99:2254-2259 (1997). The mice will be randomized into groups and treatment initiated on the day of tumor implantation, with 0.1-50 mg/kg of monoclonal antibody of interest administered twice weekly by injection intravenously or in the intraperitoneal cavity. Caliper measurements of tumor width and length will be used to calculate tumor volume. It is expected that tumor formation or growth will be inhibited as a result of the foregoing experiment.

### ii). Treating breast cancer with anti-tumor cell antigen antibodies.

Human breast cancer cell lines such as T47D, MCF-7 pr MDA-MB-435 will be implanted subcutaneously in athymic nude mice as described in Sheng et al. J. Clin. Invest. 99:2254-2259 (1997). Alternatively the cells will be implanted orthotopically in the mammary fat pad by direct injection or surgical implant. These tumor models are estrogen dependent for growth and female mice supplemented with estrogen will be used. The mice will be randomized into groups and treatment initiated on the day of tumor implantation, with 0.1-50 mg/kg of monoclonal antibody of interest administered twice weekly by injection intravenously or in the intraperitoneal cavity. Caliper measurements of tumor width and length will be used to calculate tumor volume. It is expected that tumor formation or growth will be inhibited as a result of the foregoing experiment.

### iii). Treating lung cancer with anti-tumor cell antigen antibodies.

Human non small cell lung cancer lines such as A549 will be implanted subcutaneously in athymic nude mice as described in Sheng et al. J. Clin. Invest. 99:2254-2259 (1997). The mice will be randomized into groups and treatment initiated on the day of tumor implantation, with 0.1-50 mg/kg of monoclonal antibody of interest administered twice weekly by injection intravenously or in the intraperitoneal cavity. Caliper measurements of tumor width and length taken twice weekly will be used to calculate tumor volume. It is expected that tumor formation or growth will be inhibited as a result of the foregoing experiment.

### iv). Treating prostate cancer with anti-tumor cell antigen antibodies.

The effect of the antibodies of the invention on human prostate cancer cells will be assessed against human prostate PC-3 or PC-M-luc tumor models. Cells will be injected subcutaneously into male mice. The mice will be randomized into groups (n=10 in each group) and treatment initiated intravenously or intraperitoneally (i.p.) on Day 0 with the antibodies of the invention or isotype MAb control. Animals will be treated twice weekly Tumor growth will be monitored using caliper measurements of tumor length and width taken twice weekly. Alternatively, for PC-3M luc cells, tumor growth with be monitored using bioluminescent imaging, with the Xenogen IVIS system, measuring luciferase signal in the tumor once or twice weekly.

### B. Metastasis inhibition

The effect of the antibodies of the invention on human prostate cancer cell metastasis and the growth of mestastatic lesions in the bone and soft tissue will be assessed using the human prostate PC-M-luc tumor model. Cells will be injected via the intracardiac route into male mice. The mice will be randomized into groups and treatment initiated intravenously or intraperitoneally (i.p.) on Day 0 with the antibodies of the invention or isotype MAb control. Animals will be treated twice weekly. Tumor growth in the bone and soft tissue with be monitored using bioluminescent imaging, with the Xenogen IVIS system, measuring luciferase signal in the tumor once weekly. Alternatively, animals will be imaged using the Xenogen IVIS (Xenogen 100 series with Living Image 2.5 software) system 1-3 weeks after implant and randomized into groups based on luciferase signal prior to initiating antibody treatment. Tumor growth response will be assessed by Xenogen IVIS imaging once weekly.

The following represent further embodiments of the present invention:
1. A method of diagnosing, detecting or treating cancer in a mammal, wherein the cancer expresses a tumor cell antigen that is elevated at least 3 fold relative to a normal adjacent tissue sample or pooled normal tissue sample, comprising administering to the mammal therapeutically effective amounts of an antibody or fragment thereof which binds to that tumor cell antigen, and wherein the tumor cell antigen is selected from the group of KIAA1815, LOC157378, FLJ20421, DSCD75, GPR160, GPCR41, and SLC1A5.
2. The antibody or fragment thereof of Embodiment 1, wherein said antibody is human, humanized, chimeric or fragment thereof.
3. The antibody or fragment thereof of Embodiment 1, wherein said fragment is selected from the group consisting of Fv, F(ab')2, Fab' and Fab.
4. The antibody or fragment thereof of any one of Embodiments 1-3, wherein said antibody or fragment thereof specifically binds to an extracellular domain of said tumor cell antigen, wherein the tumor cell antigen is selected from the group of KIAA1815, LOC157378, FLJ20421, DSCD75, GPR160, GPCR41, and SLC1A5.
5. The antibody or fragment there of according to Embodiment 4, wherein the antibody binds to an extracellular domain of KIAA1815, and wherein the extracellular domain is chosen from the group of amino acids from about positions 1- 408, from about positions 474- 489, from about positions 545- 581, from about positions 603- 621, from about positions 642- 653, and from about 674- 904.
6. The antibody or fragment thereof according to Embodiment 4, wherein the antibody binds to an extracellular domain of BC017881, and wherein the extracellular domain is chosen from the group of amino acids from about positions 1- 117, from about 140- 148, from about 165-211, and from about 230- 240.
7. The antibody or fragment thereof according to Embodiment 4, wherein the antibody binds to an extracellular domain of FLJ20421, and wherein the extracellular domain is from about amino acid positions 30- 359.
8. The antibody or fragment thereof according to Embodiment 4, wherein the antibody binds to an extracellular domain of DSCD75, and wherein the extracellular domain is from about amino acid positions 20- 208.
9. The antibody or fragment thereof according to Embodiment 4, wherein the antibody binds to an extracellular domain of GPR160, and wherein the extracellular domain is chosen from the group of amino acids from about positions 1- 15, from about positions 80- 93, from about positions 157- 182, from about positions 263- 276.
10. The antibody or fragment thereof according to Embodiment 4, wherein the antibody binds to an extracellular domain of GPCR41, and wherein the extracellular domain is chosen from the group of amino acids from about positions 31- 49, from about positions 104- 112, from about positions 134- 145, from about positions 170- 195, from about positions 212-270, from about positions 299- 307, from about positions 360- 368, from about positions 390- 403 and from about positions 427- 445.
11. The method of diagnosing, detecting or treating cancer in a mammal as in any one of the proceeding embodiments, wherein the antibody or fragment thereof is an immunoconjugate.
12. A diagnostic, detection or therapeutic immunoconjugate of Embodiment 11 comprising an antibody that comprises an anti-tumor cell antigen antibody or fragment thereof or an anti-tumor cell antigen antibody fusion protein or fragment thereof, wherein said antibody component is bound to at least one diagnostic/detection agent or at least one therapeutic agent.
13. The diagnostic/detection immunoconjugate of Embodiment 12, wherein said diagnostic/detection agent comprises at least one photoactive diagnostic/detection agent wherein said photoactive diagnostic agent comprises a chromagen or dye.
14. The diagnostic/detection immunoconjugate of Embodiment 12, wherein said immunoconjugate is used in intraoperative, endoscopic, or intravascular tumor detection/diagnosis.
15. The therapeutic immunoconjugate of Embodiment 12, wherein said therapeutic agent is selected from the group consisting of a radionuclide, boron, gadolinium or uranium atoms, an immunomodulator, a cytokine, a hormone, a hormone antagonist, an enzyme, an enzyme inhibitor, a photoactive therapeutic agent, a cytotoxic drug, a toxin, an angiogenesis inhibitor, a different antibody and a combination thereof.
16. The therapeutic immunoconjugate of Embodiment 15, wherein said cytotoxic drug is a drug, a prodrug, an enzyme or a toxin.
17. The therapeutic immunoconjugate of Embodiment 15, wherein said cytotoxic drug is a calicheamicin or calicheamicin analog, a maytansine or maytansine derivative or an auristatin E or auristatin E derivative.
18. The therapeutic immunoconjugate of Embodiment 15, wherein said cytotoxic drug is a toxin or fragment thereof, wherein said toxin is selected from the group consisting of plant, microbial, and animal toxins, and a synthetic variation thereof.
19. The therapeutic immunoconjugate of Embodiment 15, wherein said immunomodulator is selected from the group consisting of a cytokine, a stem cell growth factor, a lymphotoxin, a hematopoietic factor, a colony stimulating factor (CSF), an interferon (IFN), a stem cell growth factor, erythropoietin, thrombopoietin, an antibody and a combination thereof.
20. The therapeutic immunoconjugate of Embodiment 15 wherein the enzyme is a prodrug-activating enzyme.
21. The therapeutic immunoconjugate of Embodiment 20 wherein the prodrug-activating enzyme is selected from the group of alkaline phosphatase , arylsulfatase, cytosine deaminase, proteases, D-alanylcarboxypeptidases, carbohydrate-cleaving enzymes such as .beta.-galactosidase and neuraminidase, beta.-lactamase, penicillin amidases; and antibodies with enzymatic activity.
22. A multivalent, multispecific antibody or fragment thereof comprising one or more antigen binding sites having affinity toward a tumor cell antigen and one or more epitope binding sites having affinity towards epitopes, wherein said tumor cell antigen is selected from the group of KIAA1815, LOC157378, FLJ20421, DSCD75, GPR160, GPCR41 and SLC1A5.
23. The multivalent, multispecific antibody or fragment thereof of Embodiment 22, wherein said multivalent, multispecific antibody or fragment thereof is human, humanized or chimeric.
24. The multivalent, multispecific antibody or fragment thereof of Embodiment 22, further comprising a diagnostic/detection or therapeutic agent.
25. An antibody fusion protein or fragment thereof comprising at least two anti-tumor cell antigen antibodies or fragments thereof, wherein said antibodies or fragments thereof are selected from said anti-tumor cell antigen antibodies or fragments thereof according to Embodiment 1.
26. A method for treating cancer in a mammal comprising treating with a therapeutically effect amount of the antibody or fragment thereof according to any one of the proceeding embodiments, wherein the antibody is formulated in a therapeutically acceptable formulation.
27. A method of diagnosing/detecting cancer in a mammal, comprising the step of administering to said mammal a diagnostically effective amount of an antibody or fragment thereof according to any of the proceeding embodiments, formulated in a pharmaceutically acceptable vehicle.
28. A method of treating or diagnosing/detecting cancer in a mammal, comprising (i) administering to a mammal in need thereof the antibody or fragments thereof according to any one of the proceeding embodiments; (ii) waiting a sufficient amount of time for an amount of the non- binding protein to clear the mammal's bloodstream; and (iii) administering to said mammal a carrier molecule comprising a diagnostic agent, a therapeutic agent, or a combination thereof, that binds to a binding site of said antibody.
29. A DNA sequence comprising a nucleic acid encoding an anti-tumor cell antigen antibody or fragment thereof or immunoconjugate according to any one of the proceeding embodiments.
30. An expression vector comprising the DNA sequence of Embodiment 29.
31. A host cell comprising the expression vector of Embodiment 30.
32. A method of delivering a diagnostic/detection or therapeutic agent, or a combination thereof, to a target comprising (i) providing a composition comprising an immunoconjugate that comprises the antibody or fragment thereof according to any one of the proceeding embodiments and (ii) administering to a mammal in need thereof said composition.
33. A method of treating cancer in a mammal comprising administering to said mammal a therapeutically effective amount of an antibody or fragment thereof comprising at least two antibodies or fragments thereof, comprising the antibody according to any one of the proceeding embodiments, formulated in a pharmaceutically suitable excipient.
34. The method of Embodiment 33, further comprising a second antibody or fragment thereof not of Embodiment 1.
35. The method of Embodiment 33, further comprising a second antibody or fragment thereof, of Embodiment 1.
36. The method of Embodiment 33, wherein said second antibody is conjugated to a therapeutic or diagnostic/detection agent.
37. The method of Embodiment 34, wherein said anti-tumor cell antigen antibody or fragment thereof is administered before, in conjunction with, or after a second conjugated antibody reactive with a second tumor cell antigen expressed by said malignancy is administered to said mammal.
38. The method according to any one of the proceeding embodiments, wherein said anti-tumor cell antigen antibody is administered in a dosage of 10 ng/kg to up to 100 mg/kg of mammal body weight per dose.
39. The method of Embodiment 38, wherein said dosage is repeatedly administered.
40. A method of detecting or diagnosing a breast, prostate or colon cancer in a mammal, comprising use of the antibodies or immunoconjugates according to any one the proceeding embodiments.
41. A method of treating breast, prostate or colon cancer in a mammal, comprising use of the antibodies or immunoconjugates according to any one of the proceeding embodiments.
42. A method of treating breast, prostate or colon cancer in a mammal, comprising use of the antibodies or immunoconjugates according to any one of the proceeding embodiments, wherein the tumor cell antigen is selected from the group of KIAA1815, LOC157378, FLJ20421, DSCD75, GPR160, GPCR41, and SLC1A5.
43. A kit for the diagnosis or detection of cancer in a mammal, wherein said kit comprises:
   d) an antibody or fragment thereof, or an immunoconjugate or fragment thereof, according to any one of the proceeding embodiments, wherein said antibody or fragment is capable of specifically binding a tumor cell antigen wherein said tumor cell antigen is selected from the group of KIAA1815, LOC157378, FLJ20421, DSCD75, GPR160, GPCR41, and SLC1A5;
   e) a detection method enabling said diagnosis or detection of said cancer in said mammal; and
   instructions for use of the kit.

## Claims

1. Antibody or antibody fragment which binds to the FLJ20421 tumor cell antigen for use in a method of treating cancer in a mammal, wherein the antibody or antibody fragment is formulated in a therapeutically acceptable formulation.

2. Antibody or antibody fragment of claim 1 which is human, humanized or chimeric.

3. Antibody or antibody fragment of claim 1, wherein said antibody fragment is selected from the group consisting of Fv, F(ab')2, Fab' and Fab.

4. Antibody or antibody fragment of any one of claims 1-3 which specifically binds to an extracellular domain of the FLJ20421 tumor cell antigen, wherein the extracellular domain preferably is from about amino acid positions 30-359.

5. Antibody or antibody fragment of any one of claims 1-4, wherein said cancer expresses the FLJ20421 tumor cell antigen which is elevated at least 3-fold relative to a normal adjacent tissue sample or pooled normal tissue sample.

6. Antibody or antibody fragment of any one of claims 1-5, wherein said cancer is breast, prostate or colon cancer.

7. Antibody or antibody fragment of any one of claims 1-6, wherein said method of treating cancer comprises the administration of a second antibody or antibody fragment.

8. Antibody or antibody fragment of claim 7, wherein said second antibody or antibody fragment binds to a tumor cell antigen selected from the group of KIAA1815, LOC157378, DSCD75, GPR160, GPCR41, and SLC1A5.

9. Antibody or antibody fragment of any one of claims 1-8, wherein said antibody or antibody fragment is present in an immunoconjugate.

10. Antibody or antibody fragment of claim 9, which is bound to at least one therapeutic agent.

11. Antibody or antibody fragment of claim 10, wherein said therapeutic agent is selected from the group consisting of a radionuclide, boron, gadolinium or uranium atoms, an immunomodulator, a cytokine, a hormone, a hormone antagonist, an enzyme, an enzyme inhibitor, a photoactive therapeutic agent, a cytotoxic drug, a toxin, an angiogenesis inhibitor, a different antibody and a combination thereof.

12. Antibody or antibody fragment of any of claims 1-4, for use in a method of diagnosing/detecting cancer in a mammal.

13. Antibody or antibody fragment of claim 12, wherein said antibody or antibody fragment is present in an immunoconjugate.

14. Antibody or antibody fragment of claim 13, which is bound to at least one diagnostic/detection agent.

15. Antibody or antibody fragment of claim 14, wherein said diagnostic/detection agent comprises at least one photoactive diagnostic/detection agent wherein said photoactive diagnostic agent comprises a chromagen or dye.

16. Antibody or antibody fragment of claim 14, wherein said immunoconjugate is for use in intraoperative, endoscopic, or intravascular tumor detection/diagnosis.

17. An antibody fusion protein or fragment thereof comprising at least two anti-tumor cell antigen antibodies or fragments thereof, wherein the first antibody or antibody fragment binds to the FLJ20421 tumor cell antigen, and the second antibody or antibody fragment binds to a tumor cell antigen selected from the group of KIAA1815, LOC157378, DSCD75, GPR160, GPCR41, and SLC1A5.

18. A kit for the diagnosis or detection of cancer in a mammal, wherein said kit comprises:
a) an antibody or fragment thereof according to any one of the preceding claims,
b) a detection method enabling said diagnosis or detection of said cancer in said mammal; and
c) instructions for use of the kit.
